# EUROPEAN PATENT SPECIFICATION

(11) **EP 0 973 512 B1**
(45) Date of publication and mention of the grant of the patent: **07.04.2004**
(21) Application number: 98906468.8
(22) Date of filing: 17.02.1998
(51) Int. Cl.: A61K 31/16, C07D 211/66

(54) **N-HYDROXY-2-(ALKYL, ARYL OR HETEROARYL SULFANYL, SULFINYL OR SULFONYL)-3-SUBSTITUTED ALKYL, ARYL OR HETEROARYLAMIDES AS MATRIX METALLOPROTEINASE INHIBITORS**
N-HYDROXY-2-(ALKYL, ARYL ODER HETEROARYL SULFANYL, SULFINYL ODER SULFONYL)-3-SUBSTITUIERTE ALKYL-, ARYL- ODER HETEROARYLAMIDE ALS MATRIXMETALLOPROTEINASEINHIBITOREN
N-HYDROXY-2-(ALKYLE, ARYLE, OU HETEROARYLE SULFANYLE, SULFINYLE OU SULFONYLE)-3-SUBSTITUE ALKYLE, ARYLE OU HETEROARYLAMIDES, EN TANT QU'INHIBITEURS DE METALLOPROTEASES MATRICIELLES

(30) Priority: 27.02.1997 US 806728
(43) Date of publication of application: 26.01.2000
(73) Proprietor: Wyeth Holdings Corporation, Madison, NJ 07940 (US)
(72) Inventor: VENKATESAN, Aranapakam, Mudumbai, Rego Park, NY 11374 (US); GROSU, George, Theodore, Pearl River, NY 10965 (US); DAVIS, Jamie, Marie, Nyack, NY 10960 (US); BAKER, Jannie, Lea, White Plains, NY 10607 (US)
(74) Representative: Talbott, Dawn Jacqueline
(86) International application number: PCT/US1998/002987
(87) International publication number: WO 1998/037877

(56) References cited:
- EP-A- 0 606 046
- WO-A-93/20047
- WO-A-96/06074
- WO-A-96/35714

## Description

### BACKGROUND OF THE INVENTION

Matrix metalloproteinases (MMPs) are a group of enzymes that have been implicated in the pathological destruction of connective tissue and basement membranes. These zinc containing endopeptidases consist of several subsets of enzymes including collagenases, stromelysins and gelatinases. Of these classes, the gelatinases have been shown to be the MMPs most intimately involved with the growth and spread of tumors. It is known that the level of expression of gelatinase is elevated in malignancies, and that gelatinase can degrade the basement membrane which leads to tumor metastasis. Angiogenesis, required for the growth of solid tumors, has also recently been shown to have a gelatinase component to its pathology. Furthermore, there is evidence to suggest that gelatinase is involved in plaque rupture associated with atherosclerosis. Other conditions mediated by MMPs are restenosis, MMP-mediated osteopenias, inflammatory diseases of the central nervous system, skin aging, tumor growth, osteoarthritis, rheumatoid arthritis, septic arthritis, corneal ulceration, abnormal wound healing, bone disease, proteinuria, aneurysmal aortic disease, degenerative cartilage loss following traumatic joint injury, demyelinating diseases of the nervous system, cirrhosis of the liver, glomerular disease of the kidney, premature rupture of fetal membranes, inflammatory bowel disease, periodontal disease, age related macular degeneration, diabetic retinopathy, proliferative vitreoretinopathy, retinopathy of prematurity, ocular inflammation, keratoconus, Sjogren's syndrome, myopia, ocular tumors, ocular angiogenesis/neovascularization and corneal graft rejection. For recent reviews, see: (1) Recent Advances in Matrix Metalloproteinase Inhibitor Research, R. P. Beckett, A. H. Davidson, A. H. Drummond, P. Huxley and M. Whittaker, Research Focus, Vol. 1, 16-26, (1996), (2) Curr. Opin. Ther. Patents (1994) 4(1): 7-16, (3) Curr. Medicinal Chem. (1995) 2: 743-762, (4) Exp. Opin. Ther. Patents (1995) 5(2): 1087-110, (5) Exp. Opin. Ther. Patents (1995) 5(12): 1287-1196.

TNF-α converting enzyme (TACE) catalyzes the formation of TNF-α from membrane bound TNF-α precursor protein. TNF-α is a pro-inflammatory cytokine that is now thought to have a role in rheumatoid arthritis, septic shock, graft rejection, cachexia, anorexia, inflammation, congestive heart failure, inflammatory disease of the central nervous system, inflammatory bowel disease, insulin resistance and HIV infection in addition to its well documented antitumor properties. For example, research with anti- TNF-α antibodies and transgenic animals has demonstrated that blocking the formation of TNF-α inhibits the progression of arthritis. This observation has recently been extended to humans as well.

It is expected that small molecule inhibitors of MMPs and TACE therefore have the potential for treating a variety of disease states. While a variety of MMP and TACE inhibitors have been identified and disclosed in the literature, the vast majority of these molecules are peptidic and peptide-like compounds that one would expect to have bioavailability and pharmacokinetic problems common to such compounds that would limit their clinical effectiveness. Low molecular weight, potent, long acting, orally bioavailable inhibitors of MMPs and/or TACE are therefore highly desirable for the potential chronic treatment of the above mentioned disease states.

Recently, two references have appeared (U.S. 5,455,258 and European Patent Appl. 606,046) that disclose arylsulfonamido-substituted hydroxyamic acids. These documents cover compounds exemplified by CGS 27023A. These are the only non-peptide matrix metalloproteinase inhibitors disclosed to date.

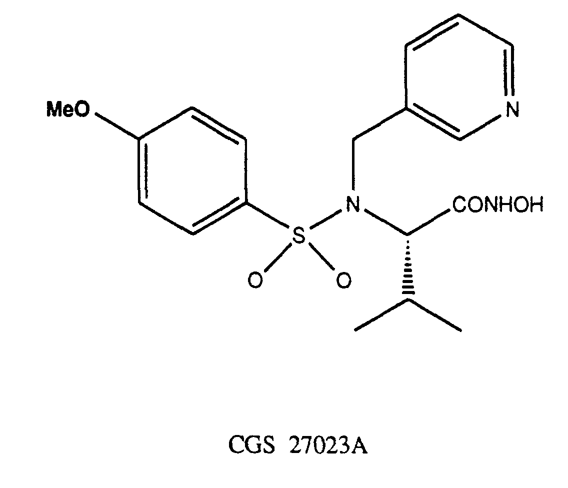

Salah et al., Liebigs Ann. Chem. 195, (1973) discloses some aryl substituted thio and aryl substituted sulfonyl acetohydroxamic acid derivatives of general formula 1. These compounds were prepared to study the Mannich reaction. Subsequently, they were tested for their fungicidal activity.

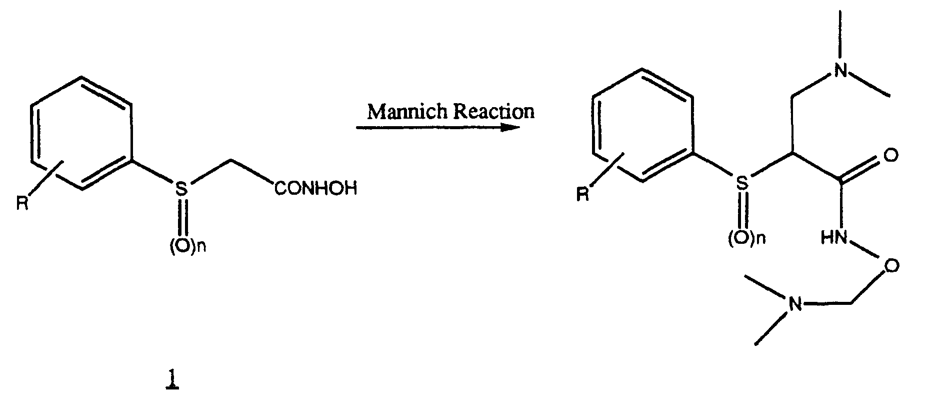

Some sulfone carboxylic acids are disclosed in U.S. patent 4,933,367. Those compounds were shown to exhibit hypoglycemic activity.

### SUMMARY OF THE INVENTION:

The present invention relates to novel, low molecular weight, non-peptide inhibitors of matrix metalloproteinases (MMPs) and TNF-α converting enzyme (TACE) for the treatment of arthritis, tumor metastasis, tissue ulceration, abnormal wound healing, periodontal disease, bone disease, diabetes (insulin resistance) and HIV infection.
In accordance with this invention there is provided a group of compounds of general formula I 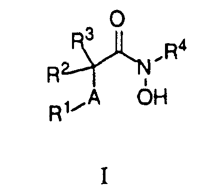 wherein:
- R¹ is: alkyl of 1 to 18 carbon atoms, optionally substituted with one or two groups selected independently from R⁵;
alkenyl of 3 to 18 carbon atoms having 1 to 3 double bonds, optionally substituted with one or two groups selected independently from R⁵;
alkynyl of 3 to 18 carbon atoms having 1 to 3 triple bonds, optionally substituted with one or two groups selected independently from R⁵;
aryl of 6 to 10 carbon atoms, optionally substituted with one or two groups selected independently from R⁵;
cycloalkyl of 3 to 8 carbon atoms, optionally substituted with one or two groups selected independently from R⁵;
saturated or unsaturated 5 to 10 membered mono or bicyclic heterocycle containing one heteroatom selected from O, S or NR⁷, optionally substituted with one or two groups selected independently from R⁵;
or heteroaryl-(CH₂)₀₋₆- wherein the heteroaryl group is 5 to 6 membered with one or two heteroatoms selected independently from O, S, and N and may be optionally substituted with one or two groups selected independently from R⁵;
- A is: -S-, -SO- or SO₂-;
- R² and R³,: taken with the carbon atom to which they are attached, form a 5 to 7 membered heterocyclic ring containing O, S or N-R⁷ optionally having one or two double bonds;
- R⁴ is: hydrogen,
alkyl of 1 to 6 carbon atoms, optionally substituted with one or two groups selected independently from R⁵;
alkenyl of 3 to 18 carbon atoms having 1 to 3 double bonds, optionally substituted with one or two groups selected independently from R⁵;
alkynyl of 3 to 18 carbon atoms having 1 to 3 triple bonds, optionally substituted with one or two groups selected independently from R⁵;
phenyl or naphthyl optionally substituted with one or two groups selected independently from R⁵;
C₃ to C₈ cycloalkyl or bicycloalkyl optionally substituted with one or two groups selected independently from R⁵;
saturated or unsaturated 5 to 10 membered mono or bicyclic heterocycle containing one heteroatom selected from O, S or NR⁷, optionally substituted with one or two groups selected independently from R⁵;
- R⁵ is: H, C₇-C₁₁ aroyl, C₂-C₆ alkanoyl, C₁ to C₁₂ alkyl, C₂ to C₁₂ alkenyl, C₂-C₁₂ alkynyl, F, Cl, Br, I, CN, CHO, C₁-C₆ alkoxy, aryloxy, heteroaryloxy, C₃-C₆ alkenyloxy, C₃-C₆ alkynyloxy, C₁-C₆ alkoxyaryl, C₁-C₆ alkoxyheteroaryl, C₁-C₆ alkylamino-C₁-C₆ alkoxy, C₁-C₂ alkylene dioxy, aryloxy-C₁-C₆ alkyl amine, C₁-C₁₂ perfluoro alkyl, S(O)ₙ-C₁-C₆ alkyl, S(O)ₙ-aryl where n is 0, 1 or 2; OCOO C₁-C₆ alkyl, OCOOaryl, OCONR⁶, COOH, COO C₁-C₆ alkyl, COOaryl, CONR⁶R⁶, CONHOH, NR⁶R⁶, SO₂NR⁶R⁶, NR⁶SO₂aryl, -NR⁶CONR⁶R⁶, NHSO₂CF₃, SO₂NHheteroaryl,SO₂NHCOaryl, CONHSO₂-C₁-C₆ alkyl, CONHSO₂aryl, SO₂NHCOaryl, CONHSO₂-C₁-C₆ alkyl, CONHSO₂aryl, NH₂, OH, aryl, heteroaryl, C₃ to C₈ cycloalkyl; or saturated or unsaturated 5 to 10 membered mono or bicyclic heterocycle containing one heteroatom selected from O, S or NR⁷, wherein C₁-C₆
alkyl is straight or branched, heteroaryl is a 5-10 membered mono or bicyclic heteroaryl group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
- R⁶ is: H, C₁ to C₁₈ alkyl optionally substituted with OH; C₃ to C₆ alkenyl, C₃ to C₆ alkynyl, C₁ to C₆ perfluoro alkyl, S(O)ₙ-C₁-C₆ alkyl S(O)ₙ aryl where n is 0, 1 or 2;, or COheteroaryl, wherein heteroaryl is a 5-10 membered mono or bicyclic heteroaryl
group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
- and R⁷ is: C₇-C₁₁ aroyl, C₂-C₆ alkanoyl, C₁-C₁₂ perfluoro alkyl, S(O)ₙ-C₁-C₆-alkyl, S(O)ₙ-aryl where n is 0, 1 or 2; COO-C₁-C₆-alkyl, COOaryl, CONHR⁶, CONR⁶R⁶, CONHOH, SO₂NR⁶R⁶, SO₂CF₃, SO₂NHheteroaryl, SO₂NHCOaryl, CONHSO-C₁-C₆-alkyl, CONHSO₂aryl, aryl, or heteroaryl, where aryl is
phenyl or naphthyl, optionally substituted by 1 or 2 groups selected independently from halogen, cyano, amino, nitro, C₁-C₆ alkyl, C₁-C₆ alkoxy, or hydroxy; and heteroaryl is a 5-10 membered mono or bicyclic heteroaryl group having 1 to 3 heteroatoms selected independently from O, S or N-C₁-C₆ alkyl;
alkyl of 1 to 18 carbon atoms, optionally substituted with one or two groups selected independently from R⁵;
alkenyl of 3 to 18 carbon atoms having from 1 to 3 double bonds, optionally substituted with one or two groups selected independently from R⁵;
alkynyl of 3 to 18 carbon atoms having from 1 to 3 triple bonds, optionally substituted with one or two groups selected independently from R⁵;
arylalkyl of 7 to 16 carbon atoms, wherein aryl is optionally substituted with one or two groups selected independently from R⁵;
biphenylalkyl of 13 to 18 carbon atoms, wherein biphenyl is optionally substituted with one or two groups selected independently from R⁵;
arylalkenyl of 8 to 16 carbon atoms, wherein aryl is optionally substituted with one or two groups selected independently from R⁵;
cycloalkylalkyl or bicycloalkylalkyl of 4 to 12 carbon atoms, wherein the cycloalkyl or bicycloalkyl group is optionally substituted with one or two groups selected independently from R⁵;
saturated or unsaturated mono or bicyclic heterocycle containing one heteroatom selected from O, S or N-C₁-C₆ alkyl, optionally substituted with one or two groups selected independently from R⁵; or
R⁸R⁹N-C₁-C₆-alkoxyaryl-C₁-C₆-alkyl where R⁸ and R⁹ are independently selected from C₁-C₆ alkyl or R⁸ and R⁹ together with the interposed nitrogen forms a 5-7 membered saturated heterocyclic ring optionally containing an oxygen atom, wherein the aryl group is phenyl or naphthyl;
and the pharmaceutically acceptable salts thereof.

A more preferred aspect of the present invention is the group of compounds of general formula (Ia): 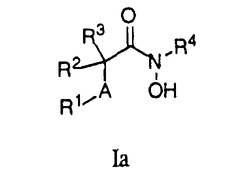 wherein:
- R¹ is: alkyl of 1 to 18 carbon atoms, optionally substituted with one or two groups selected independently from R⁵;
alkenyl of 3 to 18 carbon atoms having 1 to 3 double bonds, optionally substituted with one or two groups selected independently from R⁵;
alkynyl of 3 to 18 carbon atoms having 1 to 3 triple bonds, optionally substituted with one or two groups selected independently from R⁵;
aryl of 6 to 10 carbon atoms, optionally substituted with one to two groups selected independently from R⁵;
cycloalkyl of 3 to 8 carbon atoms, optionally substituted with one to two groups selected independently from R⁵;
saturated or unsaturated mono or bicyclic heterocycle of from 5 to 10 members containing one heteroatom selected from O, S or NR⁷, optionally substituted with one to two groups selected independently from R⁵;
or heteroaryl-(CH₂)₀₋₆- wherein the heteroaryl group is 5 to 6 membered with one or two heteroatoms selected independently from O, S, and N and may be optionally substituted with one or two groups selected independently from R⁵;
- A is: -S-, -SO- or SO₂-;
- R² and R³,: taken with the carbon atom to which they are attached, form a 5 to 7 membered heterocyclic ring containing O, S or N-R⁷ optionally having one or two double bonds;
- R⁴ is: hydrogen,
alkyl of 1 to 6 carbon atoms, optionally substituted with one or two groups selected independently from R⁵;
alkenyl of 3 to 18 carbon atoms having 1 to 3 double bonds, optionally substituted with one or two groups selected independently from R⁵;
alkynyl of 3 to 18 carbon atoms having 1 to 3 triple bonds, optionally substituted with one or two groups selected independently from R⁵;
phenyl or naphthyl optionally substituted with one or two groups selected independently from R⁵;
C₃ to C₈ cycloalkyl or bicycloalkyl optionally substituted with one or two groups selected independently from R⁵;
- R⁵ is: H, F, Cl, Br, I, CN, CHO, C₇-C₁₁ aroyl, C₂-C₆ alkanoyl, C₁ to C₁₂ alkyl, C₂ to C₁₂ alkenyl, C₂-C₁₂ alkynyl, C₁-C₆ alkoxy, aryloxy, heteroaryloxy, C₃-C₆ alkenyloxy, C₃-C₆ alkynyloxy, C₁-C₆ alkoxyaryl, C₁-C₆ alkoxyheteroaryl, C₁-C₆-alkylamino-C₁-C₆ alkoxy, C₁-C₂-alkylene dioxy, aryloxy-C₁-C₆ alkyl amine, C₁-C₁₂ perfluoro alkyl, S(O)ₙ-C₁-C₆ alkyl, S(O)ₙ-aryl where n is 0, 1 or 2; OCOO-C₁-C₆ alkyl, OCOOaryl, OCONR⁶, COOH, COO-C₁-C₆ alkyl, COOaryl, CONR⁶R⁶, CONHOH, NR⁶R⁶, SO₂NR⁶R⁶, NR⁶SO₂aryl, NR⁶CONR⁶R⁶, NHSO₂CF₃, SO₂NHheteroaryl, SO₂NHCOaryl, CONHSO₂-C₁-C₆ alkyl, CONHSO₂aryl, SO₂NHCOaryl, CONHSO₂-C₁-C₆ alkyl, CONHSO₂aryl, NH₂, OH, aryl, heteroaryl, C₃ to C₈ cycloalkyl; or saturated or unsaturated 5 to 10 membered mono or bicyclic heterocycle containing one heteroatom selected from O, S or NR⁷;
wherein heteroaryl is a 5-10 membered mono or bicyclic heteroaryl group having 1 to 3 heteroatoms selected independently from O, S or NR⁷ and aryl is phenyl or naphthyl, optionally substituted by 1 or 2 groups selected independently from halogen, cyano, amino, nitro, C₁-C₆ alkyl, C₁-C₆ alkoxy, or hydroxy;
- R⁶ is: H, C₁ to C₁₈ alkyl optionally substituted with OH; C₃ to C₆ alkenyl, C₃ to C₆ alkynyl, C₁ to C₆ perfluoro alkyl, S(O)ₙ alkyl or aryl where n is 0, 1, or 2; or COheteroaryl;
wherein heteroaryl is a 5-10 membered mono or bicyclic heteroaryl group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
- and R⁷ is: C₇-C₁₁ aroyl, C₂-C₆ alkanoyl, C₁-C₁₂ perfluoro alkyl, S(O)ₙ-alkyl, S(O)ₙ-aryl where n is 0, 1 or 2; COOalkyl, COOaryl, CONHR⁶, CONR⁶R⁶, CONHOH, SO₂NR⁶R⁶,SO₂CF₃, SO₂NHheteroaryl, SO₂NHCOaryl, CONHSO₂alkyl, CONHSO₂aryl, aryl, heteroaryl; wherein C₁-C₆ alkyl is straight or
branched, heteroaryl is a 5-10 membered mono or bicyclic heteroaryl group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
alkyl of 1 to 18 carbon atoms, optionally substituted with one or two groups selected independently from R⁵;
alkenyl of 3 to 18 carbon atoms having from 1 to 3 double bonds, optionally substituted with one or two groups selected independently from R⁵;
alkynyl of 3 to 18 carbon atoms having from 1 to 3 triple bonds, optionally substituted with one or two groups selected independently from R⁵;
arylalkyl of 7 to 16 carbon atoms, wherein aryl is optionally substituted with one or two groups selected independently from R⁵;
biphenylalkyl of 13 to 18 carbon atoms, wherein biphenyl is optionally substituted with one or two groups selected independently from R⁵;
arylalkenyl of 8 to 16 carbon atoms, wherein aryl is optionally substituted with one or two groups selected independently from R⁵;
cycloalkylalkyl or bicycloalkylalkyl of 4 to 12 carbon atoms, wherein cycloalkyl or bicycloalkyl is optionally substituted with one or two groups selected independently from R⁵;
saturated or unsaturated mono or bicyclic heterocycle containing one heteroatom selected from O, S or N-C₁-C₆ alkyl, optionally substituted with one or two groups selected independently from R⁵;
R⁸R⁹N-C₁-C₆-alkoxyaryl-C₁-C₆-alkyl where R⁸ and R⁹ are independently selected from C₁-C₆ alkyl or R⁸ and R⁹ together with the interposed nitrogen forms a 5-7 membered saturated heterocyclic ring optionally containing an oxygen atom, wherein the aryl group is phenyl or naphthyl;
and the pharmaceutically acceptable salts thereof.

The most preferred group of compounds are those of the following formula (Ib): 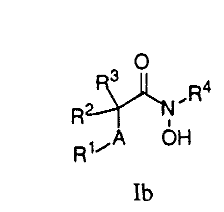 in which
- R¹ is: phenyl, naphthyl, alkyl of 1-18 carbon atoms or heteroaryl such as pyridyl, thienyl, imidazolyl or furanyl, optionally substituted with C₁-C₆ alkyl, C₁-C₆ alkoxy, C₆-C₁₀ aryloxy, heteroaryloxy, C₃-C₆ alkenyloxy, C₃-C₆ alkynyloxy, halogen; or S(O)ₙ -C₁-C₆alkyl C₁-C₆ alkoxyaryl or C₁-C₆ alkoxyheteroaryl;
- A is: -S-, -SO- or -SO₂-;
- R² and R³ ,: taken with the carbon atom to which they are attached, form a 5 to 7 membered heterocyclic ring containing O, S or N-R⁷ optionally having one or two double bonds;
- R⁴ is: hydrogen,
alkyl of 1 to 6 carbon atoms, optionally substituted with one or two groups selected independently from R⁵;
alkenyl of 3 to 18 carbon atoms having 1 to 3 double bonds, optionally substituted with one or two groups selected independently from R⁵;
alkynyl of 3 to 18 carbon atoms having 1 to 3 triple bonds, optionally substituted with one or two groups selected independently from R⁵;
phenyl or naphthyl optionally substituted with one or two groups selected independently from R⁵;
C₃ to C₈ cycloalkyl or bicycloalkyl optionally substituted with one or two groups selected independently from R⁵;
- R⁵ is: H, C₇-C₁₁ aroyl, C₂-C₆ alkanoyl, C₁ to C₁₂ alkyl, C₂ to C₁₂ alkenyl, C₂-C₁₂ alkynyl, F, Cl, Br, I, CN, CHO, C₁-C₆ alkoxy, aryloxy, heteroaryloxy, C₃-C₆ alkenyloxy, C₃-C₆ alkynyloxy, C₁-C₆ alkylamino-C₁-C₆ alkoxy, C₁-C₂ alkylene dioxy, aryloxy-C₁-C₆ alkyl amine, C₁-C₁₂ perfluoro alkyl, S(O)ₙ-C₁-C₆ alkyl, S(O)ₙ-aryl where n is 0, 1 or 2; OCOO C₁-C₆ alkyl, OCOOaryl, OCONR⁶, COOH, COO C₁-C₆ alkyl, COOaryl, CONR⁶R⁶, CONHOH, NR⁶R⁶, SO₂NR⁶R⁶, NR⁶SO₂aryl, -NR⁶CONR⁶R⁶, NHSO₂CF₃, SO₂NHheteroaryl,SO₂NHCOaryl, CONHSO₂-C₁-C₆ alkyl, CONHSO₂aryl, SO₂NHCOaryl, CONHSO₂-C₁-C₆ alkyl, CONHSO₂aryl, NH₂, OH, aryl, heteroaryl, C₃ to C₈ cycloalkyl; saturated or unsaturated 5 to 10 membered mono or bicyclic heterocycle containing one heteroatom selected from O, S or NR⁷, wherein C₁-C₆ alkyl is straight or branched, heteroaryl is a 5-10 membered
mono or bicyclic heteroaryl group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
- R⁶ is: H, C₁ to C₁₈ alkyl optionally substituted with OH; C₃ to C₆ alkenyl, C₃ to C₆ alkynyl, C₁ to C₆ perfluoro alkyl, S(O)ₙ alkyl or aryl where n is 0, 1 or 2; or COheteroaryl; wherein heteroaryl is a 5-10 membered mono or bicyclic heteroaryl
group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
- and R⁷: is C₇-C₁₁ aroyl, C₂-C₆ alkanoyl, C₁-C₁₂ perfluoro alkyl, S(O)ₙ-alkyl, S(O)ₙ-aryl where n is 0, 1 or 2; COOalkyl, COOaryl, CONHR⁶, CONR⁶R⁶, CONHOH, SO₂NR⁶R⁶,SO₂CF₃, SO₂NHheteroaryl, SO₂NHCOaryl, CONHSO₂alkyl, CONHSO₂aryl, aryl, or heteroaryl; where aryl is phenyl or naphthyl,
optionally substituted by 1 or 2 groups selected independently from halogen, cyano, amino, nitro, C₁-C₆ alkyl, C₁-C₆ alkoxy, or hydroxy; and heteroaryl is a 5-10 membered mono or bicyclic heteroaryl group having 1 to 3 heteroatoms selected independently from O, S or N-C₁-C₆ alkyl;
alkyl of 1 to 18 carbon atoms, optionally substituted with one or two groups selected independently from R5;
alkenyl of 3 to 18 carbon atoms having from 1 to 3 double bonds, optionally substituted with one or two groups selected independently from R⁵;
alkynyl of 3 to 18 carbon atoms having from 1 to 3 triple bonds, optionally substituted with one or two groups selected independently from R⁵;
arylalkyl of 7 to 16 carbon atoms, optionally substituted with one or two groups selected independently from R⁵;
biphenylalkyl of 13 to 18 carbon atoms, optionally substituted with one or two groups selected independently from R⁵;
arylalkenyl of 8 to 16 carbon atoms, optionally substituted with one or two groups selected independently from R⁵;
cycloalkylalkyl or bicycloalkylalkyl of 4 to 12 carbon atoms, optionally substituted with one or two groups selected independently from R⁵;
saturated or unsaturated mono or bicyclic heterocycle containing one heteroatom selected from O, S or NR-C₁-C₆ alkyl, optionally substituted with one or two groups selected independently from R⁵;
R⁸R⁹N-C₁-C₆-alkoxyaryl-C₁-C₆-alkyl where R⁸ and R⁹ are independently selected from C₁-C₆ alkyl or R⁸ and R⁹ together with the interposed nitrogen forms a 5-7 membered saturated heterocyclic ring optionally containing an oxygen atom, wherein the aryl group is phenyl or naphthyl;
and the pharmaceutically acceptable salts thereof.

The most preferred matrix metalloproteinase and TACE inhibiting compounds of this invention are:
1-benzyl-4-(4-methoxy-benzenesulfonyl)-piperidine-4-carboxylic acid hydroxyamide,
4-(4-methoxy-benzenesulfonyl)-1-(3-methoxy-benzyl)-piperidine-4-carboxylic acid hydroxyamide,
1-(3,4-dichlorobenzyl) -4-(4-methoxy-benzenesulfonyl)-piperidine-4-carboxylic acid hydroxamide,
4-(4-methoxy-benzenesulfonyl)-1-(4-methylbenzyl)-piperidine-4-carboxylic acid hydroxamide,
4-(4-methoxy-benzene-sulfonyl)-1-napthalene-2-yl-methylpiperidine-4-carboxylic acid hydroxamide,
1-biphenyl-4-ylmethyl-4-(4-methoxy-benzenesulfonyl)piperidine-4-carboxylic acid hydroxamide,
4-(4-methoxy-benzene-sulfonyl)-1-(3-methyl-but-2-enyl)piperidine-4-carboxylic acid hydroxamide,
1-(4-bromo-benzyl)-4-(4-methoxy-benzenesulfonyl)-piperidine-4-carboxylic acid hydroxyamide,
4-(4-methoxy-benzenesulfonyl)-1-(3-phenyl-propyl)-piperidine-4-carboxylic acid hydroxyamide,
1-tert-butyl-4-(4-methoxy-benzenesulfonyl)-piperidine-4-carboxylic acid hydroxyamide,
1-butyl-4-(4-methoxy-benzenesulfonyl)-piperidine-4-carboxylic acid hydroxyamide,
1-cyclooctyl-4-(4-methoxy-benzenesulfonyl)-piperidine-4-carboxylic acid hydroxyamide,
1-ethyl-4-(4-methoxy-benzenesulfonyl)-piperidine-4-carboxylic acid hydroxyamide,
1-isopropyl-4-(4-methoxy-benzenesulfonyl)-piperidine-4-carboxylic acid hydroxyamide,
1-methyl-4-(4-methoxy-benzenesulfonyl)-piperidine-4-carboxylic acid hydroxyamide,
1-benzyl-4-(4-butoxy-benzenesulfonyl)-piperidine-4-carboxylic acid hydroxyamide,
1-(4-fluoro-benzyl)-4-(4-methoxy-benzenesulfonyl)-piperidine-4-carboxylic acid hydroxyamide,
1-(4-fluoro-benzyl)-4-(4-butoxy-benzenesulfonyl)-piperidine-4-carboxylic acid hydroxyamide,
4-(4-methoxy-benzenesulfonyl)-1-(4-methoxy-benzyl)-piperidine-4-carboxylic acid hydroxyamide,
4-(4-methoxy-benzenesulfonyl)-1-[2-(4-methoxyphenyl)-ethyl]-piperidine-4-carboxylic acid hydroxyamide,
4-(4-methoxy-benzenesulfonyl)-1-(2-phenyl-ethyl)-piperidine-4-carboxylic acid hydroxyamide,
4-(4-n-butoxy-benzenesulfonyl)-1-(4-methoxy-benzyl)-piperidine-4-carboxylic acid hydroxyamide,
4-(4-methoxy-benzenesulfonyl)-1-(3-phenoxy-propyl)-piperidine-4-carboxylic acid hydroxyamide,
4-(4-n-butoxy-benzenesulfonyl)-1-(3-phenoxy-propyl)-piperidine-4-carboxylic acid hydroxyamide,
4-(4-methoxy-benzenesulfonyl)-1-(2-phenoxy-ethyl)-piperidine-4-carboxylic acid hydroxyamide,
4-(4-n-butoxy-benzenesulfonyl)-1-(2-phenoxy-ethyl)-piperidine-4-carboxylic acid hydroxyamide,
4-(4-methoxy-benzenesulfonyl)-1-[4-(2-piperidin-1-yl-ethoxy)-benzyl]-piperidine-4-carboxylic acid hydroxyamide,

It is understood that the definition of the compounds of formulas I, Ia and Ib, when R¹, R², R³ and R⁴ contains asymmetric carbons, encompass all possible stereoisomers and mixtures thereof which posses the activity discussed below. In particular, it encompasses racemic modifications and any optical isomers which possesses the indicated activity. Optical isomers may be obtained in pure form by standard separation techniques. Where not stated otherwise, the term "alkyl" refers to a straight or branched C₁-C₆ alkyl group and aryl is phenyl or naphthyl. The pharmaceutically acceptable salts are those derived from pharmaceutically acceptable organic and inorganic acids such as lactic, citric, acetic, tartaric, succinic, maleic, malonic, hydrochloric, hydrobromic, phosphoric, nitric, sulfuric, methanesulfonic, and similarly known acceptable acids.

The present invention accordingly provides a pharmaceutical composition which comprises a compound of this invention in combination or association with a pharmaceutically acceptable carrier. In particular, the present invention provides a pharmaceutical composition which comprises an effective amount of compound of this invention and a pharmaceutically acceptable carrier.

The compositions are preferably adapted for oral administration. However, they may be adapted for other modes of administration, for example, parenteral administration for patients.

In order to obtain consistency of administration, it is preferred that a composition of the invention is in the form of a unit dose. Suitable unit dose forms include tablets, capsules, and powders in sachets or vials. Such unit dose forms may contain from 0.1 to 100 mg of a compound of the invention. The compounds of the present invention can be administered orally at a dose range of about 0.01 to 100 mg per kg. Such composition may be administered from 1 to 6 times a day, more usually from 1 to 4 times a day.

The compositions of the invention may be formulated with conventional excipients, such as fillers, a disintegrating agent, a binder, a lubricant, a flavoring agent, and the like. They are formulated in conventional manner.

Also according to the present invention, there are provided processes for producing the compounds of the present invention.

### PROCESS OF THE INVENTION.

The compounds of the present invention may be prepared according to one of the general processes out lined below.

The appropriately substituted mercaptan derivative was alkylated using either substituted (Scheme I) or unsubstituted ( Scheme 2) α-bromo acetic acid ester derivative in refluxing acetone using K₂CO₃ as base. The sulphide derivative thus obtained was oxidized using m-chloroperbenzoic acid in CH₂Cl₂ or by using Oxone in methanol/ water. The sulfone obtained from the above mentioned process can be either further alkylated using variety of alkyl halides to obtain the disubstituted derivative or it can be hydrolyzed using NaOH/ MeOH at room temp. However instead of using the ethyl ester, if the tertiary butyl ester is present, the hydrolysis can be carried out with TFA/CH₂Cl₂ at room temperature. Subsiquently, the carboxylic acid obtained was converted to the hydroxamic acid derivative by reaction with oxalyl chloride/ DMX (catalytic) and hydroxyl amine/ triethyl amine.

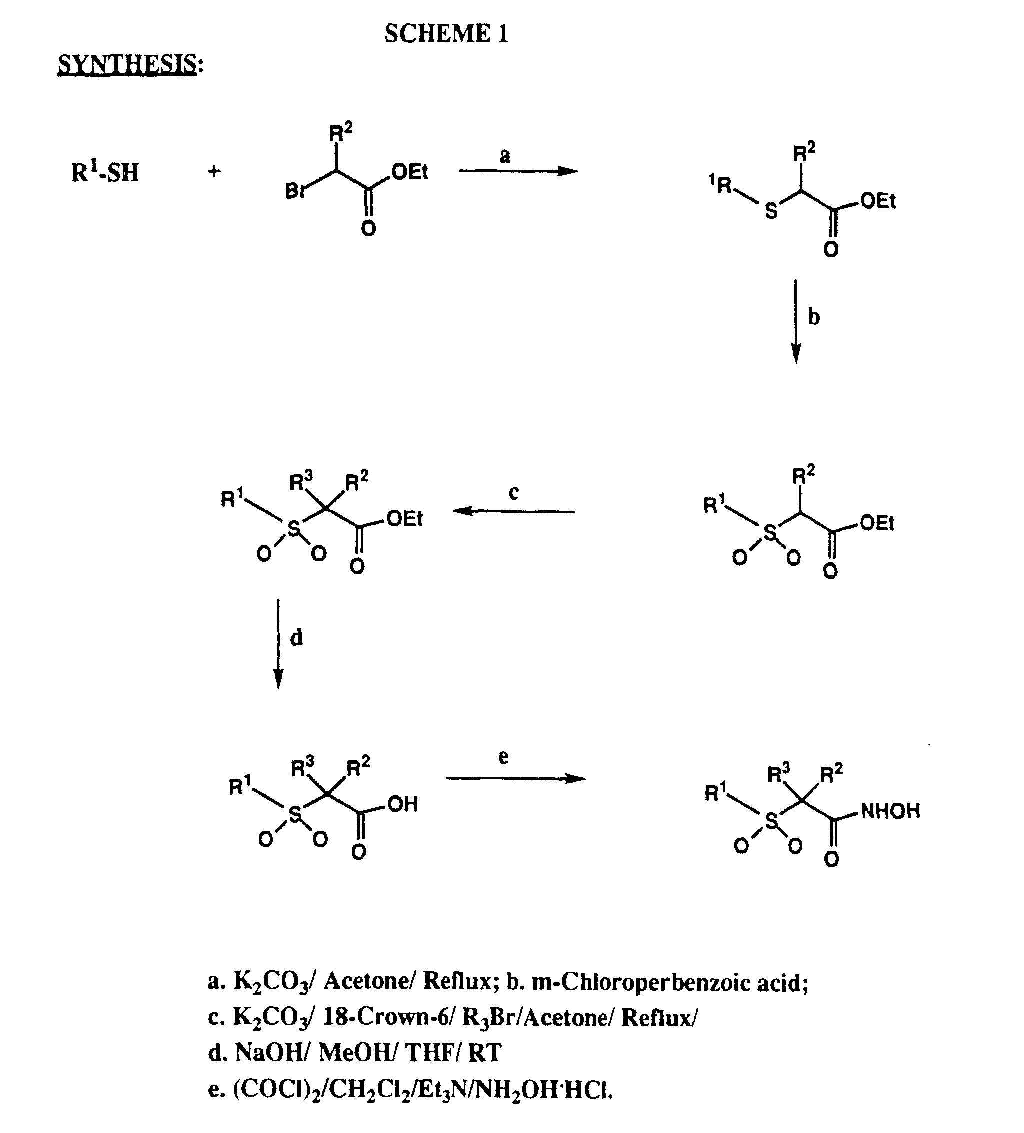

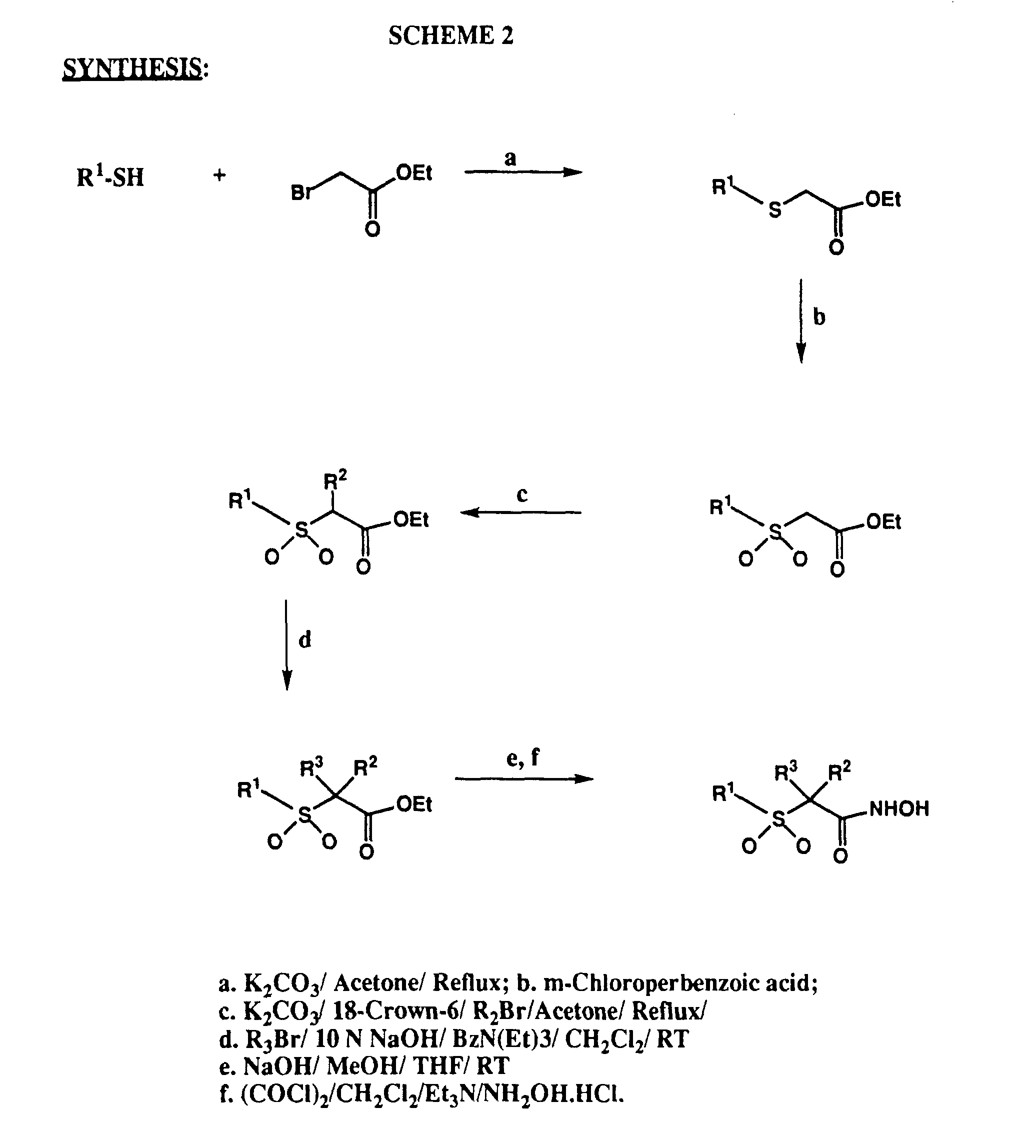

As outlined in Scheme 3, the sulfide derivative can be further alkylated using lithium bis(trimethyl silyl)amide in THF at 0° C. The alkylated or mono substituted compound was hydrolyzed and converted to the hydroxamic acid derivative. The sulfinyl derivatives were prepared by oxidizing the sulfide hydroxamic acid derivatives with H₂O₂ in MeOH solution.

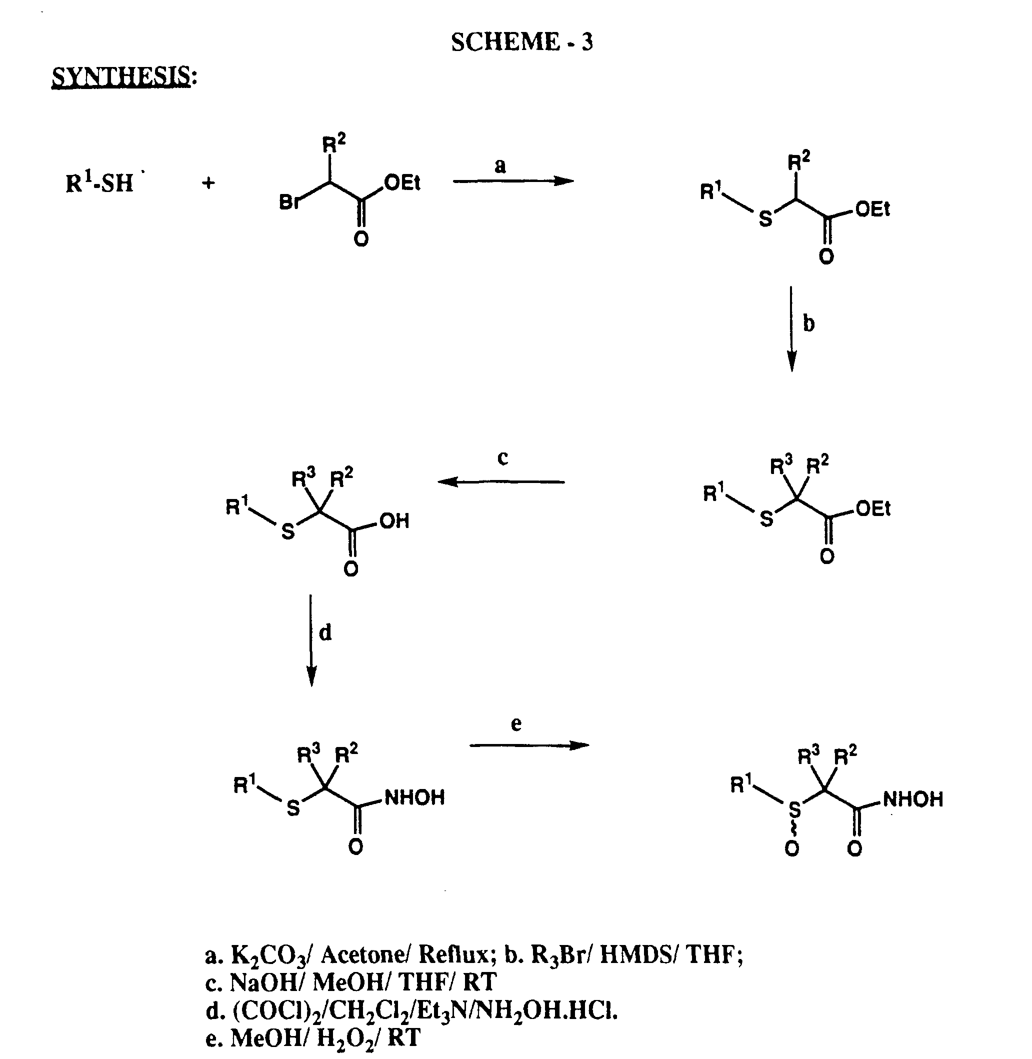

The corresponding 1-substituted-4-(4-methoxy-benzenesulfonyl)-piperidine-4-carboxylic acid hydroxyamides were prepared starting from diethanolamine and appropriately substituted alkyl or aryl halides (Scheme 4). The N-substituted diethanol amine derivatives were converted to the dichloro compounds using thionyl chloride. The corresponding dichlorides were reacted with substituted sulfonyl acetic acid ethyl ester derivatives in the presence of K₂CO₃/18-Crown-6 in boiling acetone. 1-substituted-4-(4-methoxybenzenesulfonyl)-piperidine-4-carboxylic acid ethyl esters thus obtained were converted to the hydroxy amide as outlined in Scheme 4. Alternatively these classes of compounds and other hetrocycles can be prepared as indicated in Scheme 5 and 6. 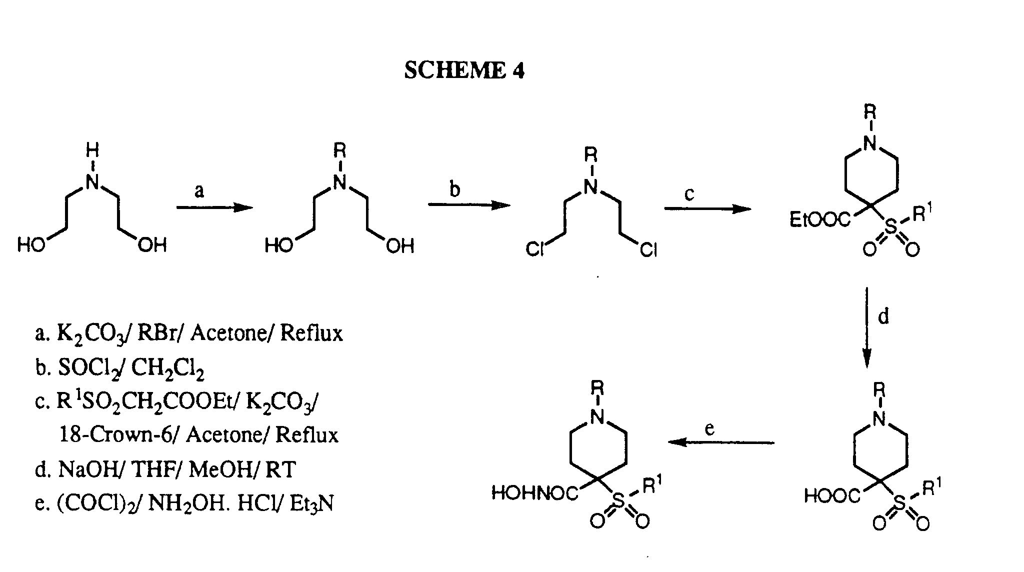 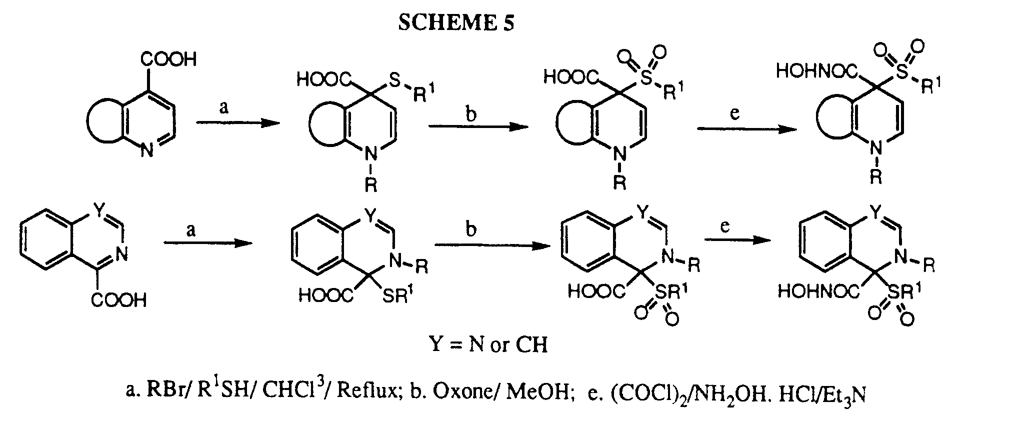 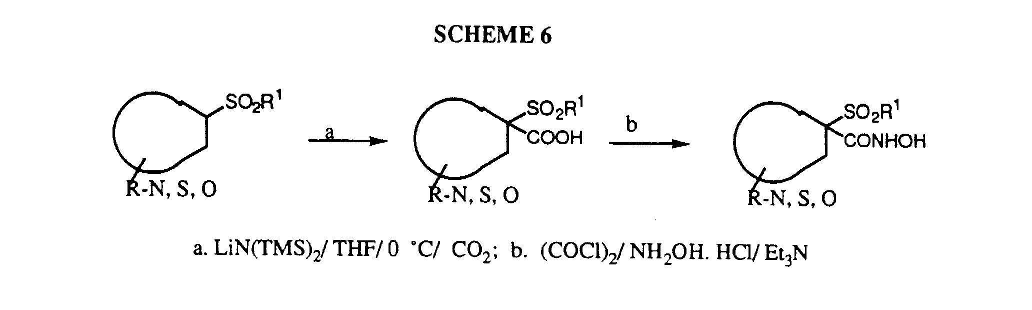 Alternatively, Schemes 7 to 11 show methods for the preparation of hydroxamic acid compounds using a solid phase support (P). 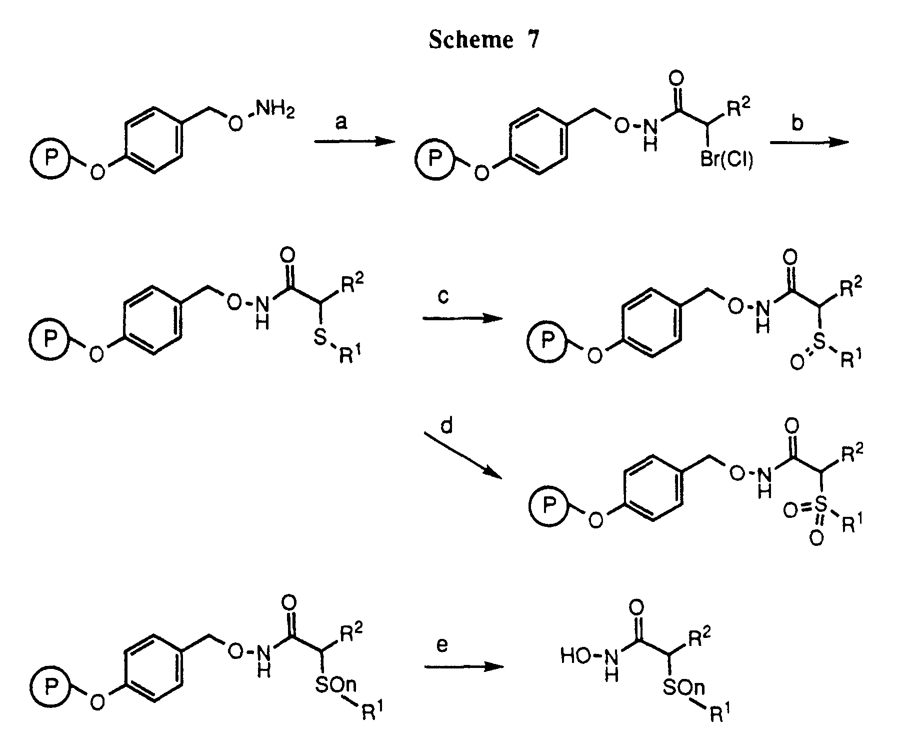 Reagents and Conditions: a) 2-Halo acid (3.0 eq.); 1-hydroxybenzotriazole hydrate (HOBt, 6.0 eq.); 1,3-diisopropylcarbodiimide (DIC, 4.0 eq.); DMF, 25°C; 2-16 hours. b) Thiol (5.0 eq.); sodium iodide (5.0 eq.); 1,8-diazabicyclo[5.4.0]undec-7-ene (DBU, 3.0 eq.); THF; 25°C; 12-16 hours. c) 70% *tert*-butylhydroperoxide (40 eq.); benzenesulfonic acid (2.0 eq.); DCM; 25°C; 12-24 hours. d) mCPBA (5.0 eq.); DCM; 25°C; 12-24 hours. e) TFA : DCM (1:1); 25°C; 1 hour.

The 4-*O*-methylhydroxylamine-phenoxymethyl-copoly(styrene-1%-divinylbenzene)-resin (hydroxylamine resin) may be coupled with a 2-halo acid to give the hydroxamate ester resin. The coupling reaction may be carried out in the presence of carbodiimide, such as DIC, in an inert solvent such as DMF at room temperature. The halogen group may be displaced with a thiol in the presence of a base, such as DBU, in an inert solvent such as THF at room temperature. The sulfide may be oxidized to the sulfoxide by reaction with an oxidizing agent such as *tert*-butylhydroperoxide in the presence of an acid catalyst such as benzenesulfonic acid, in an inert solvent such as DCM at room temperature. Alternatively, the sulfide may be oxidized to the sulfone by reaction with an oxidizing agent such as *meta*-chloroperoxybenzoic acid, in an inert solvent such as DCM at room temperature. The sulfide, sulfoxide, or sulfone may be treated with and acid, such as trifluoroacetic acid, in and inert solvent such as DCM to liberate the free hydroxamic acid.
Scheme 8 shows a method of preparing hydroxamic acids having alkoxy groups attached to the aromatic ring. 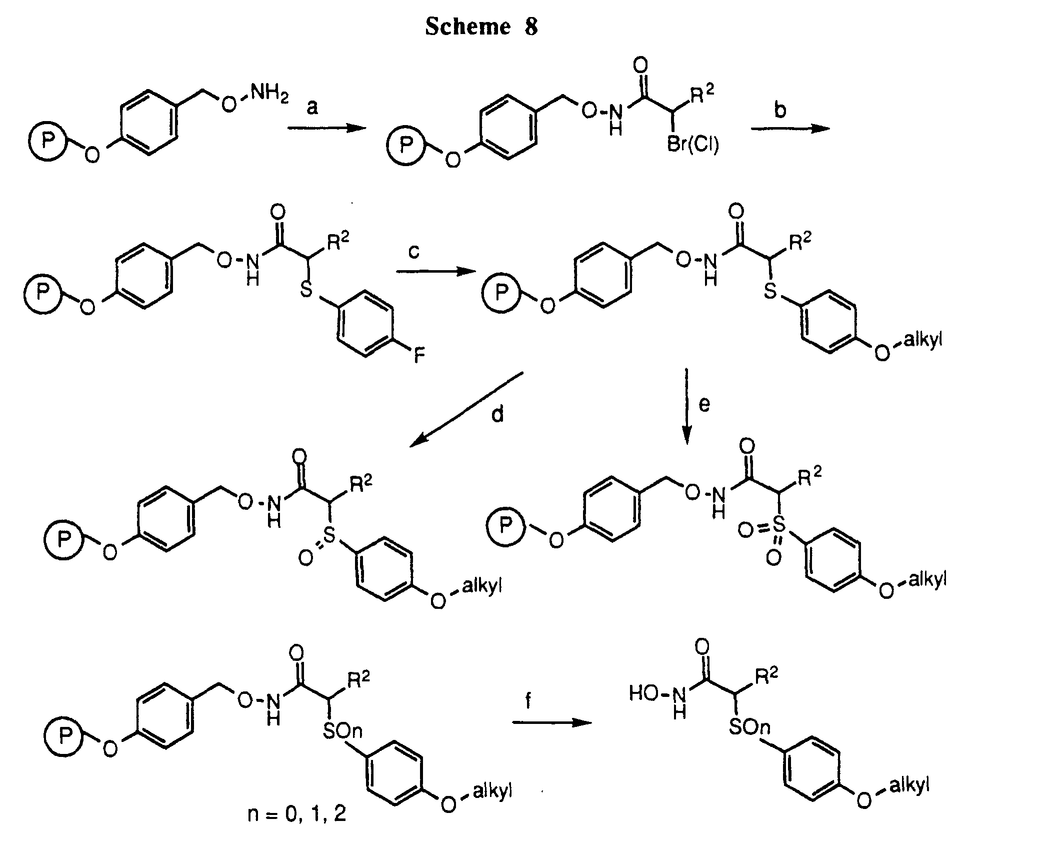 Reagents and Conditions: a) 2-Halo acid (3.0 eq.); 1-hydroxybenzotriazole hydrate (HOBt, 6.0 eq.); 1,3-diisopropylcarbodiimide (DIC, 4.0 eq.); DMF, 25°C; 2-16 hours. b) 4-Fluorobenzenethiol (5.0 eq.); sodium iodide (5.0 eq.); 1,8-diazabicyclo[5.4.0]undec-7-ene (DBU, 3.0 eq.); THF; 25°C; 12-16 hours. c) Alcohol (15.0 eq.); sodium hydride (15.0 eq.); DMF; 80°C; 15 hours. d) 70% *tert*-butylhydroperoxide (40 eq.); benzenesulfonic acid (2.0 eq.); DCM; 25°C; 12-24 hours. e) mCPBA (5.0 eq.); DCM; 25°C; 12-24 hours. f) TFA : DCM (1:1); 25°C; 1 hour.

The hydroxylamine resin may be coupled with the 2-halo acid and the halo group may be displaced by fluorobenzenethiol as previously described. The fluoro group may then be displaced with an alcohol in the presence of a base such as sodium hydride, in an inert solvent such as DMF at about 80°C. The alkoxybenzenesulfanyl hydroxamate ester may then be oxidized either to the corresponding sulfinyl or sulfonyl hydroxamate ester as previously described. The free hydroxamic acids may be liberated as previously described.
Scheme 9 shows a method of preparing 2-bisarylsulfanyl-, sulfinyl-, and sulfonylhydroxamic acids. 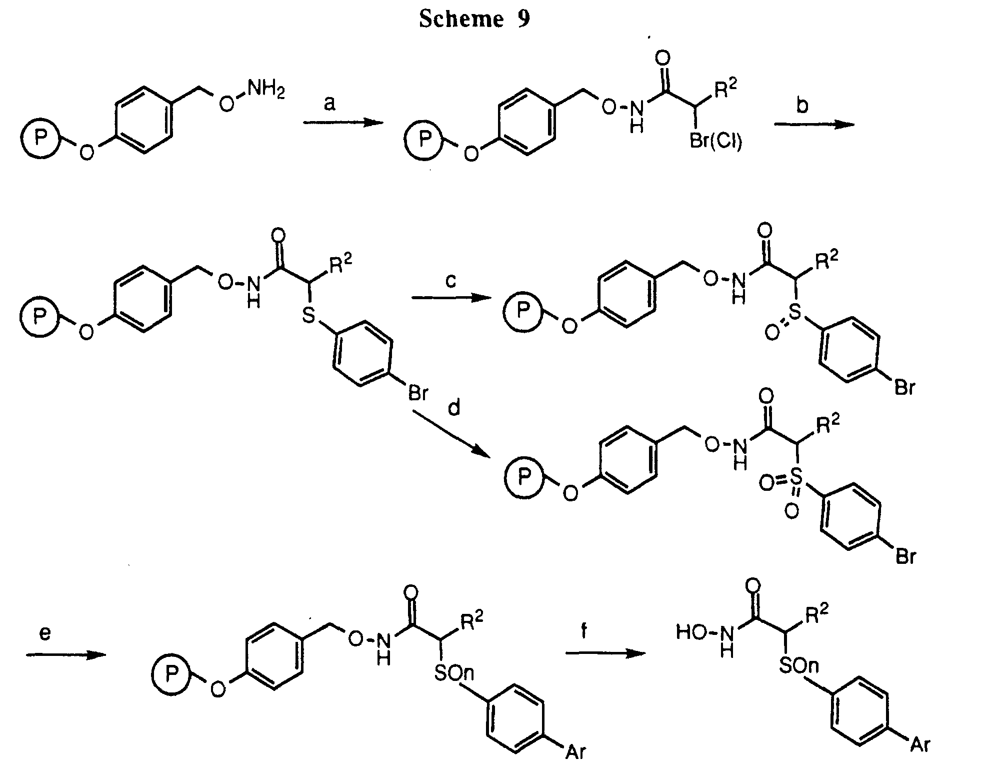 Reagents and Conditions: a) 2-Halo acid (3.0 eq.); 1-hydroxybenzotriazole hydrate (HOBt, 6.0 eq.); 1,3-diisopropylcarbodiimide (DIC, 4.0 eq.); DMF, 25°C; 2-16 hours. b) 4-Bromobenzenethiol (5.0 eq.); sodium iodide (5.0 eq.); 1,8-diazabicyclo[5.4.0]undec-7-ene (DBU, 3.0 eq.); THF; 25°C; 12-16 hours. c) 70% *tert*-butylhydroperoxide (40 eq.); benzenesulfonic acid (2.0 eq.); DCM; 25°C; 12-24 hours. d) *m*CPBA (5.0 eq.); DCM; 25°C; 12-24 hours. e) Arylboronic acid (2.0 eq.); tetrakis(triphenylphosphine) palladium(0) (0.1 eq.); 10% aqueous sodium carbonate (10.0 eq.); DME; ; 80°C; 8 hours. f) TFA : DCM (1:1); 25°C; 1 hour.

The hydroxylamine resin may be coupled with the 2-halo acid and the halo group may be displaced by bromobenzenethiol as previously described. The bromobenzenesulfanyl hydroxamate ester may then be oxidized either to the corresponding sulfinyl or sulfonyl hydroxamate ester as previously described. The bromo group may then be replaced with an aryl group by reaction with the arylboronic acid in the presence of a catalyst such as tetrakis(triphenylphosphine) palladium(0) and a base such as sodium carbonate, in an inert solvent such as DME at about 80°C. The free hydroxamic acids may be liberated as previously described.
Scheme 10 shows a method of preparing hydroxamic acids having amine groups attached to the aromatic ring. 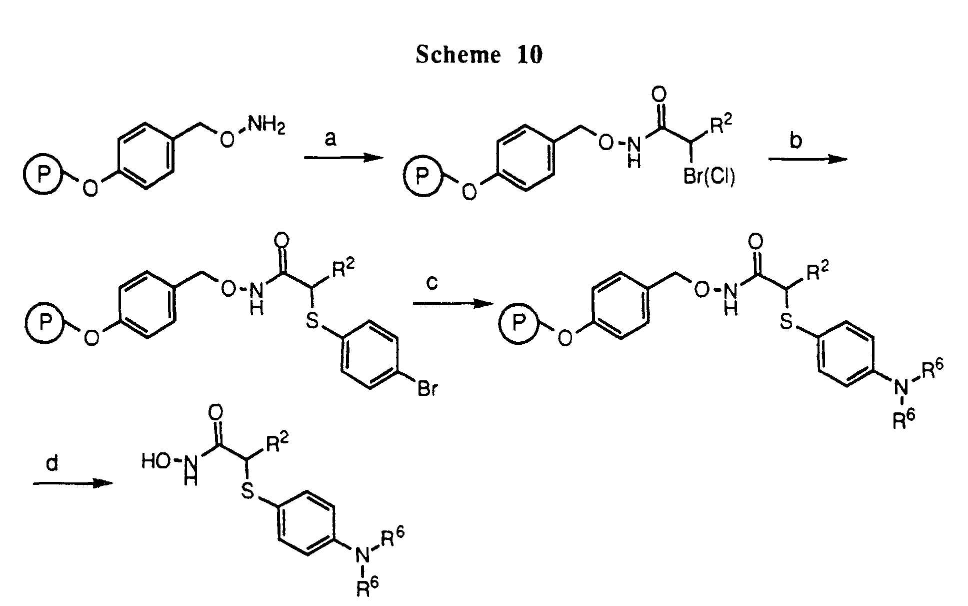 Reagents and Conditions: a) 2-Halo acid (3.0 eq.); 1-hydroxybenzotriazole hydrate (HOBt, 6.0 eq.); 1,3-diisopropylcarbodiimide (DIC, 4.0 eq.); DMF, 25°C; 2-16 hours. b) 4-Bromobenzenethiol (5.0 eq.); sodium iodide (5.0 eq.); 1,8-diazabicyclo[5.4.0]undec-7-ene (DBU, 3.0 eq.); THF; 25°C; 12-16 hours. c) Amine (20.0 eq.); tris(dibenzylideneacetone)-dipalladium(0) (0.2 eq.); (S)-(-)-2,2'-bis(diphenylphosphimo)-1,1'-binaphthyl ((S)-BINAP, 0.8 eq.); sodium *tert*-butoxide (18.0 eq.); dioxane; 80°C, 8 hours; d) TFA : DCM (1:1); 25°C; 1 hour.

The hydroxylamine resin may be coupled with the 2-halo acid and the halo group may be displaced by bromobenzenethiol as previously described. The bromo group may then be displaced with an amine in the presence of a catalyst such as tris(dibenzylideneacetone)-dipalladium(0) and a ligand such as (S)-BINAP and a base such as sodium tert-butoxide, in an inert solvent such as dioxane at about 80°C. The free hydroxamic acids may be liberated as previously described.
Scheme 11 shows a method of preparing hydroxamic acids having sulfonate groups attached to the aromatic ring. 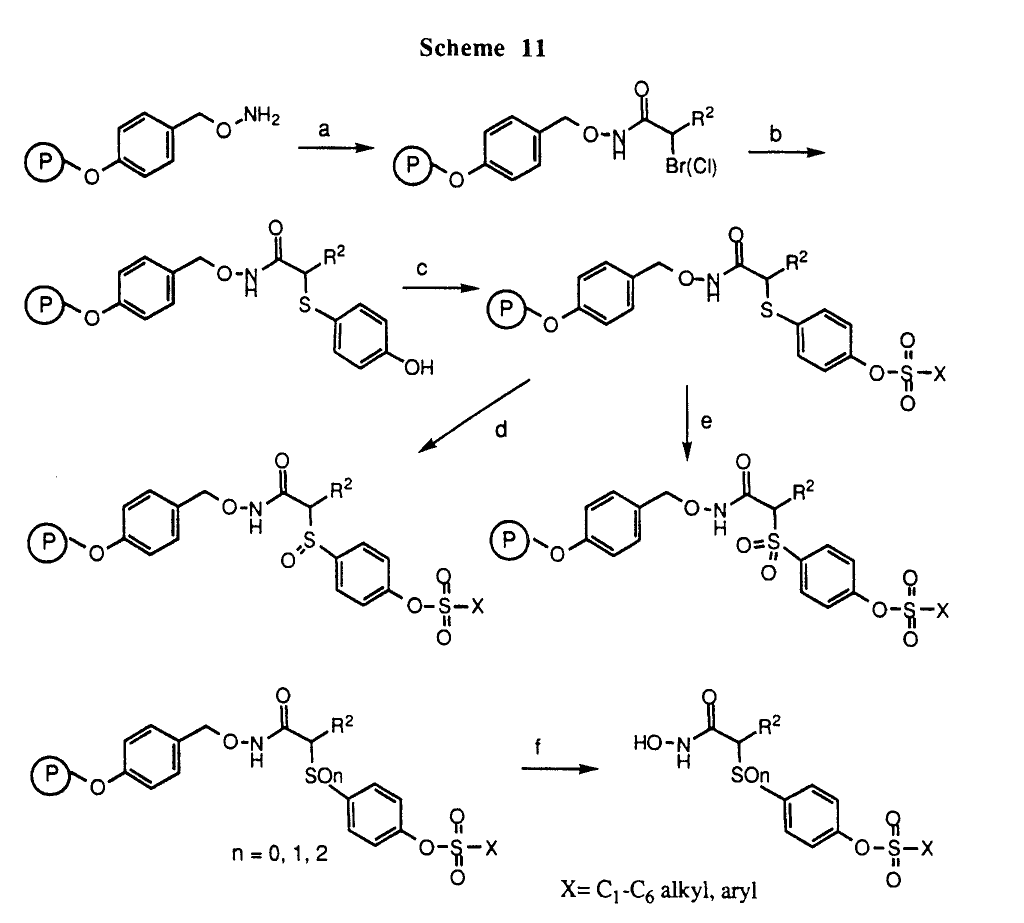 Reagents and Conditions: a) 2-Halo acid (3.0 eq.); 1-hydroxybenzotriazole hydrate (HOBt, 6.0 eq.); 1,3-diisopropylcarbodiimide (DIC, 4.0 eq.); DMF, 25°C; 2-16 hours. b) 4-Hydroxybenzenethiol (5.0 eq.); sodium iodide (5.0 eq.); 1,8-diazabicyclo[5.4.0]undec-7-ene (DBU, 3.0 eq.); THF; 25°C; 12-16 hours. c) Sulfonyl chloride (5.0 eq.); triethylamine (2.0 eq.); DCM; 25°C; 8 hours. d) 70% *tert*-butylhydroperoxide (40 eq.); benzenesulfonic acid (2.0 eq.); DCM; 25°C; 12-24 hours. e) mCPBA (5.0 eq.); DCM; 25°C; 12-24 hours. f) TFA : DCM (1:1); 25°C; 1 hour.

The hydroxylamine resin may be coupled with the 2-halo acid and the halo group may be displaced by hydroxybenzenethiol as previously described. The hydroxybenzenesulfanyl hydroxamate ester may then be oxidized either to the corresponding sulfinyl or sulfonyl hydroxamate ester as previously described. The hydroxy group may then be sulfonylated by reaction with a sulfonyl chloride in the presence of a base such as triethylamine, in an inert solvent such as DCM at about room temperature. The free hydroxamic acids may be liberated as previously described.
The following examples are presented to illustrate the invention. HPLC purity of compounds prepared by combinatorial procedures is presented as area percentage at a prescribed wavelength (%@ nm).

### Example 1

### 1-Benzyl-4-(4-methoxy-benzenesulfonyl)-piperidine-4-carboxylic Acid hydroxyamide

To a stirred solution of 4-methoxybenzenethiol (2.8 gm, 20 mmol) and anhydrous K₂CO₃ (10 gm, excess) in dry acetone (100 ml), α-bromo ethyl acetate (3.3 gm, 20 mmol) was added in a round bottom flask and the reaction mixture was heated at reflux for 8 hours with good stirring. At the end, the reaction mixture was allowed to cool and the potassium salts were filtered off and the reaction mixture was concentrated. The residue was extracted with chloroform and washed with H₂O and 0.5 N NaOH solution. The organic layer was further washed well with water, dried over MgSO₄, filtered and concentrated. (4-methoxy-phenylsulfanyl)-acetic acid ethyl ester was isolated as pale yellow oil. Yield: 4.4 g (100%); MS; 227 (M+H)⁺. To a stirred solution of 60% 3-chloroperoxybenzoic acid (14.0 gm, 40 mmol) in methylene chloride (100 ml) at 0°C, (4-methoxy-phenylsulfanyl)-acetic acid ethyl ester (4.4 g, 20 mmol) in CH₂Cl₂ (15 ml) was added slowly. The reaction mixture turned cloudy and was stirred at room temperature for 6 hours. The reaction mixture was then diluted with hexanes (300 ml) and stirred for 15 minutes. The solids were filtered off and Na₂SO₃ solution was added to the organic layer which was stirred for at least 3 hours before the mixture was extracted with CHCl₃ and washed with H₂O. The organic layer was dried over MgSO₄, filtered and concentrated and the colorless (4-methoxy-phenylsulfonyl)-acetic acid ethyl ester was isolated as an oil. Yield: 100%; MS: 259.1 (M+H)⁺.

To a stirred solution of diethanol amine (10.5 g, 100 mmol), and anhydrous K₂CO₃ (30 gm, excess) in dry acetone (250 ml), benzyl bromide (17.2 gm, 100 mmol) was added in a round bottom flask and the reaction mixture was heated at reflux for 8 hours with good stirring. At the end, the reaction mixture was allowed to cool and the potassium salts were filtered off and the reaction mixture was concentrated. The residue was extracted with chloroform and washed with H₂O. The organic layer was further washed well with water, dried over MgSO₄, filtered and concentrated. Colorless oil. Yield: 19.0 g, 97%; MS: 196 (M+H).

N-Benzyldiethanolamine (9.75 g, 50 mmol) was dissolved in saturated methanolic hydrochloric acid and concentrated to dryness. The hydrochloride thus formed was dissolved in methylene chloride (300 ml) and thionyl chloride (20 g, excess) was added dropwise and stirred at room temperature for 1 hr. At the end reaction mixture was concentrated to dryness and the product bis-(2-chloro-ethyl)-benzyl amine hydrochloride was used for further transformation with out any purification. Yield: 13.0 g, 97%; Mp: MS: 232 (M+H).

To a stirred solution of bis-(2-chloro-ethyl)-benzyl amine hydrochloride (6.6 g, 24.7 mmol), 18-Crown-6 (500 mg), and anhydrous K₂CO₃ (30 gm, excess) in dry acetone (250 ml), (4-methoxy-phenylsulfonyl)-acetic acid ethyl ester (6.12 gm, 24 mmol) was added in a round bottom flask and the reaction mixture was heated at reflux for 16 hours with good stirring. At the end, the reaction mixture was allowed to cool and the potassium salts were filtered off and the reaction mixture was concentrated. The residue was extracted with chloroform and washed with H₂O. The organic layer was further washed well with water, dried over MgSO₄, filtered and concentrated. The dark brown reaction mixture was purified by silica gel column chromatography by eluting it with 30% ethylacetate: hexane and the product 4-(4-Methoxybenzenesulfonyl)-1-benzyl-piperidine-4-carboxylic acid ethyl ester was isolated as Brown oil. Yield: 6.0 g, 60%; MS: 418 (M+H).

4-(4-Methoxy-benzenesulfonyl)-1-benzyl-piperidine-4-carboxylic acid ethyl ester (5.0 g, 11.9 mmol) was dissolved in MeOH/THF (1:1, 200 ml) and stirred at room temperature for 72 hrs. At the end reaction mixture was concentrated and the product was neutralized with con. HCl by dissolving it in water (200 ml). After the neutralization reaction mixture was concentrated to dryness. Ice cold water (100 ml) was added to the solid and filtered. The product 4-(4-Methoxy-benzenesulfonyl)-1-benzyl-piperidine-4-carboxylic acid was dried at 50°C and taken to next step with out any purification. Colorless solid. Yield: 3.2 g, 69% ; MS: 390 (M+H).

To a stirred solution of 4-(4-Methoxy-benzenesulfonyl)-1-benzyl-piperidine-4-carboxylic acid (2.0 g, 5.1 mmol) and DMF ( 2 drops) in CH₂Cl₂ (100 ml) at O°C, oxalyl chloride (1.0 gm, 8 mmol) was added in a drop-wise manner. After the addition, the reaction mixture was stirred at room temperature for 1 hour. Simultaneously, in a separate flask a mixture of hydroxylamine hydrochloride (2.0 gm, 29 mmol) and triethylamine (5 ml, excess) was stirred in THF:water (5:1, 30 ml) at O°C for 1 hour. At the end of 1 hour, the oxalyl chloride reaction mixture was concentrated and the pale yellow residue was dissolved in 10 ml of CH₂Cl₂ and added slowly to the hydroxylamine at O°C. The reaction mixture was stirred at room temperature for 24 hours and concentrated. The residue obtained was extracted with chloroform and washed well with water. The product obtained was purified by silica gel column chromatography and eluted with chloroform the product 4-(4-Methoxy-benzenesulfonyl)-1-benzyl-piperidine-4-carboxylic acid hydroxyamide was isolated as a colorless solid. mp 90-95 °C; Yield, 1.2 g, 48%; MS: 405 (M+H)⁺; 1H NMR (300 MHz, DMSO-d₆): δ 2.29 (m, 3H), 2.76-2.79 (m, 2H), 3.43 (m, 4H),4.30 (s, 2H), 7.14-7.17 (d,2H), 7.50-7.73 (m, 5H), 9.37 (s,1H), 10.53 (s,1H), 11.18 (s,1H).

### Example 2

### 4-(4-methoxy-benzenesulfonyl)-1-(3-methoxy-benzyl)-piperidine-4-carboxylic acid hydroxyamide

2-[(2-Hydroxy-ethyl)-(3-methoxy-benzyl)-amino]-ethanol was prepared according to the general method as outlined in example 1. Starting from diethanolamine (3.1 g, 29.5 mmol) and 3-methoxybenzyl chloride (5 g, 31.9 mmol). Yield 9.28 g, (99 %); yellow oil; MS: 226 (M+H).

3-Methoxybenzyl-bis-(2-chloro-ethyl)-amine was prepared according to the general method as outlined in example 1. Starting from 3-Methoxy-benzyl diethanolamine (4.4 g, 20 mmol). Yield 4.5 g (93 %); yellow solid mp 86 -88°C; MS: 263. (M+H)⁺.

4-(4-Methoxy-benzenesulfonyl)-1-(3-methoxy-benzyl)piperidine-4-carboxylic acid ethyl ester was prepared according to the general method as outlined in example 1. Starting from 4-(methoxy-benzenesulfonyl)-acetic acid ethyl ester (5.0 g, 22 mmol) and bis- (2-chloro ethyl)-(3-methoxy-benzyl)-amine (8.0 g, 23.5 mmol). Yield 2.4 g (24 %); low melting solid; MS: 447.9 (M+H)⁺.

4-(4-Methoxy-benzenesulfonyl)1-(3-methoxy-benzyl)-piperidine-4-carboxylic acid was prepared starting from 4-(4-Methoxy-benzenesulfonyl)-1-(3-methoxy-benzyl)piperidine-4-carboxylic acid ethyl ester (2.4g, 5.36 mmol) dissolve in methanol (30 mL) , 10 N sodium hydroxide (10 mL), tetrahydrohydrofuran (20 mL). The resulting reaction mixture was worked up as outlined in example 1. Yield 710 mg (32 %). white solid mp 199°C , MS: 419.9 (M+H)⁺.

Starting from 4-(4-methoxy-benzenesulfonyl)-1-(3-methoxy-benzyl)-piperidine-4-carboxylic acid (830 mg, 1.98 mmol) and following the procedure as outlined in example 1, 190 mg of 4-(4-methoxy-benzenesulfonyl)-1-(3-methoxy-benzyl)-piperidine-4-carboxylic acid hydroxamide was isolated as a white solid. mp 130°C; Yield 20.4%; MS: 435.0 (M+H)⁺; ¹H NMR (300 MHz, DMSO-d₆): δ 2.24-2.32 (m, 2H), 2.51(d, 2H), 2.73-2.83 (m, 2H), 3.37 (d, 2H), 3.76 (s, 3H), 3.88 (s, 3H), 4.32 (s, 2H), 7.01-7.77 (m,8H), 9.38 (s, 1H0, 10.1 (s, 1H).

### Example 3

### 1-(3,4-dichlorobenzyl) -4-(4-methoxy-benzenesulfonyl)-piperidine-4-carboxylic acid hydroxamide

2-[(2-Hydroxy-ethyl)-(3,4-dichloro-benzyl)-amino]-ethanol was prepared according to the general method as outlined in example 1. Starting from diethanolamine (4.84 g, 46 mmol) and 3,4-dichlorobenzyl chloride (9.0 g, 46 mmol). Yield 13.8 g (99 %); colorless oil; MS: 264.3 (M+H)⁺.

3,4-Dichlorobenzyl-bis-(2-chloro-ethyl)-amine was prepared according to the general method as outlined in example 1. Starting from 3,4-dichlorobenzyl diethanolamine (10.7 g, 41 mmol). Yield 99%; yellow solid mp 218-220°C; MS: 301.8 (M+H)⁺.

1-(3,4-Dichloro-benzyl)-4-(methoxy-benzenesulfonyl)-piperidine-4-carboxylic acid ethyl ester was prepared according to the general method as outlined in example 1. Starting from 4-(methoxy-benzenesulfonyl)-acetic acid ethyl ester (2.9 g, 11 mmol) and 3,4-dichlorobenzyl-bis(2-chloroethyl)-amine (3.4 g, 11 mmol). Yield 5.9g (60 %); brown oil; MS: 494.5 (M+H)⁺.

1-(3,4-Dichloro-benzyl)-4-(4-methoxy-benzenesulfulfonyl)-piperidine-4-carboxylic acid was prepared starting from 1-(3,4-dichloro-benzyl)-4-(methoxy-benzenesulfonyl)-piperidine-4-carboxylic acid ethyl ester (5.0 g, 10 mmol) dissolved in methanol (50 mL), 10 N sodium hydroxide (15 mL) and tetrahydrofuran (75 mL). The resulting reaction mixture was worked up as outlined in example 1. Yield 2.94 g (62 %), MS: 458.3 (M+H)⁺.

Starting from 1-(3,4-dichlorobenzyl)-4-(4-methoxy-benzenesulfonyl)-piperidine-4-carboxylic acid (2.67g, 5.8 mmol) and following the procedure as outlined in example 1, .2 g of 1-(3,4-dichlorobenzyl) -4-(4-methoxy-benzenesulfonyl)-piperidine-4-carboxylic acid hydroxamide was isolated as a white solid. mp 192-195°C; Yield 10%; MS 472.9 (M+H)⁺;
¹H NMR (300 MHz, DMSO-d₆): δ 2.20-2.28 (m, 2H), 2.76-2.79 (m, 2H), 3.43-3.44 (m, 4H), 4.30 (s, 2H), 7.14-7.17 (d, J=.030, 2H), 7.50-7.73 (d, J=.027, 1H), 7.65-7.68 (d, J=.029, 2H), 7.72-7.75 (d, J=.027, 2H), 7.87 (s, 1H), 9.37 (s, 1H), 10.53 (s, 1H), 11.18 (s, 1H).

### Example 4

### 4-(4-methoxy-benzenesulfonyl)-1-(4-methylbenzyl)-piperidine-4-carboxylic acid hydroxamide

2-[(2-Hydroxy-ethyl)-(4-methyl-benzyl)- amino]-ethanol was prepared according to the general method as outlined in example 1. Starting from diethanolamine (4.8 g, 46 mmol) and 4-methylbenzyl chloride (8.5 g, 46 mmol). Yield 9.8 g (99 %); MS: 209.9 (M+H)⁺.

4-Methylbenzyl-bis-(2-chloro-ethyl)-amine was prepared according to the general method as outlined in example 1. Starting from 4-methyl-benzyl diethanolamine (6 g, 20 mmol). Yield 5.2 g (84 %); yellow solid mp 145-147°C; MS: 245.9 (M+H)⁺.

4-(4-Methoxy-benzenesulfonyl)-1-(4-methyl-benzyl)piperidine-4-carboxylic acid ethyl ester was prepared according to the general method as outlined in example 1. Starting from 4-(methoxybenzenesulfonyl)-acetic acid ethyl ester (7.0 g, 27 mmol) and 4-methyl-bis-(2-chloro-ethyl)-amine (5.0 g, 17 mmol). Yield 4.64 g (63 %); low melting solid; MS: 431.9 (M+H)⁺.

4-(4-Methoxy-benzenesulfonyl)1-(4-methyl-benzyl)-piperidine-4-carboxylic acid was prepared starting from 4-(4-methoxy-benzenesulfonyl)-piperidine-4-carboxylic acid ethyl ester (4.3g, 9.9 mmol) dissolve in methanol (30 mL) , 10 N sodium hydroxide (10 mL), tetrahydrohydrofuran (20 mL). The resulting reaction mixture was worked up as outlined in example 1. Yield 1.6 g (40 %). white solid mp 207-208°C , MS: 404.3 (M+H)⁺.

Starting from 4-(4-methoxy-benzenesulfonyl)-1-(4-methylbenzyl)-piperidine-4-carboxylic acid (1.59g, 3.9 mmol) and following the procedure as outlined in example 1, .505 g of 4-(4-methoxy-benzenesulfonyl)-1-(4-methylbenzyl)-piperidine-4-carboxylic acid hydroxamide was isolated as a white solid. mp 176-177°C; Yield 32%; MS: 419.0 (M+H)⁺; ¹H NMR (300 MHz, DMSO-d₆): δ 2.24-2.32 (m, 2H), 2.51(t, 3H), 2.73-2.80 (m, 2H), 3.35-3.50 (m, 4H), 3.87 (s, 3H), 4.24 (s, 2H), 7.13-7.17 (d, J=.039, 2H), 7.23-7.60 (d, J=.036, 2H), 7.38-7.41 (d, J=.025, 2H), 7.65-7.68 (d, J=.039, 2H).

### Example 5

### 4-(4-methoxy-benzene-sulfonyl)-1-napthalene-2-yl-methylpiperidine-4-carboxylic acid hydroxamide

2-[(2-Hydroxy-ethyl)-(2-napthyl-2-ylmethyl)-amino]-ethanol was prepared according to the general method as outlined in example 1. Starting from diethanolamine (6.18 g, 59 mmol) and 2-(bromomethyl)napthalene (10 g, 45 mmol). Yield 12.7 g (96 %); yellow solid mp 162-164°C; MS: 246.0 (M+H)⁺.

2-Napthyl-2-ylmethyl-bis-(2-chloro-ethyl)-amine was prepared according to the general method as outlined in example 1. Starting from 2-napthyl-ylmethyl-diethanol amine (10 g, 36 mmol). Yield 9.1 g (79 %); brown solid mp 124-126°C; MS: 281.9 (M+H)⁺.

4-(4-Methoxy-benzenesulfonyl)-napthalene-ylmethyl-piperidine-4-carboxylic acid ethyl ester was prepared according to the general method as outlined in example 1. Starting from 4-(methoxy-benzenesulfonyl)-acetic acid ethyl ester (8.4 g, 32 mmol) and 1-napthalene-ylmethyl-bis-(2-chloro-ethyl)-amine ((8.6 g, 27 mmol). Yield 6.5 g (52 %); low melting solid; MS: 440.0 (M+H)⁺.

4-(4-Methoxy-benzenesulfonyl)-1-napthalene-ylmethyl-piperidine-4-carboxylic acid was prepared starting from 4-(4-methoxy-benzenesulfonyl)-napthalene-ylmethyl-piperidine-4-carboxylic acid ethyl ester (6.3g, 13 mmol) dissolved in methanol (30 mL), 10 N sodium hydroxide (30 mL) and tetrahydrofuran (30 mL). The resulting reaction mixture was worked up as outlined in example 1. Yield 2.3 g (36 %). yellow solid mp 226-228°C, MS: 440.0 (M+H)⁺.

Starting from 4-(4-methoxy-benzenesulfonyl)-1-napthalene-2-yl-methylpiperidine-4-carboxylic acid (2.18g, 5.0 mmol) and following the procedure as outlined in example 1, .753 g of 4-(4-methoxy-benzene-sulfonyl)-1-napthalene-2-yl-methylpiperidine-4-carboxylic acid hydroxamide was isolated as a off white solid. mp 168-170°C; Yield 31 %; MS 455.0 (M+H)⁺; ¹H NMR (300 MHz, DMSO-d₆): δ 2.29-2.33 (m, 2H), 2.86-2.89 (m, 2H), 3.42-3.46 (m, 4H), 3.85 (s, 3H), 4.46 (s, 2H), 7.13-7.16 (d, J=.030, 2H), 7.56-7.64 (m, 3H), 7.65-7.68 (d, J=.030, 2H), 7.98-8.00 (m, 3H), 8.21 (s, 1H), 10.70 (s, 1H), 11.20 (s, 1H).

### Example 6

### 1-Biphenyl-4-ylmethyl-4-(4-methoxy-benzenesulfonyl)piperidine-4-carboxylic acid hydroxamide

2-[(2-Hydroxy-ethyl)-(1-biphenyl-4-ylmethyl))-amino]-ethanol was prepared according to the general method as outlined in example 1. Starting from diethanol amine (5.2 g, 49 mmol) and 4-(chloromethyl)biphenyl (10 g, 49 mmol). Yield 9.98 g (66 %); white solid mp 160-162°C; MS: 271.9 (M+H)⁺. This was converted to the dichloride as outlined in example 1

1-Biphenyl-4-ylmethyl-4-(4-methoxy-benzenesulfonyl)-piperidine-4-carboxylic acid ethyl ester was prepared according to the general method as outlined in example 1. Starting from 4-(methoxy-benzenesulfonyl)-acetic acid ethyl ester (2.85 g, 11 mmol) and 1-biphenyl-4-ylmethyl-bis-(2-chloro-ethyl)-amine (3.4 g, 11 mmol). Yield 2.1 g, (39 %); beige solid, mp 176-178°C, MS: 494.1 (M+H)⁺.

1-Biphenyl-4-ylmethyl-4-(4-methoxy-benzenesulfonyl)-piperidine-4-carboxylic acid was prepared starting from 1-biphenyl-4ylmethyl-(4-methoxy-benzenesulfonyl)-piperidine-4-carboxylic acid ethyl ester (5.7g, 12 mmol) dissolved in ethanol (20 mL), tetrahydrofuran (20 mL) and 10 N sodium hydroxide (10 mL). The resulting reaction mixture was worked up as outlined in example 1. Yield 2.1g (39% ) MS: 465.8 (M+H)⁺.

Starting from 1-biphenyl-4-ylmethyl-4-(4-methoxy-benzenesulfonyl)-piperidine-4-carboxylic acid (1.0g, 2.2 mmol and following the procedure as outlined in example 1, .132g of 1-biphenyl-4-ylmethyl-4-(4-methoxy-benzenesulfonyl)piperidine-4-carboxylic acid hydroxamide was isolated as a tan solid. mp168°C; Yield 20%; MS: 440.9 (M+H)⁺; ¹H NMR (300 MHz, DMSO-d₆): δ 2.30-2.35 (m, 2H), 2.83-2.87 (m, 2H), 3.35-3.5 (m, 4H), 3.87 (s, 3H), 7.15-7.721 (d, J=.059 Hz, 2H), 7.49-7.65 (m, 5H), 7.68-7.74 (d, J=.06 Hz, 2H), 9.3 (s, 1H), 10.3 (s, 1H), 11.15 (s, 1H).

### Example 7

### 4-(4-methoxy-benzene-sulfonyl)-1-(3-methyl-but-2-enyl)piperidine-4-carboxylic acid hydroxamide

2-[(2-Hydroxy-ethyl)-1-(3-methyl-but-2-enyl)-amino]-ethanol was prepared according to the general method as outlined in example 1. Starting from diethanol amine (4.1 g, 39 mmol) and 4-bromo-2-methyl-butene (6.0 g, 40 mmol). Yield ( 98 %); brown oil; MS: 173.8 (M+H)⁺.

1-(3-methyl-but-2-enyl)]-bis-(2-chloro-ethyl)-amine was prepared according to the general method as outlined in example 1. Starting from 2-[(2-hydroxy-ethyl)-1-(3-methyl-but-2-enyl)-amino]-ethanol (10.4g, 50 mmol). Yield 10.5g (99%); brown solid; MS: 210.3 (M+H) 4-(4-Methoxy-benzenesulfonyl)-1-(3-methyl-but-2-enyl)-piperidine-4-carboxylic acid ethyl ester was prepared according to the general method as outlined in example 1. Starting from 4-(methoxy-benzenesulfonyl)-acetic acid ethyl ester (11.32 g, 44 mmol) and 3-methyl-but-2-enyl)-bis-(2-chloroethyl)-amine (10.4 g, 50 mmol). Yield 6.2 g (36 %); brown oil; MS: 395.6 (M+H)⁺.

4-(4-Methoxy-benzenesulfonyl)-1-(3-methyl-but-2-enyl)-piperidine-4-carboxylic acid was prepared starting from 4-(4-methoxy-benzenesulfonyl)-1-(3-methyl-but-2-enyl)-piperidine-4-carboxylic acid ethyl ester (6.2g, 16 mmol) dissolved in ethanol (15 mL), 10 N sodium hydroxide (10 mL) and tetrahydrofuran (75 mL). The resulting reaction mixture was worked up as outlined in example 1. Yield 1.2 g (21 %). brown solid mp 196-197°C, MS: 367.9 (M+H)⁺.

Starting from 4-(4-methoxy-benzenesulfonyl)-1-(3-methyl-but-2-enyl)-piperidine-4-carboxylic acid (1.0g. 3.0 mmol) and following the procedure as outlined in example 1, .110 mg of 4-(4-methoxy-benzene-sulfonyl)-1-(3-methyl-but-2-enyl)piperidine-4-carboxylic acid hydroxamide was isolated as a yellow solid. mp 142-145°C; Yield 12%; MS: 382.9 (M+H)⁺; ¹H NMR (300 MHz, DMSO-d₆): δ 1.67 (s, 3H), 1.79 (s, 3H), 2.18-2.23 (m, 2H), 2.66-2.73 (m, 2 H), 3.37-3.46 (m, 2H), 3.67-3.69 (m, 2H), 5.19-5.24 (m, 1H), 7.15-7.18 (d, J=.03, 2H), 7.67-7.70 (d, J=.030, 2H), 9.34 (s, 1H), 9.88 (s, 1H), 11.15 (s, 1H).

### Example 8

### 1-(4-Bromo-benzyl)-4-(4-methoxy-benzenesulfonyl)-piperidine-4-carboxylic acid hydroxyamide

2-[(4-Bromobenzyl)-(2-hydroxy-ethyl)-amino]-ethanol was prepared according to the general method as outlined in example 1. Starting from diethanolamine (22.5 g, 150 mmol). and 4-bromobenzyl bromide (25 g, 100 mmol). Yield 33.66g, (99%); yellow oil; MS: 273.8 (M+H)⁺.

(4-Bromo-benzyl)-bis-(2-chloro-ethyl)-amine was prepared according to the general method as outlined in example 1. Starting from 2-[(4-bromobenzyl)-(2-hydroxy-ethyl)-amino]-ethanol (33.28 g, 122 mmol). Yield 47 g, (99%); brown solid; mp 125 °C; MS: 309.8 (M+H)⁺.

1-(4-Bromo-benzyl)-4-(4-methoxy-benzenesulfonyl)-piperidine-4-carboxylic acid ethyl ester was prepared according to the general method as outlined in example 1. Starting from 4-(methoxy-benzenesulfonyl) acetic acid ethyl ester (8.6 g, 33.5 mmol) and (4-bromo-benzyl)-bis-(2-chloro-ethyl)-amine (13.3 g, 38.6 mmol). Yield 17 g (44%); brown oil; MS: 497.8 (M+H)⁺.

1-(4-Bromo-benzyl)-4-(4-methoxy-benzenesulfonyl)-piperidine-4-carboxylic acid was prepared starting from 1-(4-bromo-benzyl)-4-(4-methoxy-benzenesulfonyl)-piperidine-4-carboxylic acid ethyl ester (16.5 g, 33.3 mmol) dissolved in THF:methanol 3:1 and 10 N NaOH (20 ml). The resulting reaction mixture was worked up as outlined in example 1. Yield 6.18 g (40%); tan solid; mp 215 °C; MS: 469.7 (M+H)⁺.

Starting from 1-(4-Bromo-benzyl)-4-(4-methoxy-benzenesulfonyl)-piperidine-4-carboxylic acid (1.95 g, 4.2 mmol) and following the procedure as outlined in example 1, 1.29 g of 1-(4-bromobenzyl)-4-(4-methoxy-benzenesulfonyl)-piperidine-4-carboxylic acid hydroxyamide was isolated as an off white solid. Yield 60%; mp 180 °C; MS: 484.7 (M+H)⁺; ¹H NMR (300 M□z, DMSO-d₆): δ 2.18-2.29 (m, 2H), 2.46 (d, 2H), 2.74-2.89 (m, 2H), 3.39 (d, 2H), 3.87 (s, 3H), 4.28 (s, 2H), 7.18 (d, J = 17 Hz, 2H), 7.49 (d, J = 8.1 Hz, 2H), 7.65- 7.68 (m, 4H), 9.37 (s, 1H), 10.5 (s, 1H).

### Example 9

### 4-(4-methoxy-benzenesulfonyl)-1-(3-phenyl-propyl)-piperidine-4-carboxylic acid hydroxyamide

2-[(2-Hydroxy-ethyl)-(3-phenyl-propyl)-amino]-ethanol was prepared according to the general method as outlined in example 1. Starting from diethanolamine (15.8 g, 151 mmol). and 1-bromo-3-phenylpropane (20 g, 101 mmol). Yield 21.31 g, (95%); yellow oil; MS: 223.9 (M+H)⁺.

Bis-(2-Chloro-ethyl)-(3-phenyl-propyl)-amine was prepared according to the general method as outlined in example 1. Starting from 2-[(2-hydroxy-ethyl)-(3-phenyl-propyl)-amino]-ethanol (20.32 g, 90.7 mmol). Yield 24.9 g (92%); brown oil; MS: 259.8 (M+H)⁺.

4-(4-Methoxy-benzenesulfonyl)-1-(3-phenyl-propyl)-piperidine-4-carboxylic acid ethyl ester was prepared according to the general method as outlined in example 1. Starting from from 4-(methoxy-benzenesulfonyl) acetic acid ethyl ester (12 g, 46.5 mmol) and bis-(2-chloro-ethyl)-(3-phenyl-propyl)-amine (24.8 g, 93.8 mmol). Yield 11.24 g (54%); brown oil; MS: 446 (M+H)⁺.

4-(4-Methoxy-benzenesulfonyl)-1-(3-phenyl-propyl)-piperidine-4-carboxylic acid was prepared starting from 4-(4-Methoxy-benzenesulfonyl)-1-(3-phenyl-propyl)-piperidine-4-carboxylic acid ethyl ester (10.74 g, 24.13 mmol) dissolved in THF:methanol 3:1 and 10 N NaOH (40 ml). The resulting reaction mixture was worked up as outlined in example 1. Yield 4.67 g (47%); off white powder; mp 203 °C; MS: 418.2 (M+H)⁺.

Starting from 4-(4-methoxy-benzenesulfonyl)-1-(3-phenyl-propyl)-piperidine-4-carboxylic acid (4.37 g, 10.4 mmol) and following the procedure as outlined in example 1, 1.64 g of 4-(4-methoxy-benzenesulfonyl)-1-(3-phenyl-propyl)-piperidine-4-carboxylic acid hydroxyamide was isolated as an off white solid. Yield 37%; mp 143 °C; MS: 432.9 (M+H)⁺; ¹H NMR (300 MHz, DMSO-d₆): δ 1.92-1.97 (m, 2H), 2.18-2.29 (m, 2H), 2.47 (d, 2H), 2.58 (t, J = 7.7 Hz, 2H), 2.6-2.73 (m, 2H), 3.0-3.06 (m, 2H), 3.60 (d, J = 12.3 Hz, 2H), 3.87 (s, 2H), 7.15-7.30 (m, 7 H), 7.68, (d, J = 9 Hz, 2H), 9.3 (s, 1H), 10.1 (s, 1H).

### Example 10

### 1-Tert-butyl-4-(4-methoxy-benzenesulfonyl)-piperidine-4-carboxylic acid hydroxyamide

tert-Butyl-bis-(2-chloro-ethyl)-amine was prepared according to the general method as outlined in example 1. Starting from 1-tert-butyl-diethanolamine (6 g, 37.2 mmol). Yield 11.15 g, (99%); white solid; MS: 197.8 (M+H)⁺.

1-tert-Butyl-4-(4-methoxy-benzenesulfonyl)-piperidine-4-carboxylic acid ethyl ester was prepared according to the general method as outlined in example 1. Starting from 4-(methoxybenzenesulfonyl) acetic acid ethyl ester (10 g, 38.76 mmol) and tert-butyl-bis-(2-chloro-ethyl)-amine (5.25 g, 22.53 mmol). Yield 5.37 g, (62%); brown oil; MS: 384 (M+H)⁺.

1-tert-Butyl-4-(4-methoxy-benzenesulfonyl)-piperidine-4-carboxylic acid was prepared starting from 1-tert-butyl-4-(4-methoxy-benzenesulfonyl)-piperidine-4-carboxylic acid ethyl ester (5.37 g 14 mmol) dissolved in methanol (300 ml) and 10 N NaOH (23 ml). The resulting reaction mixture was worked up as outlined in example 1. Yield 1.52 g (30.6%); white powder; mp 204 °C; MS: 356 (M+H)⁺.

Starting from 1-tert-butyl-4-(4-methoxy-benzenesulfonyl)-piperidine-4-carboxylic acid (320 mg, 0.9 mmol) and following the procedure as outlined in example 1, 190 mg of 1-tert-butyl-4-(4-methoxy-benzenesulfonyl)-piperidine-4-carboxylic acid hydroxyamide was isolated as a green solid. Yield 52%; mp 40 °C; MS: 371.1 (M+H)⁺; ¹H NMR (300 MHz, DMSO-d₆): δ 1.29 (s, 9H), 1.54 (m, 2H), 1.66 (m, 2H), 2.39 (m, 2H), 2.98 (m, 2H), 3.88 (s, 3H), 7.18 (d, 2H), 7.67 (d, 2H).

### Example 11

### 1-Butyl-4-(4-methoxy-benzenesulfonyl)-piperidine-4-carboxylic acid hydroxyamide

Butyl-bis-(2-chloro-ethyl)-amine was prepared according to the general method as outlined in example 1. Starting from N-butyldiethanolamine (6 g, 37.2 mmol). Yield 11.3 g, (99%); white powder; mp 165 °C; MS: 197.9 (M+H)⁺.

1-Butyl-4-(4-methoxy-benzenesulfonyl)-piperidine-4-carboxylic acid ethyl ester was prepared according to the general method as outlined in example 1. Starting from 4-(methoxybenzenesulfonyl) acetic acid ethyl ester (5 g, 19.38 mmol) and butyl-bis-(2-chloro-ethyl)-amine (4.52 g, 19.38 mmol). Yield 6.86 g, (93%); brown oil; MS: 384 (M+H)⁺.

1-Butyl-4-(4-methoxy-benzenesulfonyl)-piperidine-4-carboxylic acid was prepared starting from 1-butyl-4-(4-methoxy-benzenesulfonyl)-piperidine-4-carboxylic acid ethyl ester (6.42 g 16.8 mmol) dissolved in methanol (200 ml) and 10 N NaOH (20 ml). The resulting reaction mixture was worked up as outlined in example 1. Yield 1.6 g (27%); white powder; mp 206 °C; MS: 356.4 (M+H)⁺.

Starting from 1-butyl-4-(4-methoxy-benzenesulfonyl)-piperidine-4-carboxylic acid (1.51 g, 4.3 mmol) and following the procedure as outlined in example 1, 200 mg of 1-butyl-4-(4-methoxybenzenesulfonyl)-piperidine-4-carboxylic acid hydroxyamide was isolated as an off white solid. Yield 9.3%; mp 75 °C; MS: 371.1 (M+H)⁺; ¹H NMR (300 MHz, DMSO-d₆): δ 0.87 (t, J = 7.2 Hz, 3H), 1.27 (m, 2H), 1.59 (m, 2H), 2.27 (m, 2H), 2.45 (m, 2H), 2.50 (m, 2H), 2.65 (m, 2H), 2.97 (m, 2H) 3.88 (s, 3H), 7.18 (d, 2H), 7.69 (d, 2H).

### Example 12

### 1-Cyclooctyl-4-(4-methoxy-benzenesulfonyl)-piperidine-4-carboxylic acid hydroxyamide

Cyclooctyl-bis-(2-chloro-ethyl)-amine was prepared according to the general method as outlined in example 1. Starting from N-cyclooctyldiethanolamine (6 g, 28 mmol). Yield 10 g, (99%); off white solid; mp 158 °C; MS: 251.9 (M+H)⁺.

1-Cyclooctyl-4-(4-methoxy-benzenesulfonyl)-piperidine-4-carboxylic acid ethyl ester was prepared according to the general method as outlined in example 1. Starting from 4-(methoxybenzenesulfonyl) acetic acid ethyl ester (5 g, 19.4 mmol) and cyclooctyl-bis-(2-chloro-ethyl)-amine (5.57 g, 19.4 mmol). Yield 8.2 g, (96%); brown oil; MS: 438 (M+H)⁺.

1-Cyclooctyl-4-(4-methoxy-benzenesulfonyl)-piperidine-4-carboxylic acid was prepared starting from 1-cyclooctyl-4-(4-methoxy-benzenesulfonyl)-piperidine-4-carboxylic acid ethyl ester (8 g, 18.3 mmol) dissolved in methanol (200 ml) and 10 N NaOH (25 ml). The resulting reaction mixture was worked up as outlined in example 1. Yield 2.36 g (32%); white powder; mp 180 °C; MS: 410 (M+H)⁺.

Starting from 1-Cyclooctyl-4-(4-methoxy-benzenesulfonyl)-piperidine-4-carboxylic acid (2.26 g, 5.53 mmol) and following the procedure as outlined in example 1, 570 mg of 1-cyclooctyl-4-(4-methoxy-benzenesulfonyl)-piperidine-4-carboxylic acid hydroxyamide was isolated as a white powder. Yield 22%; mp >200 °C; MS: 425 (M+H)⁺; ¹H NMR (300 MHz, DMSO-d₆): δ 1.42-1.66 (m, 14H), 1.83 (m, 2H), 2.33 (m, 2H), 2.67 (m, 2H), 3.30-3.51 (m, 3H) 3.88 (s, 3H) 7.17 (d, 2H), 7.66 (d, 2H).

### Example 13

### 1-Ethyl-4-(4-methoxy-benzenesulfonyl)-piperidine-4-carboxylic acid hydroxyamide

1-Ethyl-4-(4-methoxy-benzenesulfonyl)-piperidine-4-carboxylic acid ethyl ester was prepared according to the general method as outlined in example 1. Starting from 4-(methoxybenzenesulfonyl) acetic acid ethyl ester (3 g, 11.6 mmol) and ethyl-bis-(2-chloro-ethyl)-amine (2.39g, 11.6 mmol). Yield 3.09 g, (75%); low melting brown solid; MS: 356 (M+H)⁺.

1-Ethyl-4-(4-methoxy-benzenesulfonyl)-piperidine-4-carboxylic acid was prepared starting from 1-ethyl-4-(4-methoxy-benzenesulfonyl)-piperidine-4-carboxylic acid ethyl ester (2.42 g, 6.8 mmol) dissolved in methanol (100 ml) and 10 N NaOH (15 ml). The resulting reaction mixture was worked up as outlined in example 1. Yield 1.29 g (58%); white solid; mp 209 °C; MS: 328 (M+H)⁺.

Starting from 1-ethyl-4-(4-methoxy-benzenesulfonyl)-piperidine-4-carboxylic acid (1.23 g, 3.76 mmol) and following the procedure as outlined in example 1, 1.02 g of 1-ethyl-4-(4-methoxybenzenesulfonyl)-piperidine-4-carboxylic acid hydroxyamide was isolated as an off white powder. Yield 80%; mp 85 °C; MS: 343 (M+H)⁺; ¹H NMR (300 MHz, DMSO-d₆): δ 0.926 (t, J =7.1 Hz, 3H), 1.68-1.89 (m, 4H), 2.05-2.24 (m, 4H), 2.73 (q, 2H), 3.85 (s, 3H), 7.07 (d, 2H), 7.64 (d, 2H).

### Example 14

### 1-Isopropyl-4-(4-methoxy-benzenesulfonyl)-piperidine-4-carboxylic acid hydroxyamide

1-Isopropyl-4-(4-methoxy-benzenesulfonyl)-piperidine-4-carboxylic acid ethyl ester was prepared according to the general method as outlined in example 1. Starting from 4-(methoxybenzenesulfonyl) acetic acid ethyl ester (5.7 g, 22.2 mmol) and isopropyl-bis-(2-chloro-ethyl)-amine (4.9 g, 22.2 mmol). Yield 5.64 g, (68%); low melting brown solid; MS: 370 (M+H)⁺.

1-Isopropyl-4-(4-methoxy-benzenesulfonyl)-piperidine-4-carboxylic acid was prepared starting from 1-isopropyl-4-(4-methoxy-benzenesulfonyl)-piperidine-4-carboxylic acid ethyl ester (5.6 g, 15.2 mmol) dissolved in methanol (75 ml) and 10 N NaOH (25 ml). The resulting reaction mixture was worked up as outlined in example 1. Yield 2.18 g (42%); white powder; mp 204 °C; MS: 341.9 (M+H)⁺.

Starting from 1-isopropyl-4-(4-methoxy-benzenesulfonyl)-piperidine-4-carboxylic acid (2.13 g, 6.25 mmol) and following the procedure as outlined in example 1, 590 mg of 1-isopropyl-4-(4-methoxy-benzenesulfonyl)-piperidine-4-carboxylic acid hydroxyamide was isolated as a white powder. Yield 2.4%; mp 75 °C; MS: 357 (M+H)⁺; ¹H NMR (300 MHz, DMSO-d₆): δ 1.21 (d, J = 6.6 Hz, 6H), 2.33-3.53 (m, 9H), 3.88 (s, 3H), 7.16 (d, 2H), 7.66 (d, 2H).

### Example 15

### 1-Methyl-4-(4-methoxy-benzenesulfonyl)-piperidine-4-carboxylic acid hydroxyamide

1-Methyl-4-(4-methoxy-benzenesulfonyl)-piperidine-4-carboxylic acid ethyl ester was prepared according to the general method as outlined in example 1. Starting from 4-(methoxybenzenesulfonyl) acetic acid ethyl ester (3 g, 11.6 mmol) and methyl-bis-(2-chloro-ethyl)-amine (2.2g, 11.6 mmol). Yield 3.09 g, (75%); low melting brown solid; MS: 342 (M+H)⁺.

1-Methyl-4-(4-methoxy-benzenesulfonyl)-piperidine-4-carboxylic acid was prepared starting from 1-methyl-4-(4-methoxy-benzenesulfonyl)-piperidine-4-carboxylic acid ethyl ester (8.7 g, 25.6 mmol) dissolved in methanol (300 ml) and 10 N NaOH (35 ml). The resulting reaction mixture was worked up as outlined in example 1. Yield 3.23 g (41%); white solid; mp 204 °C; MS: 313.9 (M+H)⁺.

Starting from 1-methyl-4-(4-methoxy-benzenesulfonyl)-piperidine-4-carboxylic acid (2.0 g, 6.38 mmol) and following the procedure as outlined in example 1, 1.10 g of 1-methyl-4-(4-methoxybenzenesulfonyl)-piperidine-4-carboxylic acid hydroxyamide was isolated as a yellow powder. Yield 53%; mp 89 °C; MS: 329 (M+H)⁺; ¹H NMR (300 MHz, DMSO-d₆): δ 1.67-1.76 (m, 2H), 1.85-1.96 (m, 2H), 2.05 (s, 3H), 2.17 (d, J = 11.4 Hz, 2H), 2.57 (d, J = 10.4 Hz, 2H) 3.83 (s, 3H), 7.02 (d, 2H), 7.62 (d, 2H).

### Example 16

### 1-Benzyl-4-(4-butoxy-benzenesulfonyl)-piperidine-4-carboxylic acid hydroxyamide

1-Benzyl-4-(4-butoxy-benzenesulfonyl)-piperidine-4-carboxylic acid ethyl ester was prepared according to the general method as outlined in example 1. Starting from from 4-(butoxybenzenesulfonyl) acetic acid ethyl ester (6 g, 20 mmol) and bis-(2-chloro-ethyl)-benzylamine (10 g, 30 mmol). Yield 5.15 g (56%); yellow oil; MS: 460 (M+H)⁺.

1-Benzyl-4-(4-butoxy-benzenesulfonyl)-piperidine-4-carboxylic acid was prepared starting from 1-benzyl-4-(4-butoxy-benzenesulfonyl)-piperidine-4-carboxylic acid ethyl ester (5.1 g, 11.1 mmol) dissolved in THF:methanol 3:1 and 10 N NaOH (10 ml). The resulting reaction mixture was worked up as outlined in example 1. Yield 2.66 g (56%); off white solid; mp 210 °C; MS: 432 (M+H)⁺.

Starting from 1-benzyl-4-(4-butoxy-benzenesulfonyl)-piperidine-4-carboxylic acid (2.61 g, 6.06 mmol) and following the procedure as outlined in example 1, 860 mg of 1-benzyl-4-(4-butoxybenzenesulfonyl)-piperidine-4-carboxylic acid hydroxyamide was isolated as an off white powder. Yield 32%; mp 144 °C; MS: 446.9 (M+H)⁺; ¹H NMR (300 MHz, DMSO-d₆): δ 0.94 (t, J = 7.3 Hz, 3H), 1.44 (q, J = 7.5 Hz, 2H), 1.70 (q, 2H), 2.28-2.32 (m, 2H), 2.50 (d, 2H), 2.74-2.83 (m, 2H), 3.35 (d, 2H), 4.08 (t, J = 6.3 Hz, 2H), 4.34 (s, 2H), 7.13 (d, J = 8.7, 2H), 7.45 (s, 3H), 7.54 (s, 2H), 7.74 (d, J = 8.7, 2H), 9.35 (s, 1H), 10.7 (s, 1H).

### Example 17

### 1-(4-Fluoro-benzyl)-4-(4-methoxy-benzenesulfonyl)-piperidine-4-carboxylic acid hydroxyamide

1-(4-Fluoro-benzyl)-4-(4-methoxy-benzenesulfonyl)-piperidine-4-carboxylic acid ethyl ester was prepared according to the general method as outlined in example 1. Starting from 4-(methoxy-benzenesulfonyl) acetic acid ethyl ester (18.8 g, 72.8 mmol) and (4-fluoro-benzyl)-bis-(2-chloro-ethyl)-amine (20.8 g, 73 mmol). Yield 25 g (79%); brown oil; MS: 436.9 (M+H)⁺.

1-(4-Fluoro-benzyl)-4-(4-methoxy-benzenesulfonyl)-piperidine-4-carboxylic acid was prepared starting from 1-(4-fluoro-benzyl)-4-(4-methoxy-benzenesulfonyl)-piperidine-4-carboxylic acid ethyl ester (17.4 g, 40 mmol) dissolved in THF:methanol 3:1 and 10 N NaOH (40 ml). The resulting reaction mixture was worked up as outlined in example 1. Yield 10.8 g (66%); colorless solid; mp 154 °C; MS: 408 (M+H)⁺.

Starting from 1-(4-Fluoro-benzyl)-4-(4-methoxy-benzenesulfonyl)-piperidine-4-carboxylic acid (8.14 g, 20 mmol) and following the procedure as outlined in example 1, 4.3 g of 1-(4-fluorobenzyl)-4-(4-methoxy-benzenesulfonyl)-piperidine-4-carboxylic acid hydroxyamide was isolated as an off white solid. Yield 51%; mp 176-178 °C; MS: 484.7 (M+H)⁺; ¹H NMR (300 MHz, DMSO-d₆): δ 2.12-2.20 (m, 2H), 2.64-2.79 (m, 2H), 3.32-3.45 (m, 4H), 3.87 (s, 3H), 4.31 (s, 2H), 7.14-7.19 (d, J = 17 Hz, 2H), 7.27-7.33 (d, J = 8.1 Hz, 2H), 7.50-7.54 (d, 2H), 7.65-7.68 (d, 2H), 9.38 (s, 1H), 9.75 (s, 1H).

### Example 18

### 1-(4-Fluoro-benzyl)-4-(4-butoxy-benzenesulfonyl)-piperidine-4-carboxylic acid hydroxyamide

1-(4-Fluoro-benzyl)-4-(4-butoxy-benzenesulfonyl)-piperidine-4-carboxylic acid ethyl ester was prepared according to the general method as outlined in example 1. Starting from 4-(butoxybenzenesulfonyl) acetic acid ethyl ester (6 g, 20 mmol) and (4-fluoro-benzyl)-bis-(2-chloroethyl)-amine (5.73 g, 20 mmol). Yield 8.2 g (86%); yellow oil; MS: 478 (M+H)⁺.

1-(4-Fluoro-benzyl)-4-(4-butoxy-benzenesulfonyl)-piperidine-4-carboxylic acid was prepared starting from 1-(4-Fluoro-benzyl)-4-(4-butoxy-benzenesulfonyl)-piperidine-4-carboxylic acid ethyl ester (4.77 g, 10 mmol) dissolved in THF:methanol 3:1 and 10 N NaOH (10 ml). The resulting reaction mixture was worked up as outlined in example 1. Yield 3.5 g (79%); off white solid; mp 114 °C; MS: 450 (M+H)⁺.

Starting from 1-(4-Fluoro-benzyl)-4-(4-butoxy-benzenesulfonyl)-piperidine-4-carboxylic acid (2.24 g, 5.0 mmol) and following the procedure as outlined in example 1, 200 mg of 1-(4-Fluoro-benzyl)-4-(4-butoxy-benzenesulfonyl)-piperidine-4-carboxylic acid hydroxyamide was isolated as an off white powder. Yield 9%; mp 112 °C; MS: 465.9 (M+H)⁺; ¹H NMR (300 MHz, DMSO-d₆): δ 0.94 (t, J = 7.3 Hz, 3H), 1.35-1.50 (m, 2H), 1.68-1.77 (m, 2H), 2.20-2.28 (m, 2H), 2.66-2.77 (m, 2H), 3.77-3.78 (m, 4H), 4.06-4.10 (m, 2H), 4.19 (s, 2H), 7.14-7.19 (d, J = 8.7, 2H), 7.27-7.33 (d, 2H), 7.50-7.54 (d, 2H), 7.65-7.68 (d, 2H), 9.34 (s, 1H), 10.55 (s, 1H).

### Example 19

### 4-(4-methoxy-benzenesulfonyl)-1-(4-methoxy-benzyl)-piperidine-4-carboxylic acid hydroxyamide

2-[(2-Hydroxy-ethyl)-(4-methoxy-benzyl)-amino]-ethanol was prepared according to the general method as outlined in example 1. Starting from diethanolamine ( 12.0 g, 114 mmol) and 4-methoxybenzyl chloride ( 14.2 g, 100 mmol). Yield 17.5 g, (77 %); yellow oil; MS: 226 (M+H).

4-Methoxybenzyl-bis-(2-chloro-ethyl)-amine was prepared according to the general method as outlined in example 1. Starting from 4-Methoxy-benzyl diethanolamine (10 g, 44 mmol). Yield 10 g (75 %); yellow solid mp 55°C; MS: 263.1 (M+H)⁺.

4-(4-Methoxy-benzenesulfonyl)-1-(4-methoxy-benzyl)piperidine-4-carboxylic acid ethyl ester was prepared according to the general method as outlined in example 1. Starting from 4-(methoxy-benzenesulfonyl)-acetic acid ethyl ester (5.0 g, 20 mmol) and bis- (2-chloro ethyl)-(4-methoxy-benzyl)-amine (7.0 g, 22 mmol). Yield 5.0 g (56 %); low melting solid; MS: 448.5 (M+H)⁺.

4-(4-Methoxy-benzenesulfonyl) 1-(4-methoxy-benzyl)-piperidine-4-carboxylic acid was prepared starting from 4-(4-Methoxy-benzenesulfonyl)-1-(4-methoxy-benzyl)piperidine-4-carboxylic acid ethyl ester (4.2g, 10 mmol) dissolve in methanol (30 mL) , 10 N sodium hydroxide (10 mL), tetrahydrohydrofuran (20 mL). The resulting reaction mixture was worked up as outlined in example 1. Yield 3.0 g (71 %). white solid mp 190 °C , MS: 420.4 (M+H)⁺.

Starting from 4-(4-methoxy-benzenesulfonyl)-1-(4-methoxy-benzyl)-piperidine-4-carboxylic acid (2.0 g, 4.7 mmol) and following the procedure as outlined in example 1, 1.2 g of 4-(4-methoxy-benzenesulfonyl)-1-(4-methoxy-benzyl)-piperidine-4-carboxylic acid hydroxamide was isolated as a white solid. mp 175°C (HCl); Yield: 1.2 g, 59 %; MS: 433.0 (M+H)⁺; ¹H NMR (300 MHz, DMSO-d₆): δ 1.8 (m, 4H), 2.3(m, 2H), 2.73 (m, 2H), 3.37 (d, 2H), 3.76 (s, 3H), 3.88 (s,3H), 6.87 (d, 2H), 7.11 (d, 2H), 7.21 (d, 2H), 7.65 (d, 2H), 9.2 (bs, 1H), 10.9 (bs, 1H).

### Example 20

### 4-(4-methoxy-benzenesulfonyl)-1-[2-(4-methoxyphenyl)-ethyl]-piperidine-4-carboxylic acid hydroxyamide

2-{(2-Hydroxy-ethyl)-[2-(4-methoxy-phenyl)-ethyl]-amino}-ethanol was prepared according to the general method as outlined in example 1. Starting from diethanolamine (10.0 g, excess) and 1-(2-chloroethyl)-4-methoxybenzene (8.5 g, 50 mmol). Yield 11 g, (92%); yellow oil; MS: 240 (M+H)⁺.

The corresponding dichloride, bis-(2-chloro-ethyl)-(4-methoxyphenyl-2-ethyl)-amine was prepared according to the general method as outlined in example 1. Starting from 2-{(2-hydroxy-ethyl)-[2-(4-methoxy-phenyl)-ethyl]-amino}-ethanol (10 g, 41.8 mmol). Yield 11 g (95%); brown oil; MS: 277.2 (M+H)⁺.

4-(4-methoxy-benzenesulfonyl)-1-[2-(4-methoxyphenyl)-ethyl]-piperidine-4-carboxylic acid ethyl ester was prepared according to the general method as outlined in example 1. Starting from from 4-(methoxy-benzenesulfonyl) acetic acid ethyl ester (5.0 g, 20 mmol) and bis-(2-chloro-ethyl)-(4-methoxyphenyl-2-ethyl)-amine (6.4 g, 20 mmol). Yield 6.0 g (65%); brown oil; MS: 462.5 (M+H)⁺.

4-(4-methoxy-benzenesulfonyl)-1-[2-(4-methoxyphenyl)-ethyl]-piperidine-4-carboxylic acid was prepared starting from 4-(4-methoxy-benzenesulfonyl)-1-[2-(4-methoxyphenyl)-ethyl]-piperidine-4-carboxylic acid ethyl ester (5.0 g, 10.8 mmol) dissolved in THF:methanol 3:1 and 10 N NaOH (40 ml). The resulting reaction mixture was worked up as outlined in example 1. Yield 4.0 g (85%); off white powder; mp 205 °C; MS: 434.5 (M+H)⁺.

Starting from 4-(4-methoxy-benzenesulfonyl)-1-[2-(4-methoxyphenyl)-ethyl]-piperidine-4-carboxylic acid (1.5 g, 3.46 mmol) and following the procedure as outlined in example 1, 900 mg of 4-(4-methoxy-benzenesulfonyl)-1-[2-(4-methoxyphenyl)-ethyl]-piperidine-4-carboxylic acid hydroxyamide was isolated as an off white solid. Yield 58%; mp 206 °C (HCl); MS: 449.5 (M+H)⁺; ¹H NMR (300 MHz, DMSO-d₆): δ 2.3 (m, 2H), 2.5 (m, 3H), 2.8 (m, 2H), 2.95 (m, 2H), 3.25 (m, 2H), 3.4 (m,4H), 3.60 (d, J = 12.3 Hz, 2H), 3.77 (s, 3H),3.99 (s, 3H), 6.9 (d, 2 H), 7.1 - 7.25, (q, 4H), 7.7 (d, 2H), 9.3 (s, 1H), 10.6 (s, 1H).

### Example 21

### 4-(4-methoxy-benzenesulfonyl)-1-(2-phenyl-ethyl)-piperidine-4-carboxylic acid hydroxyamide

2-[(2-Hydroxy-ethyl)-(2-phenyl-ethyl)-amino]-ethanol was prepared according to the general method as outlined in example 1. Starting from diethanolamine (6.0 g, 57). and 2-bromoethylbenzene (9.0 g, 48.3 mmol). Yield 9 g, (90%); yellow oil; MS: 210 (M+H)⁺.

Bis-(2-Chloro-ethyl)-(2-phenyl-ethyl)-amine was prepared according to the general method as outlined in example 1. Starting from 2-[(2-Hydroxy-ethyl)-(2-phenyl-ethyl)-amino]-ethanol (8.5 g, 40.6 mmol). Yield 11 g (95%); brown oil; MS: 247.1 (M+H)⁺.

4-(4-methoxy-benzenesulfonyl)-1-(2-phenyl-ethyl)-piperidine-4-carboxylic acid ethyl ester was prepared according to the general method as outlined in example 1. Starting from from 4-(methoxy-benzenesulfonyl) acetic acid ethyl ester (5.0 g, 20 mmol) and bis-(2-chloro-ethyl)-(2-phenyl-ethyl)-amine (5.6 g, 20 mmol). Yield 5.5 g (63%); brown oil; MS: 432.5 (M+H)⁺.

4-(4-methoxy-benzenesulfonyl)-1-(2-phenyl-ethyl)-piperidine-4-carboxylic acid was prepared starting from 4-(4-methoxy-benzenesulfonyl)-1-(2-phenyl-ethyl)-piperidine-4-carboxylic acid ethyl ester (3.0 g, 6.9 mmol) dissolved in THF:methanol 3:1 and 10 N NaOH (40 ml). The resulting reaction mixture was worked up as outlined in example 1. Yield 2.0 g (72%); off white powder; mp 208 °C; MS: 404.5 (M+H)⁺.

Starting from 4-(4-methoxy-benzenesulfonyl)-1-(2-phenyl-ethyl)-piperidine-4-carboxylic acid (1.5 g, 3.7 mmol) and following the procedure as outlined in example 1, 900 mg of 4-(4-methoxy-benzenesulfonyl)-1-(2-phenyl-ethyl)-piperidine-4-carboxylic acid hydroxyamide was isolated as an off white solid. Yield 58%; mp 205 °C (HCl); MS: 419.4 (M+H)⁺; ¹H NMR (300 MHz, DMSO-d₆): δ 2.3 (m, 2H), 2.5 (m, 3H), 2.8 (m, 2H), 2.95 (m, 2H), 3.25 (m, 2H), 3.4 (m,4H), 3.9 (s, 3H),7.22 - 7.8 (m, 9H), 10.6 (s, 1H), 11.2 (bs, 1H).

### Example 22

### 4-(4-n-Butoxy-benzenesulfonyl)-1-(4-methoxy-benzyl)-piperidine-4-carboxylic acid hydroxyamide

4-(4-n-Butoxy-benzenesulfonyl)-1-(4-methoxy-benzyl)piperidine-4-carboxylic acid ethyl ester was prepared according to the general method as outlined in example 1. Starting from 4-(n-Butoxy-benzenesulfonyl)-acetic acid ethyl ester (2.5 g, 10 mmol) and bis- (2-chloro ethyl)-(4-methoxy-benzyl)-amine (3.0 g, 10 mmol). Yield 3.5 g (71 %); low melting solid; MS: 490.5 (M+H)⁺.

4-(4-n-Butoxy-benzenesulfonyl)1-(4-methoxy-benzyl)-piperidine-4-carboxylic acid was prepared starting from 4-(4-Butoxy-benzenesulfonyl)-1-(4-methoxy-benzyl)piperidine-4-carboxylic acid ethyl ester (3.0g, 6.1 mmol) dissolve in methanol (30 mL), 10 N sodium hydroxide (10 mL), tetrahydrohydrofuran (20 mL). The resulting reaction mixture was worked up as outlined in example 1. Yield 1.5 g (53 %). white solid mp 207°C , MS: 462.5 (M+H)⁺.

Starting from 4-(4-n-Butoxy-benzenesulfonyl)-1-(4-methoxy-benzyl)-piperidine-4-carboxylic acid (1.0 g, 2.1 mmol) and following the procedure as outlined in example 1, 1.2 g of 4-(4-Butoxy-benzenesulfonyl)-1-(4-methoxy-benzyl)-piperidine-4-carboxylic acid hydroxamide was isolated as a white solid. mp 173°C (HCl); Yield: 800 mg, 77 %; MS: 477.5 (M+H)⁺; ¹H NMR (300 MHz, DMSO-d₆): δ 0.9 (t, 3H), 1.4 (m, 2H), 1.7 (m,2H), 2.3 (m, 2H), 2.5 (m, 2H), 2.7 (m, 2H), 3.3 (m, 2H), 3.5(m, 2H), 4.1 (t, 2H), 4.3 (m, 2H), 6.97 ( d, 2H), 7.14 (d, 2H), 7.48 (d, 2H), 7.7 (d, 2H), 9.4 (bs, 1H), 10.9 (bs, 1H).

### Example 23

### 4-(4-Methoxy-benzenesulfonyl)-1-(3-phenoxy-propyl)-piperidine-4-carboxylic acid hydroxyamide

2-[(2-Hydroxy-ethyl)-(3-phenoxy-propyl)-amino]-ethanol was prepared according to the general method as outlined in example 1. Starting from diethanolamine (15.8 g, 151 mmol). and 3-Phenoxypropyl bromide (21.5 g, 100 mmol). Yield 21.31 g, (95%); yellow oil; MS: 238.1 (M+H)⁺.

Bis-(2-Chloro-ethyl)-(3-phenoxy-propyl)-amine was prepared according to the general method as outlined in example 1. Starting from 2-[(2-hydroxy-ethyl)-(3-phenoxy-propyl)-amino]-ethanol (20.0 g, 84 mmol). Yield 24.0 g (91%); brown oil; MS: 277.8 (M+H)⁺.

4-(4-Methoxy-benzenesulfonyl)-1-(3-phenoxy-propyl)-piperidine-4-carboxylic acid ethyl ester was prepared according to the general method as outlined in example 1. Starting from 4-(methoxy-benzenesulfonyl) acetic acid ethyl ester (5.2 g, 20 mmol) and bis-(2-chloro-ethyl)-(3-phenoxy-propyl)-amine (7.0 g, 22 mmol). Yield 6.5 g (70%); brown oil; MS: 462.5 (M+H)⁺.

4-(4-Methoxy-benzenesulfonyl)-1-(3-phenoxy-propyl)-piperidine-4-carboxylic acid was prepared starting from 4-(4-Methoxy-benzenesulfonyl)-1-(3-phenoxy-propyl)-piperidine-4-carboxylic acid ethyl ester (4.2 g, 9.1 mmol) dissolved in THF:Methanol 3:1 and 10 N NaOH (40 ml). The resulting reaction mixture was worked up as outlined in example 1. Yield 3.0 g (75%); off white powder; mp 195 °C; MS: 434.5 (M+H)⁺.

Starting from 4-(4-methoxy-benzenesulfonyl)-1-(3-phenoxy-propyl)-piperidine-4-carboxylic acid (2.5 g, 5.77 mmol) and following the procedure as outlined in example 1, 1.2 g of 4-(4-methoxy-benzenesulfonyl)-1-(3-phenoxy-propyl)-piperidine-4-carboxylic acid hydroxyamide was isolated as an off white solid. Yield 46%; mp 101 °C; MS: 448.5 (M+H)⁺; ¹H NMR (300 MHz, DMSO-d₆): δ 2.18 (m, 2H), 2.3 (m, 2H), 2.58 (m, 2H), 2.6-2.73 (m, 2H), 3.0-3.06 (m, 2H), 3.60 (m 2H), 3.87 (s, 3H), 4.01 (t, 2H), 6.9 - 7.7 (m, 9H), 9.33 (bs, 1H), 10.28 (bs, 1H).

### Example 24

### 4-(4-n-Butoxy-benzenesulfonyl)-1-(3-phenoxy-propyl)-piperidine-4-carboxylic acid hydroxyamide

4-(4-n-Butoxy-benzenesulfonyl)-1-(3-phenoxy-propyl)-piperidine-4-carboxylic acid ethyl ester was prepared according to the general method as outlined in example 1. Starting from 4-(butoxy-benzenesulfonyl) acetic acid ethyl ester (3.0 g, 10 mmol) and bis-(2-chloro-ethyl)-(3-phenoxy-propyl)-amine (3.0 g, 11 mmol). Yield 4.5 g (89%); brown oil; MS: 504.6 (M+H)⁺.

4-(4-n-Butoxy-benzenesulfonyl)-1-(3-phenoxy-propyl)-piperidine-4-carboxylic acid was prepared starting from 4-(4-n-Butoxy-benzenesulfonyl)-1-(3-phenoxy-propyl)-piperidine-4-carboxylic acid ethyl ester (4.0 g, 7.9 mmol) dissolved in THF:Methanol 3:1 and 10 N NaOH (40 ml). The resulting reaction mixture was worked up as outlined in example 1. Yield 3.0 g (79%); off white powder; mp 191 °C; MS: 476.5 (M+H)⁺.

Starting from 4-(4-n-butoxy-benzenesulfonyl)-1-(3-phenoxy-propyl)-piperidine-4-carboxylic acid (700 mg, 1.4 mmol) and following the procedure as outlined in example 1, 300 mg of 4-(4-n-butoxy-benzenesulfonyl)-1-(3-phenoxy-propyl)-piperidine-4-carboxylic acid hydroxyamide was isolated as an off white solid. Yield 43%; mp 84 °C; MS: 491.5 (M+H)⁺; ¹H NMR (300 MHz, DMSO-d₆): δ 0.9 (t, 3H), 1.5 (m, 2H), 1.8 (m, 2H), 2.18 (m, 2H), 2.3 (m, 2H), 2.58 (m, 2H), 2.6-2.73 (m, 2H), 3.2 (m, 2H), 3.40 (m 6H), 3.97 (t, 2H), 4.1 (t, 2H), 6.9 - 7.7 (m, 9H), 10.7 (bs, 1H), 11.28 (bs, 1H).

### Example 25

### 4-(4-methoxy-benzenesulfonyl)-1-(2-phenoxy-ethyl)-piperidine-4-carboxylic acid hydroxyamide

2-[(2-Hydroxy-ethyl)-(2-phenoxy-ethyl)-amino]-ethanol was prepared according to the general method as outlined in example 1. Starting from diethanolamine (15.0 g, 150). and 2-chlorophenetol (15.6 g, 100 mmol). Yield 18 g, (80%); Colorless oil; MS: 226 (M+H)⁺.

Bis-(2-Chloro-ethyl)-(2-phenoxy-ethyl)-amine was prepared according to the general method as outlined in example 1. Starting from 2-[(2-Hydroxy-ethyl)-(2-phenoxy-ethyl)-amino]-ethanol (20.0 g, 88.8 mmol). Yield 25 g (94%); brown oil; MS: 263.1 (M+H)⁺.

4-(4-methoxy-benzenesulfonyl)-1-(2-phenoxy-ethyl)-piperidine-4-carboxylic acid ethyl ester was prepared according to the general method as outlined in example 1. Starting from 4-(methoxy-benzenesulfonyl) acetic acid ethyl ester (5.0 g, 20 mmol) and bis-(2-chloroethyl)-(2-phenoxy-ethyl)-amine (6.0 g, 20 mmol). Yield 5.8 g (64%); brown oil; MS: 448.5 (M+H)⁺.

4-(4-methoxy-benzenesulfonyl)-1-(2-phenoxy-ethyl)-piperidine-4-carboxylic acid was prepared starting from 4-(4-methoxy-benzenesulfonyl)-1-(2-phenyl-ethoxy)-piperidine-4-carboxylic acid ethyl ester (5.0 g, 11.1 mmol) dissolved in THF:methanol 3:1 and 10 N NaOH (40 ml). The resulting reaction mixture was worked up as outlined in example 1. Yield 3.0 g (63%); off white powder; mp 235 °C; MS: 420.5 (M+H)⁺.

Starting from 4-(4-methoxy-benzenesulfonyl)-1-(2-phenoxy-ethyl)-piperidine-4-carboxylic acid (2.5 g, 5.9 mmol) and following the procedure as outlined in example 1, 1.3 g of 4-(4-methoxybenzenesulfonyl)-1-(2-phenoxy-ethyl)-piperidine-4-carboxylic acid hydroxyamide was isolated as an off white solid. Yield 50%; mp 168-172 °C (HCl); MS: 435.4 (M+H)⁺; ¹H NMR (300 MHz, DMSO-d₆): δ 2.3 (m, 2H), 2.5 (m, 2H), 2.9 (m, 2H), 3.4 (m, 4H), 3.5 (m, 2H), 3.7 (m,2H), 3.9 (s, 3H), 4.4 (m, 2H), 6.9 - 7.8 (m, 9H), 9.3 (s, 1H), 10.2 (bs, 1H), 11.3 (s, 1H).

### Example 26

### 4-(4-n-Butoxy-benzenesulfonyl)-1-(2-phenoxy-ethyl)-piperidine-4-carboxylic acid hydroxyamide

4-(4-Butoxy-benzenesulfonyl)-1-(2-phenoxy-ethyl)-piperidine-4-carboxylic acid ethyl ester was prepared according to the general method as outlined in example 1. Starting from from 4-(methoxy-benzenesulfonyl) acetic acid ethyl ester (2.5 g, 10 mmol) and bis-(2-chloro-ethyl)-(2-phenoxy-ethyl)-amine (2.98 g, 10 mmol). Yield 3.0 g (69%); brown oil; MS: 490.6 (M+H)⁺.

4-(4-n-Butoxy-benzenesulfonyl)-1-(2-phenoxy-ethyl)-piperidine-4-carboxylic acid was prepared starting from 4-(4-n-butoxy-benzenesulfonyl)-1-(2-phenyl-ethoxy)-piperidine-4-carboxylic acid ethyl ester (2.5 g, 5.76 mmol) dissolved in THF:methanol 3:1 and 10 N NaOH (40 ml). The resulting reaction mixture was worked up as outlined in example 1. Yield 1.5 g (56%); off white powder; mp 204 °C; MS: 462.5 (M+H)⁺.

Starting from 4-(4-n-butoxy-benzenesulfonyl)-1-(2-phenoxy-ethyl)-piperidine-4-carboxylic acid (1.0 g, 2.16 mmol) and following the procedure as outlined in example 1, 600 mg of 4-(4-butoxy-benzenesulfonyl)-1-(2-phenoxy-ethyl)-piperidine-4-carboxylic acid hydroxyamide was isolated as an off white solid. Yield 58%; mp 112 °C (HCl); MS: 477.4 (M+H)⁺; ¹H NMR (300 MHz, DMSO-d₆): δ 0.942 (t, 3H), 1.4 (m, 2H), 1.7 (m, 2H), 2.3 (m, 2H), 2.5 (m, 4H), 2.8 (m, 2H), 2.9-3.4 (m, 4H), 3.3 (m, 4H), 4.2 (t, 2H), 4.4 (m, 2H), 6.9 - 7.7 (m, 9H), 9.4 (s, 1H), 10.5 (bs, 1H), 11.3 (s, 1H).

### Example 27

### 4-(4-Methoxy-benzenesulfonyl)-1-[4-(2-piperidin-1-yl-ethoxy)-benzyl]-piperidine-4-carboxylic acid hydroxyamide

Bis-(2-chloro-ethyl)-[4-(2-piperidin-1-yl-ethoxy)-benzyl]-amine was prepared according to the general method as outlined in example 1. Starting from diethanolamine (15.0 g, 150). and 4-(2-piperidin-1-yl-ethoxy)-benzyl chloride (5.9 g, 20 mmol). Yield 5.5 g, (85%); Brown semi-solid; MS: 323 (M+H)⁺.

Bis-(2-chloro-ethyl)-[4-(2-piperidin-1-yl-ethoxy)-benzyl]-amine was prepared according to the general method as outlined in example 1. Starting from 2-[(2-Hydroxy-ethyl)-[4-(2-piperidin-1-yl-ethoxy)-benzyl]-amine (3.22 g, 10 mmol). Yield 4.0 g (92%); brown semi-solid; MS: 361.1 (M+H)⁺.

4-(4-Methoxy-benzenesulfonyl)-1-[4-(2-piperidin-1-yl-ethoxy)-benzyl]-piperidine-4-carboxylic acid ethyl ester was prepared according to the general method as outlined in example 1. Starting from 4-(methoxy-benzenesulfonyl) acetic acid ethyl ester (5.0 g, 20 mmol) and Bis-(2-chloroethyl)-[4-(2-piperidin-1-yl-ethoxy)-benzyl]-amine (8.6 g, 20 mmol). Yield 6.0 g (55%); brown oil; MS: 545.7 (M+H)⁺.

4-(4-Methoxy-benzenesulfonyl)-1-[4-(2-piperidin-1-yl-ethoxy)-benzyl]-piperidine-4-carboxylic acid was prepared starting from 4-(4-Methoxy-benzenesulfonyl)-1-[4-(2-piperidin-1-yl-ethoxy)-benzyl]-piperidine-4-carboxylic acid ethyl ester (5.4 g, 10 mmol) dissolved in THF:methanol 3:1 and 10 N NaOH (40 ml). The resulting reaction mixture was worked up as outlined in example 1. Yield 4.0 g (77%); off white powder; mp 174 °C; MS: 517.6 (M+H)⁺.

Starting from 4-(4-Methoxy-benzenesulfonyl)-1-[4-(2-piperidin-1-yl-ethoxy)-benzyl]-piperidine-4-carboxylic acid (3.5 g, 6.78 mmol) and following the procedure as outlined in example 1, 1.8 g of 4-(4-Methoxy-benzenesulfonyl)-1-[4-(2-piperidin-1-yl-ethoxy)-benzyl]-piperidine-4-carboxylic acid hydroxy amide was isolated as an pale yellow solid. Yield 49%; mp 114 °C (HCl); MS: 532 (M+H)⁺; ¹H NMR (300 MHz, DMSO-d₆): δ 1.4-1.6 (m, 4H), 1.9 (m, 2H), 2.3 (m, 2H), 2.8 (m, 2H), 3.4 (m, 4H), 3.9 (s, 3H), 4.2 (m, 1H), 6.9 - 7.8 (m, 8H), 9.1 (s, 1H), 10.8 (bs, 1H).

### References:

1 Rickter, L. S.; Desai, M. C. *Tetrahedron Letters*, **1997**, *38*, 321-322.

The subject compounds of the present invention were tested for biological activity according to the following procedures.

### In Vitro Gelatinase Assay

The assay is based on the cleavage of the thiopeptide substrate ((Ac-Pro-Leu-Gly(2 mercapto-4 methyl-pentanoyl)-Leu-Gly-OEt), Bachem Bioscience) by the enzyme, gelatinase, releasing the substrate product which reacts colorimetrically with DTNB ((5,5'-dithio-bis(2-nitro-benzoic acid)). The enzyme activity is measured by the rate of the color increase.
The thiopeptide substrate is made up fresh as a 20 mM stock in 100% DMSO and the DTNB is dissolved in 100% DMSO as a 100 mM stock and stored in dark at room temperature. Both the substrate and DTNB are diluted together to 1 mM with substrate buffer (50 mM HEPES pH 7.5, 5 mM CaCl₂) before use. The stock of human neutrophil gelatinase B is diluted with assay buffer (50 mM HEPES pH 7.5, 5 mM CaCl₂, 0.02% Brij) to a final concentration of 0.15 nM.

The assay buffer, enzyme, DTNB/substrate (500 µM final concentration) and vehicle or inhibitor are added to a 96 well plate (total reaction volume of 200µl) and the increase in color is monitored spectrophotometrically for 5 minutes at 405 nm on a plate reader.
The increase in OD₄₀₅ is plotted and the slope of the line is calculated which represents the reaction rate.

The linearity of the reaction rate is confirmed (r² >0.85). The mean (x ± sem) of the control rate is calculated and compared for statistical significance (p <0.05) with drug-treated rates using Dunnett's multiple comparison test. Dose-response relationships can be generated using multiple doses of drug and IC₅₀ values with 95% CI are estimated using linear regression (IPRED, HTB).
References: Weingarten, H and Feder, J., Spectrophotometric assay for vertebrate collagenase, Anal. Biochem. 147, 437-440 (1985).

### In Vitro Collagenase Assay

The assay is based on the cleavage of a peptide substrate ((Dnp-Pro-Cha-Gly-Cys(Me)-His-Ala-Lys(NMa)-NH₂), Peptide International, Inc.) by collagenase releasing the fluorescent NMa group which is quantitated on the fluorometer. Dnp quenches the NMa fluorescence in the intact substrate. The assay is run in HCBC assay buffer (50 mM HEPES, pH 7.0, 5 mM Ca⁺², 0.02% Brij, 0.5% Cysteine), with human recombinant fibroblast collagenase (truncated, mw=18,828, WAR, Radnor). Substrate is dissolved in methanol and stored frozen in 1 mM aliquots. Collagenase is stored frozen in buffer in 25 µM aliquots. For the assay, substrate is dissolved in HCBC buffer to a final concentration of 10 µM and collagenase to a final concentration of 5 nM. Compounds are dissolved in methanol, DMSO, or HCBC. The methanol and DMSO are diluted in HCBC to < 1.0%. Compounds are added to the 96 well plate containing enzyme and the reaction is started by the addition of substrate.

The reaction is read (excitation 340 nm, emission 444 nm) for 10 min. and the increase in fluorescence over time is plotted as a linear line. The slope of the line is calculated and represents the reaction rate.

The linearity of the reaction rate is confirmed (r² >0.85). The mean (x ± sem) of the control rate is calculated and compared for statistical significance (p <0.05) with drug-treated rates using Dunnett's multiple comparison test. Dose-response relationships can be generated using multiple doses of drug and IC₅₀ values with 95% CI are estimated using linear regression (IPRED, HTB).

References: Bickett, D. M. et al., A high throughput fluorogenic substrate for interstitial collagenase (MMP-1) and gelatinase (MMP-9), Anal. Biochem. 212,58-64 (1993).

### Procedure for Measuring TACE Inhibition

Using 96-well black microtiter plates, each well receives a solution composed of 10 µL TACE (Immunex, final concentration 1 µg/mL), 70µL Tris buffer, pH 7.4 containing 10% glycerol (final concentration 10 mM), and 10 µL of test compound solution in DMSO (final concentration 1µM, DMSO concentration <1%) and incubated for 10 minutes at room temperature. The reaction is initiated by addition of a fluorescent peptidyl substrate (final concentration 100 µM) to each well and then shaking on a shaker for 5 sec.
The reaction is read (excitation 340 nm, emission 420 nm) for 10 min. and the increase in fluorescence over time is plotted as a linear line. The slope of the line is calculated and represents the reaction rate.

The linearity of the reaction rate is confirmed (r² >0.85). The mean (x±sem) of the control rate is calculated and compared for statistical significance (p<0.05) with drug-treated rates using Dunnett's multiple comparison test. Dose-response relationships can be generate using multiple doses of drug and IC₅₀ values with 95% CI are estimated using linear regression

The results obtained following these standard experimental test procedures are presented in the following table.

| **IC 50 (nM or % inhibition at 1 micromolar)** | | | | |
|---|---|---|---|---|
| **Example** | **MMP 1** | **MMP 9** | **MMP 13** | **TACE** |
| 1 | 492 | 10.2 | 2.0 | 229 |
| 2 | 519 | 8.8 | 2.0 | 213 |
| 3 | 450 | 5.8 | 1.5 | 115 |
| 4 | 494 | 16.8 | 1.5 | 222 |
| 5 | 368 | 5.0 | 1.6 | 170.7 |
| 6 | 1329 | 12.8 | 3.1 | 610 |
| 7 | 1389 | 38.6 | 7.0 | 49% |
| 8 | 598 | 10.3 | 2.2 | 71.9 |
| 9 | 1929 | 13.3 | 10.8 | 503 |
| 10 | 59.6% | 649 | 148 | 9.7 |
| 11 | 56.3% | 452 | 38 | 15.8% |
| 12 | 2640 | 138 | 28.6 | 22.9 |
| 13 | 3681 | 364 | 33.1 | 25.4% |
| 14 | 4437 | 374 | 33.8 | 18.1 |
| 15 | 5109 | 484 | 43.7 | 20.20% |
| 16 | 2383 | 3.8 | 1.2 | 154 |
| 17 | 656 | 16.2 | 2.4 | 250 |
| 18 | 4729 | 19.1 | 5.3 | 39.5% |
| 19 | 642 | 12.3 | 2.1 | 197 |
| 20 | 662 | 33.7 | 1.9 | 53% |
| 21 | 1306 | 45.1 | 8.8 | 470 |
| 22 | 2610 | 3.1 | 1.4 | 208 |
| 23 | 1214 | 44.2 | 4.1 | 50.2% |
| 24 | 3788 | 5.1 | 0.9 | 631 |
| 25 | 629 | 26.8 | 2.5 | 293 |
| 26 | 2896 | 5.4 | 1.7 | 270 |
| 27 | 393 | 2.7 | 2.5 | 386 |

### Pharmaceutical Composition

Compounds of this invention may be administered neat or with a pharmaceutical carrier to a patient in need thereof. The pharmaceutical carrier may be solid or liquid.

Applicable solid carriers can include one or more substances which may also act as flavoring agents, lubricants, solubilizers, suspending agents, fillers, glidants, compression aids, binders or tablet-disintegrating agents or an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers may be used in preparing solutions, suspensions, emulsions, syrups and elixirs. The active ingredient of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such a solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above, e.g., cellulose derivatives, preferable sodium carboxymethyl cellulose solution), alcohols (including monohydric alcohols and polyhydric alcohols, e.g., glycols) and their derivatives, and oils (e.g., fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Oral administration may be either liquid or solid composition form.

The compounds of this invention may be administered rectally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non-toxic to the skin, and allows delivery of the agent for systemic absorption into the blood stream via the skin. The carrier may take any number of forms such as creams and ointments, pastes, gels, and occlusive devices. The creams and ointments may be viscous liquid or semi-solid emulsions of either the oil in water or water in oil type. Pastes comprised of absorptive powders dispersed in petroleum or hydrophilic petroleum containing the active ingredient may also be suitable. A variety of occlusive devices may be used to release the active ingredient into the blood stream such as a semipermeable membrane covering a reservoir containing the active ingredient with or without a carrier, or a matrix containing the active ingredient. Other occlusive devices are known in the literature.

The dosage to be used in the treatment of a specific patient suffering from a disease or condition in which MMPs and TACE are involved must be subjectively determined by the attending physician. The variables involved include the severity of the dysfunction, and the size, age, and response pattern of the patient. Treatment will generally be initiated with small dosages less than the optimum dose of the compound. Thereafter the dosage is increased until the optimum effect under the circumstances is reached. Precise dosages for oral, parenteral, nasal, or intrabronchial administration will be determined by the administering physician based on experience with the individual subject treated and standard medical principles.

Preferably the pharmaceutical composition is in unit dosage form, e.g., as tablets or capsules. In such form, the composition is sub-divided in unit dose containing appropriate quantities of the active ingredient; the unit dosage form can be packaged compositions, for example packed powders, vials, ampoules, prefilled syringes or sachets containing liquids. The unit dosage form can be, for example, a capsule or tablet itself, or it can be the appropriate number of any such compositions in package form.

## Claims

1. A compound according to formula I 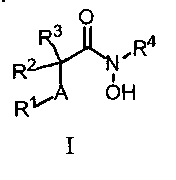 wherein:
R¹ is alkyl of 1 to 18 carbon atoms, optionally substituted with one or two groups selected independently from R⁵;
alkenyl of 3 to 18 carbon atoms having 1 to 3 double bonds, optionally substituted with one or two groups selected independently from R⁵;
alkynyl of 3 to 18 carbon atoms having 1 to 3 triple bonds, optionally substituted with one or two groups selected independently from R⁵;
aryl of 6 to 10 carbon atoms, optionally substituted with one or two groups selected independently from R⁵;
cycloalkyl of 3 to 8 carbon atoms, optionally substituted with one or two groups selected independently from R⁵;
saturated or unsaturated 5 to 10 membered mono or bicyclic heterocycle containing one heteroatom selected from O, S or NR⁷, optionally substituted with one or two groups selected independently from R⁵;
or heteroaryl-(CH₂)₀₋₆- wherein the heteroaryl group is 5 to 6 membered with one or two heteroatoms selected independently from O, S, and N and may be optionally substituted with one or two groups selected independently from R⁵;
A is -S-, -SO- or SO₂-;
R² and R³ , taken with the carbon atom to which they are attached, form a 5 to 7 membered heterocyclic ring containing O, S or N-R⁷ optionally having one or two double bonds;
R⁴ is hydrogen,
alkyl of 1 to 6 carbon atoms, optionally substituted with one or two groups selected independently from R⁵;
alkenyl of 3 to 18 carbon atoms having 1 to 3 double bonds, optionally substituted with one or two groups selected independently from R⁵;
alkynyl of 3 to 18 carbon atoms having 1 to 3 triple bonds, optionally substituted with one or two groups selected independently from R⁵;
phenyl or naphthyl optionally substituted with one or two groups selected independently from R⁵;
C₃ to C₈ cycloalkyl or bicycloalkyl optionally substituted with one or two groups selected independently from R⁵;
saturated or unsaturated 5 to 10 membered mono or bicyclic heterocycle containing one heteroatom selected from O, S or NR⁷, optionally substituted with one or two groups selected independently from R⁵;
R⁵ is H, C₇-C₁₁ aroyl, C₂-C₆ alkanoyl, C to C₁₂ alkyl, C₂ to C₁₂ alkenyl, C₂-C₁₂ alkynyl, F, Cl, Br, I, CN, CHO, C₁-C₆ alkoxy, aryloxy, heteroaryloxy, C₃-C₆ alkenyloxy, C₃-C₆ alkynyloxy, C₁-C₆ alkoxyaryl, C₁-C₆ alkoxyheteroaryl, C₁-C₆ alkylamino-C₁-C₆ alkoxy, C₁-C₂ alkylene dioxy, aryloxy-C₁-C₆ alkyl amine, C₁-C₁₂ perfluoro alkyl, S(O)ₙ-C₁-C₆ alkyl, S(O)ₙ-aryl where n is 0, 1 or 2; OCOO C₁-C₆ alkyl, OCOOaryl, OCONR⁶, COOH, COO C₁-C₆ alkyl, COOaryl, CONR⁶R⁶, CONHOH, NR⁶R⁶, SO₂NR⁶R⁶, NR⁶SO₂aryl, -NR⁶CONR⁶R⁶, NHSO₂CF₃, SO₂NHheteroaryl,SO₂NHCOaryl, CONHSO₂-C₁-C₆ alkyl, CONHSO₂aryl, SO₂NHCOaryl, CONHSO₂-C₁-C₆ alkyl, CONHSO₂aryl, NH₂, OH, aryl, heteroaryl, C₃ to C₈ cycloalkyl; or saturated or unsaturated 5 to 10 membered mono or bicyclic heterocycle containing one heteroatom selected from O, S or NR⁷, wherein C₁-C₆
alkyl is straight or branched, heteroaryl is a 5-10 membered mono or bicyclic heteroaryl group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
R⁶ is H, C₁ to C₁₈ alkyl optionally substituted with OH; C₃ to C₆ alkenyl, C₃ to C₆ alkynyl, C₁ to C₆ perfluoro alkyl, S(O)ₙ-C₁-C₆ alkyl S(O)ₙ aryl where n is 0, 1 or 2;, or COheteroaryl, wherein heteroaryl is a 5-10 membered mono or bicyclic heteroaryl
group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
and R⁷ is C₇-C₁₁ aroyl, C₂-C₆ alkanoyl, C₁-C₁₂ perfluoro alkyl, S(O)ₙ-C₁-C₆-alkyl, S(O)ₙ- aryl where n is 0, 1 or 2; COO-C₁-C₆-alkyl, COOaryl, CONHR⁶, CONR6R6, CONHOH, SO₂NR⁶R⁶, SO₂CF₃, SO₂NHheteroaryl, SO₂NHCOaryl, CONHSO-C₁-C₆-alkyl, CONHSO₂aryl, aryl, or heteroaryl, where aryl is phenyl or naphthyl, optionally substituted by 1 or 2 groups selected
independently from halogen, cyano, amino, nitro, C₁-C₆ alkyl, C₁-C₆ alkoxy, or hydroxy; and heteroaryl is a 5-10 membered mono or bicyclic heteroaryl group having 1 to 3 heteroatoms selected independently from O, S or N-C₁-C₆ alkyl;
alkyl of 1 to 18 carbon atoms, optionally substituted with one or two groups selected independently from R⁵;
alkenyl of 3 to 18 carbon atoms having from 1 to 3 double bonds, optionally substituted with one or two groups selected independently from R⁵;
alkynyl of 3 to 18 carbon atoms having from 1 to 3 triple bonds, optionally substituted with one or two groups selected independently from R⁵;
arylalkyl of 7 to 16 carbon atoms, wherein aryl is optionally substituted with one or two groups selected independently from R⁵;
biphenylalkyl of 13 to 18 carbon atoms, wherein biphenyl is optionally substituted with one or two groups selected independently from R⁵;
arylalkenyl of 8 to 16 carbon atoms, wherein aryl is optionally substituted with one or two groups selected independently from R⁵;
cycloalkylalkyl or bicycloalkylalkyl of 4 to 12 carbon atoms, wherein the cycloalkyl or bicycloalkyl group is optionally substituted with one or two groups selected independently from R⁵;
saturated or unsaturated mono or bicyclic heterocycle containing one heteroatom selected from O, S or N-C₁-C₆ alkyl, optionally substituted with one or two groups selected independently from R⁵; or
R⁸R⁹N-C₁-C₆-alkoxyaryl-C₁-C₆-alkyl where R⁸ and R⁹ are independently selected from C₁-C₆ alkyl or R⁸ and R⁹ together with the interposed nitrogen forms a 5-7 membered saturated heterocyclic ring optionally containing an oxygen atom, wherein the aryl group is phenyl or naphthyl;
or a pharmaceutically acceptable salt thereof.

2. A compound as claimed in Claim 1 wherein:
R¹ is alkyl of 1 to 18 carbon atoms, optionally substituted with one or two groups selected independently from R⁵;
alkenyl of 3 to 18 carbon atoms having 1 to 3 double bonds, optionally substituted with one or two groups selected independently from R⁵;
alkynyl of 3 to 18 carbon atoms having 1 to 3 triple bonds, optionally substituted with one or two groups selected independently from R⁵;
aryl of 6 to 10 carbon atoms, optionally substituted with one to two groups selected independently from R⁵;
cycloalkyl of 3 to 8 carbon atoms, optionally substituted with one to two groups selected independently from R⁵;
saturated or unsaturated mono or bicyclic heterocycle of from 5 to 10 members containing one heteroatom selected from O, S or NR⁷, optionally substituted with one to two groups selected independently from R⁵;
or heteroaryl-(CH₂)₀₋₆- wherein the heteroaryl group is 5 to 6 membered with one or two heteroatoms selected independently from O, S, and N and may be optionally substituted with one or two groups selected independently from R⁵;
A is -S-, -SO- or SO₂-;
R² and R³, taken with the carbon atom to which they are attached, form a 5 to 7 membered heterocyclic ring containing O, S or N-R⁷ optionally having one or two double bonds;
R⁴ is hydrogen,
alkyl of 1 to 6 carbon atoms, optionally substituted with one or two groups selected independently from R⁵;
alkenyl of 3 to 18 carbon atoms having 1 to 3 double bonds, optionally substituted with one or two groups selected independently from R⁵;
alkynyl of 3 to 18 carbon atoms having 1 to 3 triple bonds, optionally substituted with one or two groups selected independently from R⁵;
phenyl or naphthyl optionally substituted with one or two groups selected independently from R⁵;
C₃ to C₈ cycloalkyl or bicycloalkyl optionally substituted with one or two groups selected independently from R⁵;
R⁵ is H, F, Cl, Br, I, CN, CHO, C₇-C₁₁ aroyl, C₂-C₆ alkanoyl, C₁ to C₁₂ alkyl, C2 to C₁₂ alkenyl, C₂-C₁₂ alkynyl, C₁-C₆ alkoxy, aryloxy, heteroaryloxy, C₃-C₆ alkenyloxy, C₃-C₆ alkynyloxy, C₁-C₆ alkoxyaryl, C₁-C₆ alkoxyheteroaryl, C₁-C₆-alkylamino-C₁-C₆ alkoxy, C₁-C₂-alkylene dioxy, aryloxy-C₁-C₆ alkyl amine, C₁-C₁₂ perfluoro alkyl, S(O)ₙ-C₁-C₆ alkyl, S(O)ₙ-aryl where n is 0, 1 or 2; OCOO-C₁-C₆ alkyl, OCOOaryl, OCONR⁶, COOH, COO-C₁-C₆ alkyl, COOaryl, CONR⁶R⁶, CONHOH, NR⁶R⁶, SO₂NR⁶R⁶, NR⁶SO₂aryl, NR⁶CONR⁶R⁶, NHSO₂CF₃, SO₂NHheteroaryl, SO₂NHCOaryl, CONHSO₂-C₁-C₆ alkyl, CONHSO₂aryl, SO₂NHCOaryl, CONHSO₂-C₁-C₆ alkyl, CONHSO₂aryl, NH₂, OH, aryl, heteroaryl, C₃ to C₈ cycloalkyl; or saturated or unsaturated 5 to 10 membered mono or bicyclic heterocycle containing one heteroatom selected from O, S or NR⁷;
wherein heteroaryl is a 5-10 membered mono or bicyclic heteroaryl group having 1 to 3 heteroatoms selected independently from O, S or NR⁷ and aryl is phenyl or naphthyl, optionally substituted by 1 or 2 groups selected independently from halogen, cyano, amino, nitro, C₁-C₆ alkyl, C₁-C₆ alkoxy, or hydroxy;
R⁶ is H, C₁ to C₁₈ alkyl optionally substituted with OH; C₃ to C₆ alkenyl, C₃ to C₆ alkynyl, C₁ to C₆ perfluoro alkyl, S(O)ₙ alkyl or aryl where n is 0, 1, or 2; or COheteroaryl;
wherein heteroaryl is a 5-10 membered mono or bicyclic heteroaryl group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
and R⁷ is C₇-C₁₁ aroyl, C₂-C₆ alkanoyl, C₁-C₁₂ perfluoro alkyl, S(O)ₙ-alkyl, S(O)ₙ-aryl where n is 0, 1 or 2; COOalkyl, COOaryl, CONHR⁶, CONR⁶R⁶, CONHOH, SO₂NR⁶R⁶,SO₂CF₃, SO₂NHheteroaryl, SO₂NHCOaryl, CONHSO₂alkyl, CONHSO₂aryl, aryl, heteroaryl; wherein C₁-C₆ alkyl is straight or
branched, heteroaryl is a 5-10 membered mono or bicyclic heteroaryl group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
alkyl of 1 to 18 carbon atoms, optionally substituted with one or two groups selected independently from R⁵;
alkenyl of 3 to 18 carbon atoms having from 1 to 3 double bonds, optionally substituted with one or two groups selected independently from R⁵;
alkynyl of 3 to 18 carbon atoms having from 1 to 3 triple bonds, optionally substituted with one or two groups selected independently from R⁵;
arylalkyl of 7 to 16 carbon atoms, wherein aryl is optionally substituted with one or two groups selected independently from R⁵;
biphenylalkyl of 13 to 18 carbon atoms, wherein biphenyl is optionally substituted with one or two groups selected independently from R⁵;
arylalkenyl of 8 to 16 carbon atoms, wherein aryl is optionally substituted with one or two groups selected independently from R⁵;
cycloalkylalkyl or bicycloalkylalkyl of 4 to 12 carbon atoms, wherein cycloalkyl or bicycloalkyl is optionally substituted with one or two groups selected independently from R⁵;
saturated or unsaturated mono or bicyclic heterocycle containing one heteroatom selected from O, S or N-C₁-C₆ alkyl, optionally substituted with one or two groups selected independently from R⁵;
R⁸R⁹N-C₁-C₆-alkoxyaryl-C₁-C₆-alkyl where R⁸ and R⁹ are independently selected from C₁-C₆ alkyl or R⁸ and R⁹ together with the interposed nitrogen forms a 5-7 membered saturated heterocyclic ring optionally containing an oxygen atom, wherein the aryl group is phenyl or naphthyl;
or a pharmaceutically acceptable salt thereof.

3. A compound as claimed in Claim 2 wherein
R¹ is phenyl, naphthyl, alkyl of 1-18 carbon atoms or heteroaryl such as pyridyl, thienyl, imidazolyl or furanyl, optionally substituted with C₁-C₆ alkyl, C₁-C₆ alkoxy, C₆-C₁₀ aryloxy, heteroaryloxy, C₃-C₆ alkenyloxy, C₃-C₆ alkynyloxy, halogen; or S(O)ₙ-C₁-C₆alkyl C₁-C₆ alkoxyaryl or C₁-C₆ alkoxyheteroaryl;
A is -S-, -SO- or -SO₂-;
R² and R³, taken with the carbon atom to which they are attached, form a 5 to 7 membered heterocyclic ring containing O, S or N-R⁷ optionally having one or two double bonds;
R⁴ is hydrogen,
alkyl of 1 to 6 carbon atoms, optionally substituted with one or two groups selected independently from R⁵;
alkenyl of 3 to 18 carbon atoms having 1 to 3 double bonds, optionally substituted with one or two groups selected independently from R⁵;
alkynyl of 3 to 18 carbon atoms having 1 to 3 triple bonds, optionally substituted with one or two groups selected independently from R⁵;
phenyl or naphthyl optionally substituted with one or two groups selected independently from R⁵;
C₃ to C₈ cycloalkyl or bicycloalkyl optionally substituted with one or two groups selected independently from R⁵;
R⁵ is H, C₇-C₁₁ aroyl, C₂-C₆ alkanoyl, C₁ to C₁₂ alkyl, C₂ to C₁₂ alkenyl, C₂-C₁₂ alkynyl, F, Cl, Br, I, CN, CHO, C₁-C₆ alkoxy, aryloxy, heteroaryloxy, C₃-C₆ alkenyloxy, C₃-C₆ alkynyloxy, C₁-C₆ alkylamino-C₁-C₆ alkoxy, C₁-C₂ alkylene dioxy, aryloxy-C₁-C₆ alkyl amine, C₁-C₁₂ perfluoro alkyl, S(O)ₙ-C₁-C₆ alkyl, S(O)ₙ-aryl where n is 0, 1 or 2; OCOO C₁-C₆ alkyl, OCOOaryl, OCONR⁶, COOH, COO C₁-C₆ alkyl, COOaryl, CONR⁶R⁶, CONHOH, NR⁶R⁶, SO₂NR⁶R⁶, NR⁶SO₂aryl, -NR⁶CONR⁶R⁶, NHSO₂CF₃, SO₂NHheteroaryl,SO₂NHCOaryl, CONHSO₂-C₁-C₆ alkyl, CONHSO₂aryl, SO₂NHCOaryl, CONHSO₂-C₁-C₆ alkyl, CONHSO₂aryl, NH₂, OH, aryl, heteroaryl, C₃ to C₈ cycloalkyl; saturated or unsaturated 5 to 10 membered mono or bicyclic heterocycle containing one heteroatom selected from O, S or NR⁷, wherein C₁-C₆ alkyl is straight or branched, heteroaryl is a 5-10 membered mono or bicyclic heteroaryl group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
R⁶ is H, C₁ to C₁₈ alkyl optionally substituted with OH; C₃ to C₆ alkenyl, C₃ to C₆ alkynyl, C₁ to C₆ perfluoro alkyl, S(O)ₙ alkyl or aryl where n is 0, 1 or 2; or COheteroaryl; wherein heteroaryl is a 5-10 membered mono or bicyclic heteroaryl group having 1 to 3 heteroatoms selected independently from O, S or NR⁷ and aryl is phenyl or naphthyl, optionally substituted by 1 or 2 groups selected from halogen, cyano, amino, nitro, C₁-C₆ alkyl, C₁-C₆ alkoxy, or hydroxy;
and R⁷ is C₇-C₁₁ aroyl, C₂-C₆ alkanoyl, C₁-C₁₂ perfluoro alkyl, S(O)ₙ-alkyl, S(O)ₙ-aryl where n is 0, 1 or 2; COOalkyl, COOaryl, CONHR⁶, CONR⁶R⁶, CONHOH, SO₂NR⁶R⁶,SO₂CF₃, SO₂NHheteroaryl, SO₂NHCOaryl, CONHSO₂alkyl, CONHSO₂aryl, aryl, or heteroaryl; where aryl is phenyl or naphthyl,
optionally substituted by 1 or 2 groups selected independently from halogen, cyano, amino, nitro, C₁-C₆ alkyl, C₁-C₆ alkoxy, or hydroxy; and heteroaryl is a 5-10 membered mono or bicyclic heteroaryl group having 1 to 3 heteroatoms selected independently from O, S or N-C₁-C₆ alkyl;
alkyl of 1 to 18 carbon atoms, optionally substituted with one or two groups selected independently from R⁵;
alkenyl of 3 to 18 carbon atoms having from 1 to 3 double bonds, optionally substituted with one or two groups selected independently from R⁵;
alkynyl of 3 to 18 carbon atoms having from 1 to 3 triple bonds, optionally substituted with one or two groups selected independently from R⁵;
arylalkyl of 7 to 16 carbon atoms, optionally substituted with one or two groups selected independently from R⁵;
biphenylalkyl of 13 to 18 carbon atoms, optionally substituted with one or two groups selected independently from R⁵;
arylalkenyl of 8 to 16 carbon atoms, optionally substituted with one or two groups selected independently from R⁵;
cycloalkylalkyl or bicycloalkylalkyl of 4 to 12 carbon atoms, optionally substituted with one or two groups selected independently from R⁵;
saturated or unsaturated mono or bicyclic heterocycle containing one heteroatom selected from O, S or NR-C₁-C₆ alkyl, optionally substituted with one or two groups selected independently from R⁵;
R⁸R⁹N-C₁-C₆-alkoxyaryl-C₁-C₆-alkyl where R⁸ and R⁹ are independently selected from C₁-C₆ alkyl or R⁸ and R⁹ together with the interposed nitrogen forms a 5-7 membered saturated heterocyclic ring optionally containing an oxygen atom, wherein the aryl group is phenyl or naphthyl;
or a pharmaceutically acceptable salt thereof.

4. A compound as claimed in Claim 1 which is selected from the group of compounds consisting of:
1-benzyl-4-(4-methoxy-benzenesulfonyl)-piperidine-4-carboxylic acid hydroxyamide;
4-(4-methoxy-benzenesulfonyl)-1-(3-methoxy-benzyl)-piperidine-4-carboxylic acid hydroxyamide,
1-(3,4-dichlorobenzyl) -4-(4-methoxy-benzenesulfonyl)-piperidine-4-carboxylic acid hydroxamide,
4-(4-methoxy-benzenesulfonyl)-1-(4-methylbenzyl)-piperidine-4-carboxylic acid hydroxamide,
4-(4-methoxy-benzene-sulfonyl)-1-napthalene-2-yl-methylpiperidine-4-carboxylic acid hydroxamide,
1-biphenyl-4-ylmethyl-4-(4-methoxy-benzenesulfonyl)piperidine-4-carboxylic acid hydroxamide,
4-(4-methoxy-benzene-sulfonyl)-1-(3-methyl-but-2-enyl)piperidine-4-carboxylic acid hydroxamide,
1-(4-bromo-benzyl)-4-(4-methoxy-benzenesulfonyl)-piperidine-4-carboxylic acid hydroxyamide,
4-(4-methoxy-benzenesulfonyl)-1-[4-(2-piperidin-1-yl-ethoxy)-benzyl]-piperidine-4-carboxylic acid hydroxy amide
and pharmaceutically accepted salts thereof.

5. A compound as claimed in Claim 1 or a pharmaceutically acceptable salt thereof which is selected from the group of compounds consisting of:
4-(4-methoxy-benzenesulfonyl)-1-(3-phenyl-propyl)-piperidine-4-carboxylic acid hydroxyamide,
1-tert-butyl-4-(4-methoxy-benzenesulfonyl)-piperidine-4-carboxylic acid hydroxyamide,
1-butyl-4-(4-methoxy-benzenesulfonyl)-piperidine-4-carboxylic acid hydroxyamide,
1-cyclooctyl-4-(4-methoxy-benzenesulfonyl)-piperidine-4-carboxylic acid hydroxyamide,
1-ethyl-4-(4-methoxy-benzenesulfonyl)-piperidine-4-carboxylic acid hydroxyamide,
1-isopropyl-4-(4-methoxy-benzenesulfonyl)-piperidine-4-carboxylic acid hydroxyamide,
1-methyl-4-(4-methoxy-benzenesulfonyl)-piperidine-4-carboxylic acid hydroxyamide,
1-benzyl-4-(4-butoxy-benzenesulfonyl)-piperidine-4-carboxylic acid hydroxyamide,
1-(4-flourobenzyl)-4-(4-methoxy-benzenesulfonyl)-piperidine-4-carboxylic acid hydroxyamide,
1-(4-flourobenzyl)-4-(4-butoxy-benzenesulfonyl)-piperidine-4-carboxylic acid hydroxyamide,
4-(4-methoxy-benzenesulfonyl)-1-(4-methoxy-benzyl)-piperidine-4-carboxylic acid hydroxyamide,
4-(4-methoxy-benzenesulfonyl)-1-[2-(4-methoxyphenyl)-ethyl]-piperidine-4-carboxylic acid hydroxyamide,
4-(4-methoxy-benzenesulfonyl)-1-(2-phenyl-ethyl)-piperidine-4-carboxylic acid hydroxyamide,
4-(4-n-butoxy-benzenesulfonyl)-1-(4-methoxy-benzyl)-piperidine-4-carboxylic acid hydroxyamide,
4-(4-methoxy-benzenesulfonyl)-1-(3-phenoxy-propyl)-piperidine-4-carboxylic acid hydroxyamide,
4-(4-n-butoxy-benzenesulfonyl)-1-(3-phenoxy-propyl)-piperidine-4-carboxylic acid hydroxyamide,
4-(4-methoxy-benzenesulfonyl)-1-(2-phenoxy-ethyl)-piperidine-4-carboxylic acid hydroxyamide,
4-(4-n-butoxy-benzenesulfonyl)-1-(2-phenoxy-ethyl)-piperidine-4-carboxylic acid hydroxyamide, and
4-(4-methoxy-benzenesulfonyl)-1-[4-(2-piperidin-1-yl-ethoxy)-benzyl]-piperidine-4-carboxylic acid hydroxy amide.

6. A pharmaceutical composition comprising a pharmaceutical carrier and a therapeutically effective amount of a matrix metalloproteinase or TACE inhibiting compound as claimed in any one of Claims 1 to 5.

7. A compound as claimed in any one of Claims 1 to 5 for use as a medicament.

8. Use of a compound of as claimed in any one of Claims 1 to 5 for the manufacture of a medicament for inhibiting pathological changes mediated by matrix metalloproteinases in mammals or for inhibiting pathological changes mediated by TNF-α converting enzyme (TACE) in mammals.

## Patentansprüche

1. Verbindung gemäß Formel I 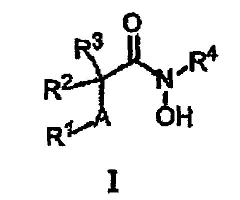 worin
R¹ für
Alkyl mit 1 bis 18 Kohlenstoffatomen, gegebenenfalls substituiert mit einer oder zwei Gruppen, unabhängig ausgewählt aus R⁵;
Alkenyl mit 3 bis 18 Kohlenstoffatomen mit 1 bis 3 Doppelbindungen, gegebenenfalls substituiert mit einer oder zwei Gruppen, unabhängig ausgewählt aus R⁵;
Alkinyl mit 3 bis 18 Kohlenstoffatomen mit 1 bis 3 Dreifachbindungen, gegebenenfalls substituiert mit einer oder zwei Gruppen, unabhängig ausgewählt aus R⁵;
Aryl mit 6 bis 10 Kohlenstoffatomen, gegebenfalls substituiert mit einer oder zwei Gruppen, unabhängig ausgewählt aus R⁵;
Cycloalkyl mit 3 bis 8 Kohlenstoffatomen, gegebenenfalls substituiert mit einer oder zwei Gruppen, unabhängig ausgewählt aus R⁵;
gesättigten oder ungesättigten 5- bis 10-gliedrigen monooder bicyclischen Heterocyclus, welcher ein Heteroatom enthält, ausgewählt aus O, S oder NR⁷, gegebenenfalls substituiert mit einer oder zwei Gruppen, unabhängig ausgewählt aus R⁵;
oder Heteroaryl-(CH₂)₀₋₆- steht, worin die Heteroarylgruppe 5- bis 6-gliedrig ist, mit einem oder zwei Heteroatomen, unabhängig ausgewählt aus O, S und N und gegebenenfalls mit einer oder zwei Gruppen substituiert sein kann, unabhängig ausgewählt aus R⁵;
A für -S-, -SO- oder SO₂- steht;
R² und R³ zusammengenommen mit dem Kohlenstoffatom, an welches sie gebunden sind, einen 5- bis 7-gliedrigen heterocyclischen Ring bilden, welcher O, S oder N-R⁷ enthält, gegebenenfalls mit einer oder zwei Doppelbindungen;
R⁴ für Wasserstoff,
Alkyl mit 1 bis 6 Kohlenstoffatomen, gegebenenfalls substituiert mit einer oder zwei Gruppen, unabhängig ausgewählt aus R⁵;
Alkenyl mit 3 bis 18 Kohlenstoffatomen mit 1 bis 3 Doppelbindungen, gegebenenfalls substituiert mit einer oder zwei Gruppen, unabhängig ausgewählt aus R⁵;
Alkinyl mit 3 bis 18 Kohlenstoffatomen mit 1 bis 3 Dreifachbindungen, gegebenenfalls substituiert mit einer oder zwei Gruppen, unabhängig ausgewählt aus R⁵;
Phenyl oder Naphthyl, gegebenenfalls substituiert mit einer oder zwei Gruppen, unabhängig ausgewählt aus R⁵;
C₃- bis C₈-Cycloalkyl oder Bicycloalkyl, gegebenenfalls substituiert mit einer oder zwei Gruppen, unabhängig ausgewählt aus R⁵;
gesättigten oder ungesättigten 5- bis 10-gliedrigen monooder bicyclischen Heterocyclus steht, welcher ein Heteroatom enthält, ausgewählt aus O, S oder NR⁷, gegebenenfalls substituiert mit einer oder zwei Gruppen, unabhängig ausgewählt aus R⁵;
R⁵ für H, C₇-C₁₁-Aroyl, C₂-C₆-Alkanoyl, C₁- bis C₁₂-Alkyl, C₂bis C₁₂-Alkenyl, C₂-C₁₂-Alkinyl, F, Cl, Br, I, CN, CHO, C₁-C₆-Alkoxy, Aryloxy, Heteroaryloxy, C₃-C₆-Alkenyloxy, C₃-C₆-Alkinyloxy, C₁-C₆-Alkoxyaryl, C₁-C₆-Alkoxyheteroaryl, C₁-C₆-Alkylamino-C₁-C₆alkoxy, C₁-C₂-Alkylendioxy, Aryloxy-C₁-C₆-alkylamin, C₁-C₁₂-Perfluoralkyl, S(O)ₙ-C₁-C₆-Alkyl, S(O)ₙ-Aryl, worin n 0, 1 oder 2 ist; OCOO-C₁-C₆-Alkyl, OCOO-Aryl, OCONR⁶, COOH, COO-C₁-C₆-Alkyl, COO-Aryl, CONR⁶R⁶, CONHOH, NR⁶R⁶, SO₂NR⁶R⁶, NR⁶SO₂-Aryl, -NR⁶CONR⁶R⁶, NHSO₂CF₃, SO₂NH-Heteroaryl, SO₂NHCO-Aryl, CONHSO₂-C₁-C₆-Alkyl, CON-HSO₂-Aryl, SO₂NHCO-Aryl, CONHSO₂-C₁-C₆-Alkyl, CONHSO₂-Aryl, NH₂, OH, Aryl, Heteroaryl, C₃- bis C₈-Cycloalkyl; oder gesättigten oder ungesättigten 5- bis 10-gliedrigen mono- oder bicyclischen Heterocyclus steht, welcher ein Heteroatom enthält, ausgewählt aus O, S oder NR⁷, worin C₁-C₆-Alkyl gerade oder verzweigt ist, Heteroaryl eine 5-10-gliedrige mono- oder bicyclische Heteroarylgruppe mit 1 bis 3 Heteroatomen darstellt, unabhängig ausgewählt aus 0, S oder NR⁷ und Aryl für Phenyl oder Naphthyl steht, gegebenenfalls substituiert durch 1 oder 2 Gruppen, ausgewählt aus Halogen, Cyano, Amino, Nitro, C₁-C₆-Alkyl, C₁-C₆-Alkoxy oder Hydroxy;
R⁶ für H, C₁- bis C₁₈-Alkyl, gegebenenfalls substituiert mit OH; C₃- bis C₆-Alkenyl, C₃- bis C₆-Alkinyl, C₁- bis C₆-Perfluoralkyl, S(O)ₙ-C₁-C₆-Alkyl S(O)ₙ-Aryl, worin n 0, 1 oder 2 ist; oder CO-Heteroaryl steht, worin Heteroaryl für eine 5-10-gliedrige mono- oder bicyclische Heteroarylgruppe mit 1 bis 3 Heteroatomen steht, unabhängig ausgewählt aus O, S oder NR⁷ und Aryl für Phenyl oder Naphthyl steht, gegebenenfalls substituiert durch 1 oder 2 Gruppen, ausgewählt aus Halogen, Cyano, Amino, Nitro, C₁-C₆-Alkyl, C₁-C₆-Alkoxy oder Hydroxy;
und R⁷ für
C₇-C₁₁-Aroyl, C₂-C₆-Alkanoyl, C₁-C₁₂-Perfluoralkyl, S(O)ₙ-C₁-C₆-Alkyl, S(O)ₙ-Aryl, worin n 0, 1 oder 2 ist; COO-C₁-C₆-Alkyl, COO-Aryl, CONHR⁶, CONR⁶R⁶, CONHOH, SO₂NR⁶R⁶, SO₂CF₃, SO₂NH-Heteroaryl, SO₂NHCO-Aryl, CONHSO-C₁-C₆-Alkyl, CONHSO₂-Aryl, Aryl oder Heteroaryl steht, worin Aryl für Phenyl oder Naphthyl steht, gegebenenfalls substituiert durch 1 oder 2 Gruppen, unabhängig ausgewählt aus Halogen, Cyano, Amino, Nitro, C₁-C₆-Alkyl, C₁-C₆-Alkoxy oder Hydroxy; und Heteroaryl eine 5-10-gliedrige mono- oder bicyclische Heteroarylgruppe mit 1 bis 3 Heteroatomen darstellt, unabhängig ausgewählt aus O, S oder N-C₁-C₆-Alkyl;
Alkyl mit 1 bis 18 Kohlenstoffatomen, gegebenenfalls substituiert mit einer oder zwei Gruppen, unabhängig ausgewählt aus R⁵;
Alkenyl mit 3 bis 18 Kohlenstoffatomen mit von 1 bis 3 Doppelbindungen, gegebenenfalls substituiert mit einer oder zwei Gruppen, unabhängig ausgewählt aus R⁵;
Alkinyl mit 3 bis 18 Kohlenstoffatomen mit von 1 bis 3 Dreifachbindungen, gegebenenfalls substituiert mit einer oder zwei Gruppen, unabhängig ausgewählt aus R⁵;
Arylalkyl mit 7 bis 16 Kohlenstoffatomen, wobei Aryl gegebenenfalls substituiert ist mit einer oder zwei Gruppen, unabhängig ausgewählt aus R⁵;
Biphenylalkyl mit 13 bis 18 Kohlenstoffatomen, worin Biphenyl gegebenenfalls substituiert ist mit einer oder zwei Gruppen, unabhängig ausgewählt aus R⁵;
Arylalkenyl mit 8 bis 16 Kohlenstoffatomen, worin Aryl gegebenenfalls substituiert ist mit einer oder zwei Gruppen, unabhängig ausgewählt aus R⁵;
Cycloalkylalkyl oder Bicycloalkylalkyl mit 4 bis 12 Kohlenstoffatomen, worin die Cycloalkyl- oder Bicycloalkylgruppe gegebenenfalls substituiert ist mit einer oder zwei Gruppen, unabhängig ausgewählt aus R⁵;
gesättigten oder ungesättigten mono- oder bicyclischen Heterocyclus, welcher ein Heteroatom enthält, ausgewählt aus O, S oder N-C₁-C₆-Alkyl, gegebenenfalls substituiert mit einer oder zwei Gruppen, unabhängig ausgewählt aus R⁵; oder
R⁸R⁹N-C₁-C₆-Alkoxyaryl-C₁-C₆-alkyl steht, worin R⁸ und R⁹ unabhängig ausgewählt werden aus C₁-C₆-Alkyl oder R⁸ und R⁹ zusammen mit dem dazwischen geschobenen Stickstoff einen 5-7-gliedrigen gesättigten heterocyclischen Ring bilden, welcher gegebenenfalls ein Sauerstoffatom enthält, worin die Arylgruppe für Phenyl oder Naphthyl steht;
oder ein pharmazeutisch annehmbares Salz davon.

2. Verbindung wie in Anspruch 1 beansprucht, worin:
R¹ für
Alkyl mit 1 bis 18 Kohlenstoffatomen, gegebenenfalls substituiert mit einer oder zwei Gruppen, unabhängig ausgewählt aus R⁵;
Alkenyl mit 3 bis 18 Kohlenstoffatomen mit 1 bis 3 Doppelbindungen, gegebenenfalls substituiert mit einer oder zwei Gruppen, unabhängig ausgewählt aus R⁵;
Alkinyl mit 3 bis 18 Kohlenstoffatomen mit 1 bis 3 Dreifachbindungen, gegebenenfalls substituiert mit einer oder zwei Gruppen, unabhängig ausgewählt aus R⁵;
Aryl mit 6 bis 10 Kohlenstoffatomen, gegebenfalls substituiert mit einer oder zwei Gruppen, unabhängig ausgewählt aus R⁵;
Cycloalkyl mit 3 bis 8 Kohlenstoffatomen, gegebenenfalls substituiert mit einer oder zwei Gruppen, unabhängig ausgewählt aus R⁵;
gesättigten oder ungesättigten mono- oder bicyclischen Heterocyclus mit von 5 bis 10 Gliedern, welcher ein Heteroatom enthält, ausgewählt aus O, S oder NR⁷, gegebenenfalls substituiert mit einer oder zwei Gruppen, unabhängig ausgewählt aus R⁵;
oder Heteroaryl-(CH₂)₀₋₆- steht, worin die Heteroarylgruppe 5- bis 6-gliedrig ist, mit einem oder zwei Heteroatomen, unabhängig ausgewählt aus 0, S und N, und gegebenenfalls mit einer oder zwei Gruppen substituiert sein kann, unabhängig ausgewählt aus R⁵;
A für -S-, -SO- oder SO₂- steht;
R² und R³ zusammengenommen mit dem Kohlenstoffatom, an welches sie gebunden sind, einen 5- bis 7-gliedrigen heterocyclischen Ring bilden, welcher O, S oder N-R⁷ enthält, gegebenenfalls mit einer oder zwei Doppelbindungen;
R⁴ für Wasserstoff,
Alkyl mit 1 bis 6 Kohlenstoffatomen, gegebenenfalls substituiert mit einer oder zwei Gruppen, unabhängig ausgewählt aus R⁵;
Alkenyl mit 3 bis 18 Kohlenstoffatomen mit 1 bis 3 Doppelbindungen, gegebenenfalls substituiert mit einer oder zwei Gruppen, unabhängig ausgewählt aus R⁵;
Alkinyl mit 3 bis 18 Kohlenstoffatomen mit 1 bis 3 Dreifachbindungen, gegebenenfalls substituiert mit einer oder zwei Gruppen, unabhängig ausgewählt aus R⁵;
Phenyl oder Naphthyl, gegebenenfalls substituiert mit einer oder zwei Gruppen, unabhängig ausgewählt aus R⁵;
C₃- bis C₈-Cycloalkyl oder Bicycloalkyl steht, gegebenenfalls substituiert mit einer oder zwei Gruppen, unabhängig ausgewählt aus R⁵;
R⁵ für H, F, Cl, Br, I, CN, CHO, C₇-C₁₁-Aroyl, C₂-C₆-Alkanoyl, C₁- bis C₁₂-Alkyl, C₂- bis C₁₂-Alkenyl, C₂-C₁₂-Alkinyl, C₁-C₆-Alkoxy, Aryloxy, Heteroaryloxy, C₃-C₆-Alkenyloxy, C₃-C₆-Alkinyloxy, C₁-C₆-Alkoxyaryl, C₁-C₆-Alkoxyheteroaryl, C₁-C₆-Alkylamino-C₁-C₆-alkoxy, C₁-C₂-Alkylendioxy, Aryloxy-C₁-C₆-alkylamin, C₁-C₁₂-Perfluoralkyl, S(O)ₙ-C₁-C₆-Alkyl, S(O)ₙ-Aryl, worin n 0, 1 oder 2 ist; OCOO-C₁-C₆-Alkyl, OCOO-Aryl, OCONR⁶, COOH, COO-C₁-C₆-Alkyl, COO-Aryl, CONR⁶R⁶, CONHOH, NR⁶R⁶, SO₂NR⁶R⁶, NR⁶SO₂-Aryl, -NR⁶CONR⁶R⁶, NHSO₂CF₃, SO₂NH-Heteroaryl, SO₂NHCO-Aryl, CONHSO₂-C₁-C₆-Alkyl, CONHSO₂-Aryl, SO₂NHCO-Aryl, CONHSO₂-C₁-C₆-Alkyl, CONHSO₂-Aryl, NH₂, OH, Aryl, Heteroaryl, C₃- bis C₈-Cycloalkyl; oder gesättigten oder ungesättigten 5- bis 10-gliedrigen mono- oder bicyclischen Heterocyclus steht, welcher ein Heteroatom enthält, ausgewählt aus O, S oder NR⁷, worin Heteroaryl eine 5-10-gliedrige mono- oder bicyclische Heteroarylgruppe mit 1 bis 3 Heteroatomen darstellt, unabhängig ausgewählt aus O, S oder NR⁷ und Aryl für Phenyl oder Naphthyl steht, gegebenenfalls substituiert durch 1 oder 2 Gruppen, unabhängig ausgewählt aus Halogen, Cyano, Amino, Nitro, C₁-C₆-Alkyl, C₁-C₆-Alkoxy oder Hydroxy;
R⁶ für H, C₁- bis C₁₈-Alkyl, gegebenenfalls substituiert mit OH; C₃-C₆-Alkenyl, C₃- bis C₆-Alkinyl, C₁- bis C₆-Perfluoralkyl, S (O)ₙ-Alkyl oder Aryl, worin n 0, 1 oder 2 ist; oder CO-Heteroaryl steht, worin Heteroaryl für eine 5-10-gliedrige mono- oder bicyclische Heteroarylgruppe mit 1 bis 3 Heteroatomen steht, unabhängig ausgewählt aus O, S oder NR⁷ und Aryl für Phenyl oder Naphthyl steht, gegebenenfalls substituiert durch 1 oder 2 Gruppen, ausgewählt aus Halogen, Cyano, Amino, Nitro, C₁-C₆-Alkyl, C₁-C₆-Alkoxy oder Hydroxy;
und R⁷ für
C₇-C₁₁-Aroyl, C₂-C₆-Alkanoyl, C₁-C₁₂-Perfluoralkyl, S(O)ₙ-Alkyl, S(O)ₙ-Aryl, worin n 0, 1 oder 2 ist; COO-Alkyl, COO-Aryl, CONHR⁶, CONR⁶R⁶, CONHOH, SO₂NR⁶R⁶, SO₂CF₃, SO₂NH-Heteroaryl, SO₂NHCO-Aryl, CONHSO₂-Alkyl, CONHSO₂-Aryl, Aryl, Heteroaryl steht; worin C₁-C₆-Alkyl gerade oder verzweigt ist, Heteroaryl eine 5-10-gliedrige mono- oder bicyclische Heteroarylgruppe mit 1 bis 3 Heteroatomen darstellt, unabhängig ausgewählt aus O, S oder NR⁷ und Aryl für Phenyl oder Naphthyl steht, gegebenenfalls substituiert durch 1 oder 2 Gruppen, unabhängig ausgewählt aus Halogen, Cyano, Amino, Nitro, C₁-C₆-Alkyl, C₁-C₆-Alkoxy oder Hydroxy;
Alkyl mit 1 bis 18 Kohlenstoffatomen, gegebenenfalls substituiert mit einer oder zwei Gruppen, unabhängig ausgewählt aus R⁵;
Alkenyl mit 3 bis 18 Kohlenstoffatomen mit von 1 bis 3 Doppelbindungen, gegebenenfalls substituiert mit einer oder zwei Gruppen, unabhängig ausgewählt aus R⁵;
Alkinyl mit 3 bis 18 Kohlenstoffatomen mit von 1 bis 3 Dreifachbindungen, gegebenenfalls substituiert mit einer oder zwei Gruppen, unabhängig ausgewählt aus R⁵;
Arylalkyl mit 7 bis 16 Kohlenstoffatomen, woin Aryl gegebenenfalls substituiert ist mit einer oder zwei Gruppen, unabhängig ausgewählt aus R⁵;
Biphenylalkyl mit 13 bis 18 Kohlenstoffatomen, worin Biphenyl gegebenenfalls substituiert ist mit einer oder zwei Gruppen, unabhängig ausgewählt aus R⁵;
Arylalkenyl mit 8 bis 16 Kohlenstoffatomen, worin Aryl gegebenenfalls substituiert ist mit einer oder zwei Gruppen, unabhängig ausgewählt aus R⁵;
Cycloalkylalkyl oder Bicycloalkylalkyl mit 4 bis 12 Kohlenstoffatomen, worin Cycloalkyl oder Bicycloalkyl gegebenenfalls substituiert ist mit einer oder zwei Gruppen, unabhängig ausgewählt aus R⁵;
gesättigten oder ungesättigten mono- oder bicyclischen Heterocyclus, welcher ein Heteroatom enthält, ausgewählt aus O, S oder N-C₁-C₆-Alkyl, gegebenenfalls substituiert mit einer oder zwei Gruppen, unabhängig ausgewählt aus R⁵; oder
R⁸R⁹N-C₁-C₆-Alkoxyaryl-C₁-C₆-alkyl steht, worin R⁸ und R⁹ unabhängig ausgewählt werden aus C₁-C₆-Alkyl oder R⁸ und R⁹ zusammen mit dem dazwischen geschobenen Stickstoff einen 5-7-gliedrigen gesättigten heterocyclischen Ring bilden, welcher gegebenenfalls ein Sauerstoffatom enthält, wobei die Arylgruppe für Phenyl oder Naphthyl steht;
oder ein pharmazeutisch annehmbares Salz davon.

3. Verbindung wie in Anspruch 2 beansprucht, worin
R¹ für Phenyl, Naphthyl, Alkyl mit 1-18 Kohlenstoffatomen oder Heteroaryl, wie Pyridyl, Thienyl, Imidazolyl oder Furanyl, gegebenenfalls substituiert mit C₁-C₆-Alkyl, C₁-C₆-Alkoxy, C₆-C₁₀-Aryloxy, Heteroaryloxy, C₃-C₆-Alkenyloxy, C₃-C₆Alkinyloxy, Halogen; oder S(O)ₙ-C₁-C₆-Alkyl-C₁-C₆-alkoxyaryl oder C₁-C₆-Alkoxyheteroaryl steht;
A für -S-, -SO- oder SO₂- steht;
R² und R³ zusammengenommen mit dem Kohlenstoffatom, an welches sie gebunden sind, einen 5- bis 7-gliedrigen heterocyclischen Ring bilden, welcher O, S oder N-R⁷ enthält, gegebenenfalls mit einer oder zwei Doppelbindungen;
R⁴ für Wasserstoff,
Alkyl mit 1 bis 6 Kohlenstoffatomen, gegebenenfalls substituiert mit einer oder zwei Gruppen, unabhängig ausgewählt aus R⁵;
Alkenyl mit 3 bis 18 Kohlenstoffatomen mit 1 bis 3 Doppelbindungen, gegebenenfalls substituiert mit einer oder zwei Gruppen, unabhängig ausgewählt aus R⁵;
Alkinyl mit 3 bis 18 Kohlenstoffatomen mit 1 bis 3 Dreifachbindungen, gegebenenfalls substituiert mit einer oder zwei Gruppen, unabhängig ausgewählt aus R⁵;
Phenyl oder Naphthyl, gegebenenfalls substituiert mit einer oder zwei Gruppen, unabhängig ausgewählt aus R⁵;
C₃- bis C₈-Cycloalkyl oder Bicycloalkyl steht, gegebenenfalls substituiert mit einer oder zwei Gruppen, unabhängig ausgewählt aus R⁵;
R⁵ für H, C₇-C₁₁-Aroyl, C₂-C₆-Alkanoyl, C₁- bis C₁₂-Alkyl, C₂bis C₁₂-Alkenyl, C₂-C₁₂-Alkinyl, F, Cl, Br, I, CN, CHO, C₁-C₆-Alkoxy, Aryloxy, Heteroaryloxy, C₃-C₆-Alkenyloxy, C₃-C₆-Alkinyloxy, C₁-C₆-Alkylamino-C₁-C₆-alkoxy, C₁-C₂-Alkylendioxy, Aryloxy-C₁-C₆-alkylamin, C₁-C₁₂-Perfluoralkyl, S(O)ₙ-C₁-C₆-Alkyl, S(O)ₙ-Aryl, worin n 0, 1 oder 2 ist; OCOO-C₁-C₆-Alkyl, OCOO-Aryl, OCONR⁶, COOH, COO-C₁-C₆-Alkyl, COO-Aryl, CONR⁶R⁶, CONHOH, NR⁶R⁶, SO₂NR⁶R⁶, NR⁶SO₂-Aryl, - NR⁶CONR⁶R⁶, NHSO₂CF₃, SO₂NH-Heteroaryl, SO₂NHCO-Aryl, CONHSO₂-C₁-C₆-Alkyl, CONHSO₂-Aryl, SO₂NHCO-Aryl, CONHSO₂-C₁-C₆-Alkyl, CONHSO₂-Aryl, NH₂, OH, Aryl, Heteroaryl, C₃- bis C₈-Cycloalkyl; gesättigten oder ungesättigten 5- bis 10-gliedrigen mono- oder bicyclischen Heterocyclus steht, welcher ein Heteroatom enthält, ausgewählt aus O, S oder NR⁷, worin C₁-C₆-Alkyl gerade oder verzweigt ist, Heteroaryl eine 5-10-gliedrige mono- oder bicyclische Heteroarylgruppe mit 1 bis 3 Heteroatomen darstellt, unabhängig ausgewählt aus O, S oder NR⁷ und Aryl für Phenyl oder Naphthyl steht, gegebenenfalls substituiert durch 1 oder 2 Gruppen, unabhängig ausgewählt aus Halogen, Cyano, Amino, Nitro, C₁-C₆-Alkyl, C₁-C₆-Alkoxy oder Hydroxy;
R⁶ für H, C₁- bis C₁₈-Alkyl, gegebenenfalls substituiert mit OH; C₃- bis C₆-Alkenyl, C₃- bis C₆-Alkinyl, C₁- bis C₆-Perfluoralkyl, S(O)ₙ-Alkyl oder Aryl, worin n 0, 1 oder 2 ist; oder CO-Heteroaryl steht, worin Heteroaryl für eine 5-10-gliedrige monooder bicyclische Heteroarylgruppe mit 1 bis 3 Heteroatomen steht, unabhängig ausgewählt aus O, S oder NR⁷ und Aryl für Phenyl oder Naphthyl steht, gegebenenfalls substituiert durch 1 oder 2 Gruppen, ausgewählt aus Halogen, Cyano, Amino, Nitro, C₁-C₆-Alkyl, C₁-C₆-Alkoxy oder Hydroxy;
und R⁷ für
C₇-C₁₁-Aroyl, C₂-C₆-Alkanoyl, C₁-C₁₂-Perfluoralkyl, S(O)ₙ-Alkyl, S(O)ₙ-Aryl, worin n 0, 1 oder 2 ist; COO-Alkyl, COO-Aryl, CONHR⁶, CONR⁶R⁶, CONHOH, SO₂NR⁶R⁶, SO₂CF₃, SO₂NH-Heteroaryl, SO₂NHCO-Aryl, CONHSO₂-Alkyl, CONHSO₂-Aryl, Aryl oder Heteroaryl steht, worin Aryl für Phenyl oder Naphthyl steht, gegebenenfalls substituiert durch 1 oder 2 Gruppen, unabhängig ausgewählt aus Halogen, Cyano, Amino, Nitro, C₁-C₆-Alkyl, C₁-C₆-Alkoxy oder Hydroxy; und Heteroaryl eine 5-10-gliedrige mono- oder bicyclische Heteroarylgruppe mit 1 bis 3 Heteroatomen darstellt, unabhängig ausgewählt aus O, S oder N-C₁-C₆-Alkyl;
Alkyl mit 1 bis 18 Kohlenstoffatomen, gegebenenfalls substituiert mit einer oder zwei Gruppen, unabhängig ausgewählt aus R⁵;
Alkenyl mit 3 bis 18 Kohlenstoffatomen mit von 1 bis 3 Doppelbindungen, gegebenenfalls substituiert mit einer oder zwei Gruppen, unabhängig ausgewählt aus R⁵;
Alkinyl mit 3 bis 18 Kohlenstoffatomen mit von 1 bis 3 Dreifachbindungen, gegebenenfalls substituiert mit einer oder zwei Gruppen, unabhängig ausgewählt aus R⁵;
Arylalkyl mit 7 bis 16 Kohlenstoffatomen, gegebenenfalls substituiert mit einer oder zwei Gruppen, unabhängig ausgewählt aus R⁵;
Biphenylalkyl mit 13 bis 18 Kohlenstoffatomen, gegebenenfalls substituiert mit einer oder zwei Gruppen, unabhängig ausgewählt aus R⁵;
Arylalkenyl mit 8 bis 16 Kohlenstoffatomen, gegebenenfalls substituiert mit einer oder zwei Gruppen, unabhängig ausgewählt aus R⁵;
Cycloalkylalkyl oder Bicycloalkylalkyl mit 4 bis 12 Kohlenstoffatomen, gegebenenfalls substituiert mit einer oder zwei Gruppen, unabhängig ausgewählt aus R⁵;
gesättigten oder ungesättigten mono- oder bicyclischen Heterocyclus, welcher ein Heteroatom enthält, ausgewählt aus 0, S oder NR-C₁-C₆-Alkyl, gegebenenfalls substituiert mit einer oder zwei Gruppen, unabhängig ausgewählt aus R⁵; oder
R⁸R⁹N-C₁-C₆-Alkoxyaryl-C₁-C₆-alkyl steht, worin R⁸ und R⁹ unabhängig ausgewählt werden aus C₁-C₆-Alkyl oder R⁸ und R⁹ zusammen mit dem dazwischen geschobenen Stickstoff einen 5-7-gliedrigen gesättigten heterocyclischen Ring bilden, welcher gegebenenfalls ein Sauerstoffatom enthält, wobei die Arylgruppe für Phenyl oder Naphthyl steht;
oder ein pharmazeutisch annehmbares Salz davon.

4. Verbindung wie in Anspruch 1 beansprucht, welche ausgewählt wird aus der Gruppe von Verbindungen, bestehend aus:
1-Benzyl-4-(4-methoxy-benzolsulfonyl)-piperidin-4-carbonsäure-hydroxyamid;
4-(4-Methoxy-benzolsulfonyl)-1-(3-methoxy-benzyl)-piperidin-4-carbonsäure-hydroxyamid,
1-(3,4-Dichlorbenzyl)-4-(4-methoxy-benzolsulfonyl)-piperidin-4-carbonsäure-hydroxamid,
4-(4-Methoxy-benzolsulfonyl)-1-(4-methylbenzyl)-piperidin-4-carbonsäure-hydroxamid,
4-(4-Methoxy-benzolsulfonyl)-1-naphthalin-2-yl-methylpiperidin-4-carbonsäure-hydroxamid,
2-Biphenyl-4-ylmethyl-4-(4-methoxy-benzolsulfonyl)piperidin-4-carbonsäure-hydroxamid,
4-(4-Methoxy-benzolsulfonyl)-1-(3-methyl-but-2-enyl)piperidin-4-carbonsäure-hydroxamid,
1-(4-Brom-benzyl)-4-(4-methoxy-benzolsulfonyl)-piperidin-4-carbonsäure-hydroxyamid,
4-(4-Methoxy-benzolsulfonyl)-1-(4-(2-piperidin-1-yl-ethoxy)-benzyl]-piperidin-4-carbonsäure-hydroxyamid
und pharmazeutisch annehmbare Salze davon.

5. Verbindung wie in Anspruch 1 beansprucht oder ein pharmazeutisch annehmbares Salz davon, welche ausgewählt wird aus der Gruppe von Verbindungen, bestehend aus:
4-(4-Methoxy-benzolsulfonyl)-1-(3-phenyl-propyl)-piperidin-4-carbonsäure-hydroxyamid,
1-tert.-Butyl-4-(4-methoxy-benzolsulfonyl)-piperidin-4-carbonsäure-hydroxyamid,
1-Butyl-4-(4-methoxy-benzolsulfonyl)-piperidin-4-carbonsäure-hydroxyamid,
1-Cyclooctyl-4-(4-methoxy-benzolsulfonyl)-piperidin-4-carbonsäure-hydroxyamid,
1-Ethyl-4-(4-methoxy-benzolsulfonyl)-piperidin-4-carbonsäure-hydroxyamid,
1-Isopropyl-4-(4-methoxy-benzolsulfonyl)-piperidin-4-carbonsäure-hydroxyamid,
1-Methyl-4-(4-methoxy-benzolsulfonyl)-piperidin-4-carbonsäure-hydroxyamid,
1-Benzyl-4-(4-butoxy-benzolsulfonyl)-piperidin-4-carbonsäure-hydroxyamid,
1-(4-Fluorbenzyl)-4-(4-methoxy-benzolsulfonyl)-piperidin-4-carbonsäure-hydroxyamid,
1-(4-Fluorbenzyl)-4-(4-butoxy-benzolsulfonyl)-piperidin-4-carbonsäure-hydroxyamid,
4-(4-Methoxy-benzolsulfonyl)-1-(4-methoxy-benzyl)-piperidin-4-carbonsäure-hydroxyamid,
4-(4-Methoxy-benzolsulfonyl)-1-[2-(4-methoxyphenyl)-ethyl]-piperidin-4-carbonsäure-hydroxyamid,
4-(4-Methoxy-benzolsulfonyl)-1-(2-phenyl-ethyl)-piperidin-4-carbonsäure-hydroxyamid,
4-(4-n-Butoxy-benzolsulfonyl)-1-(4-methoxy-benzyl)-piperidin-4-carbonsäure-hydroxyamid,
4-(4-Methoxy-benzolsulfonyl)-1-(3-phenoxy-propyl)-piperidin-4-carbonsäure-hydroxyamid,
4-(4-n-Butoxy-benzolsulfonyl)-1-(3-phenoxy-propyl)-piperidin-4-carbonsäure-hydroxyamid,
4-(4-Methoxy-benzolsulfonyl)-1-(2-phenoxy-ethyl)-piperidin-4-carbonsäure-hydroxyamid,
4-(4-n-Butoxy-benzolsulfonyl)-1-(2-phenoxy-ethyl)-piperidin-4-carbonsäure-hydroxyamid und
4-(4-Methoxy-benzolsulfonyl)-1-[4-(2-piperidin-1-yl-ethoxy)-benzyl]-piperidin-4-carbonsäure-hydroxyamid.

6. Pharmazeutische Zusammensetzung, welche einen pharmazeutischen Träger und eine therapeutisch wirksame Menge einer Matrixmetalloproteinase oder TACE-hemmenden Verbindung umfasst, wie in einem der Ansprüche 1 bis 5 beansprucht.

7. Verbindung wie in einem der Ansprüche 1 bis 5 beansprucht zur Verwendung als Medikament.

8. Verwendung einer Verbindung wie in einem der Ansprüche 1 bis 5 beansprucht zur Herstellung eines Medikaments zum Hemmen pathologischer Veränderungen, vermittelt durch Matrix-metalloproteinasen in Säugern, oder zum Hemmen von pathologischen Veränderungen, vermittelt durch TNF-α umwandelndes Enzym (TACE) in Säugern.

## Revendications

1. Composé suivant la formule I 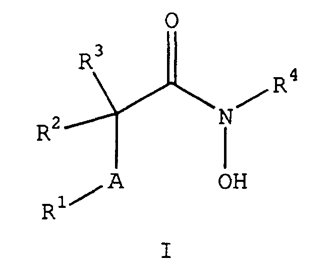 dans laquelle :
R¹ est un alkyle de 1 à 18 atomes de carbone, facultativement substitué par un ou deux groupements indépendamment sélectionnés parmi R⁵;
un alcényle de 3 à 18 atomes de carbone présentant 1 à 3 doubles liaisons, facultativement substitué par un ou deux groupements indépendamment sélectionnés parmi R⁵;
un alcynyle de 3 à 18 atomes de carbone présentant 1 à 3 triples liaisons, facultativement substitué par un ou deux groupements indépendamment sélectionnés parmi R⁵;
un aryle de 6 à 10 atomes de carbone, facultativement substitué par un ou deux groupements indépendamment sélectionnés parmi R⁵;
un cycloalkyle de 3 à 8 atomes de carbone, facultativement substitué par un ou deux groupements indépendamment sélectionnés parmi R⁵;
un hétérocycle mono- ou bicyclique saturé ou insaturé de 5 à 10 chaînons contenant un hétéroatome sélectionné parmi O, S ou NR⁷, facultativement substitué par un ou deux groupements indépendamment sélectionnés parmi R⁵;
ou hétéroaryl-(CH₂)₀₋₆- dans lequel le groupement hétéroaryle est à de 5 à 6 chaînons présentant un ou deux hétéroatomes indépendamment sélectionnés parmi 0, S et N et peut être facultativement substitué par un ou deux groupements indépendamment sélectionnés parmi R⁵;
A est -S-, -SO- ou SO₂-;
R² et R³, considérés avec l'atome de carbone auquel ils sont liés, forment un cycle hétérocyclique de 5 à 7 chaînons contenant O, S ou N-R⁷ présentant facultativement une ou deux doubles liaisons;
R⁴ est un hydrogène,
un alkyle de 1 à 6 atomes de carbone, facultativement substitué par un ou deux groupements indépendamment sélectionnés parmi R⁵;
un alcényle de 3 à 18 atomes de carbone présentant 1 à 3 doubles liaisons, facultativement substitué par un ou deux groupements indépendamment sélectionnés parmi R⁵;
un alcynyle de 3 à 18 atomes de carbone présentant 1 à 3 triples liaisons, facultativement substitué par un ou deux groupements indépendamment sélectionnés parmi R⁵;
un phényle ou un naphtyle facultativement substitué par un ou deux groupements indépendamment sélectionnés parmi R⁵;
un cycloalkyle ou un bicycloalkyle en C₃-C₈ facultativement substitué par un ou deux groupements indépendamment sélectionnés parmi R⁵;
un hétérocycle mono- ou bicyclique saturé ou insaturé de 5 à 10 chaînons contenant un hétéroatome sélectionné parmi O, S ou NR⁷, facultativement substitué par un ou deux groupements indépendamment sélectionnés parmi R⁵;
R⁵ est H, un aroyle en C₇-C₁₁, un alcanoyle en C₂-C₆, un alkyle en C₁-C₁₂, un alcényle en C₂-C₁₂, un alcynyle en C₂-C₁₂, F, Cl, Br, I, CN, CHO, un alcoxy en C₁-C₆, un aryloxy, un hétéroaryloxy, un alcényloxy en C₃-C₆, un alcynyloxy en C₃-C₆, un alcoxyaryle en C₁-C₆, un alcoxyhétéroaryle en C₁-C₆, un (alkylamino en C₁-C₆)alcoxy en C₁-C₆, un (alkylène en C₁-C₂)dioxy, une aryloxy(alkyle en C₁-C₆)amine, un perfluoroalkyle en C₁-C₁₂, S(O)ₙ-alkyle en C₁-C₆, S(O)ₙ-aryle, où n est 0, 1 ou 2; OCOO-alkyle en C₁-C₆, OCOO-aryle, OCONR⁶, COOH, COO-alkyle en C₁-C₆, COO-aryle, CONR⁶R⁶, CONHOH, NR⁶R⁶, SO₂NR⁶R⁶, NR⁶SO₂-aryle, -NR⁶CONR⁶R⁶, NHSO₂CF₃, SO₂NH-hétéroaryle, SO₂NHCO-aryle, CONHSO₂-alkyle en C₁-C₆, CONHSO₂-aryle, SO₂NHCO-aryle, CONHSO₂-alkyle en C₁-C₆, CONHSO₂-aryle, NH₂, OH, aryle, hétéroaryle, cycloalkyle en C₃-C₈; ou un hétérocycle mono- ou bicyclique saturé ou insaturé de 5 à 10 chaînons contenant un hétéroatome sélectionné parmi 0, S ou NR⁷;
dans lequel un alkyle en C₁-C₆ est linéaire ou ramifié, un hétéroaryle est un groupement hétéroaryle mono- ou bicyclique de 5-10 chaînons présentant 1 à 3 hétéroatomes indépendamment sélectionnés parmi O, S ou NR⁷ et un aryle est un phényle ou un naphtyle, facultativement substitué par 1 ou 2 groupements sélectionnés parmi un halogène, un cyano, un amino, un nitro, un alkyle en C₁-C₆, un alcoxy en C₁-C₆, ou un hydroxy;
R⁶ est H, un alkyle en C₁-C₁₈ facultativement substitué par OH; un alcényle en C₃-C₆, un alcynyle en C₃-C₆, un perfluoroalkyle en C₁-C₆, S(O)ₙ-alkyle en C₁-C₆, S(O)ₙ-aryle où n est 0, 1 ou 2; ou CO-hétéroaryle; dans lequel un hétéroaryle est un groupement hétéroaryle mono- ou bicyclique de 5-10 chaînons présentant 1 à 3 hétéroatomes indépendamment sélectionnés parmi O, S ou NR⁷ et un aryle est un phényle ou un naphtyle, facultativement substitué par 1 ou 2 groupements sélectionnés parmi un halogène, un cyano, un amino, un nitro, un alkyle en C₁-C₆, un alcoxy en C₁-C₆, ou un hydroxy;
et R⁷ est un aroyle en C₇-C₁₁, un alcanoyle en C₂-C₆, un perfluoroalkyle en C₁-C₁₂, S(O)ₙ-alkyle en C₁-C₆, S(O)ₙ-aryle où n est 0, 1 ou 2; COO-alkyle en C₁-C₆, COO-aryle, CONHR⁶, CONR⁶R⁶, CONHOH, SO₂NR⁶R⁶, SO₂CF₃, SO₂NH-hétéroaryle, SO₂NHCO-aryle, CONHSO-alkyle en C₁-C₆, CONHSO₂-aryle, un aryle, ou un hétéroaryle, où aryle est un phényle ou un naphtyle, facultativement substitué par 1 ou 2 groupements indépendamment sélectionnés parmi un halogène, un cyano, un amino, un nitro, un alkyle en C₁-C₆, un alcoxy en C₁-C₆, ou un hydroxy; et un hétéroaryle est un groupement hétéroaryle mono- ou bicyclique de 5-10 chaînons présentant 1 à 3 hétéroatomes indépendamment sélectionnés parmi O, S ou N-alkyle en C₁-C₆;
un alkyle de 1 à 18 atomes de carbone, facultativement substitué par un ou deux groupements indépendamment sélectionnés parmi R⁵;
un alcényle de 3 à 18 atomes de carbone présentant de 1 à 3 doubles liaisons, facultativement substitué par un ou deux groupements indépendamment sélectionnés parmi R⁵;
un alcynyle de 3 à 18 atomes de carbone présentant de 1 à 3 triples liaisons, facultativement substitué par un ou deux groupements indépendamment sélectionnés parmi R⁵;
un arylalkyle de 7 à 16 atomes de carbone, dans lequel l'aryle est facultativement substitué par un ou deux groupements indépendamment sélectionnés parmi R⁵;
un biphénylalkyle de 13 à 18 atomes de carbone, dans lequel le biphényle est facultativement substitué par un ou deux groupements indépendamment sélectionnés parmi R⁵;
un arylalcényle de 8 à 16 atomes de carbone, dans lequel l'aryle est facultativement substitué par un ou deux groupements indépendamment sélectionnés parmi R⁵;
un cycloalkylalkyle ou un bicycloalkylalkyle de 4 à 12 atomes de carbone, dans lequel le groupement cycloalkyle ou bicycloalkyle est facultativement substitué par un ou deux groupements indépendamment sélectionnés parmi R⁵;
un hétérocycle mono- ou bicyclique saturé ou insaturé contenant un hétéroatome sélectionné parmi O, S ou N-alkyle en C₁-C₆, facultativement substitué par un ou deux groupements indépendamment sélectionnés parmi R⁵; ou
R⁸R⁹N-(alcoxyaryle en C₁-C₆)-alkyle en C₁-C₆ où R⁸ et R⁹ sont indépendamment sélectionnés parmi un alkyle en C₁-C₆ ou R⁸ et R⁹ conjointement avec l'azote intercalé forment un cycle hétérocyclique saturé de 5-7 chaînons contenant facultativement un atome d'oxygène, dans lequel le groupement aryle est un phényle ou un naphtyle
ou un sel pharmaceutiquement acceptable de celui-ci.

2. Composé suivant la revendication 1, dans lequel :
R¹ est un alkyle de 1 à 18 atomes de carbone, facultativement substitué par un ou deux groupements indépendamment sélectionnés parmi R⁵;
un alcényle de 3 à 18 atomes de carbone présentant 1 à 3 doubles liaisons, facultativement substitué par un ou deux groupements indépendamment sélectionnés parmi R⁵;
un alcynyle de 3 à 18 atomes de carbone présentant 1 à 3 triples liaisons, facultativement substitué par un ou deux groupements indépendamment sélectionnés parmi R⁵;
un aryle de 6 à 10 atomes de carbone, facultativement substitué par un à deux groupements indépendamment sélectionnés parmi R⁵;
un cycloalkyle de 3 à 8 atomes de carbone, facultativement substitué par un à deux groupements indépendamment sélectionnés parmi R⁵;
un hétérocycle mono- ou bicyclique saturé ou insaturé de 5 à 10 chaînons contenant un hétéroatome sélectionné parmi O, S ou NR⁷, facultativement substitué par un à deux groupements indépendamment sélectionnés parmi R⁵;
ou hétéroaryle-(CH₂)₀₋₆- dans lequel le groupement hétéroaryle est de 5 à 6 chaînons présentant un ou deux hétéroatomes indépendamment sélectionnés parmi O, S, et N et il peut être facultativement substitué par un ou deux groupements indépendamment sélectionnés parmi R⁵;
A est -S-, -SO- ou SO₂-;
R² et R³, considérés avec l'atome de carbone auquel ils sont liés, forment un cycle hétérocyclique de 5 à 7 chaînons contenant O, S ou N-R⁷ présentant facultativement une ou deux doubles liaisons;
R⁴ est un hydrogène,
un alkyle de 1 à 6 atomes de carbone, facultativement substitué par un ou deux groupements indépendamment sélectionnés parmi R⁵;
un alcényle de 3 à 18 atomes de carbone présentant 1 à 3 doubles liaisons, facultativement substitué par un ou deux groupements indépendamment sélectionnés parmi R⁵;
un alcynyle de 3 à 18 atomes de carbone présentant 1 à 3 triples liaisons, facultativement substitué par un ou deux groupements indépendamment sélectionnés parmi R⁵;
un phényle ou un naphtyle facultativement substitué par un ou deux groupements indépendamment sélectionnés parmi R⁵;
un cycloalkyle ou un bicycloalkyle en C₃-C₈ facultativement substitué par un ou deux groupements indépendamment sélectionnés parmi R⁵;
R⁵ est H, F, Cl, Br, I, CN, CHO, un aroyle en C₇-C₁₁, un alcanoyle en C₂-C₆, un alkyle en C₁-C₁₂, un alcényle en C₂-C₁₂, un alcynyle en C₂-C₁₂, un alcoxy en C₁-C₆, un aryloxy, un hétéroaryloxy, un alcényloxy en C₃-C₆, un alcynyloxy en C₃-C₆, un alcoxyaryle en C₁-C₆, un allcoxyhétéroaryle en C₁-C₆, un (alkylamino en C₁-C₆)alcoxy en C₁-C₆, un (alkylène en C₁-C₂)dioxy, une aryloxy(alkyle en C₁-C₆) amine, un perfluoroalkyle en C₁-C₁₂, S(O)ₙ-alkyle en C₁-C₆, S(O)ₙ-aryle, où n est 0, 1 ou 2; OCOO-alkyle en C₁-C₆, OCOO-aryle, OCONR⁶, COOH, COO-alkyle en C₁-C₆, COO-aryle, CONR⁶R⁶, CONHOH, NR⁶R⁶, SO₂NR⁶R⁶, NR⁶SO₂-aryle, NR⁶CONR⁶R⁶, NHSO₂CF₃, SO₂NH-hétéroaryle, SO₂NHCO-aryle, CONHSO₂-alkyle en C₁-C₆, CONHSO₂-aryle, SO₂NHCO-aryle, CONHSO₂-alkyle en C₁-C₆, CONHSO₂-aryle, NH₂, OH, un aryle, un hétéroaryle, un cycloalkyle en C₃-C₈; ou un hétérocycle mono- ou bicyclique saturé ou insaturé de 5 à 10 chaînons contenant un hétéroatome sélectionné parmi O, S ou NR⁷;
dans lequel un hétéroaryle est un groupement hétéroaryle mono- ou bicyclique de 5-10 chaînons présentant 1 à 3 hétéroatomes indépendamment sélectionnés parmi O, S ou NR⁷ et un aryle est un phényle ou un naphtyle, facultativement substitué par 1 ou 2 groupements indépendamment sélectionnés parmi un halogène, un cyano, un amino, un nitro, un alkyle en C₁-C₆, un alcoxy en C₁-C₆, ou un hydroxy;
R⁶ est H, un alkyle en C₁-C₁₈ facultativement substitué par OH; un alcényle en C₃-C₆, un alcynyle en C₃-C₆, un perfluoroalkyle en C₁-C₆, S(O)ₙ-alkyle ou aryle où n est 0, 1 ou 2; ou CO-hétéroaryle;
dans lequel un hétéroaryle est un groupement hétéroaryle mono- ou bicyclique de 5-10 chaînons présentant 1 à 3 hétéroatomes indépendamment sélectionnés parmi O, S ou NR⁷ et un aryle est un phényle ou un naphtyle, facultativement substitué par 1 ou 2 groupements sélectionnés parmi un halogène, un cyano, un amino, un nitro, un alkyle en C₁-C₆, un alcoxy en C₁-C₆, ou un hydroxy;
et R⁷ est un aroyle en C₇-C₁₁, un alcanoyle en C₂-C₆, un perfluoroalkyle en C₁-C₁₂, S(O)ₙ-alkyle, S(O)ₙ-aryle où n est 0, 1 ou 2; COO-alkyle, COO-aryle, CONHR⁶, CONR⁶R⁶, CONHOH, SO₂NR⁶R⁶, SO₂CF₃, SO₂NH-hétéroaryle, SO₂NHCO-aryle, CONHSO₂-alkyle, CONHSO₂-aryle, un aryle, un hétéroaryle;
dans lequel un alkyle en C₁-C₆ est linéaire ou ramifié, un hétéroaryle est un groupement hétéroaryle mono- ou bicyclique de 5-10 chaînons présentant 1 à 3 hétéroatomes indépendamment sélectionnés parmi O, S ou NR⁷ et un aryle est un phényle ou un naphtyle, facultativement substitué par 1 ou 2 groupements sélectionnés parmi un halogène, un cyano, un amino, un nitro, un alkyle en C₁-C₆, un alcoxy en C₁-C₆, ou un hydroxy;
un alkyle de 1 à 18 atomes de carbone, facultativement substitué par un ou deux groupements indépendamment sélectionnés parmi R⁵;
un alcényle de 3 à 18 atomes de carbone présentant de 1 à 3 doubles liaisons, facultativement substitué par un ou deux groupements indépendamment sélectionnés parmi R⁵;
un alcynyle de 3 à 18 atomes de carbone présentant de 1 à 3 triples liaisons, facultativement substitué par un ou deux groupements indépendamment sélectionnés parmi R⁵;
un arylalkyle de 7 à 16 atomes de carbone, dans lequel l'aryle est facultativement substitué par un ou deux groupements indépendamment sélectionnés parmi R⁵;
un biphénylalkyle de 13 à 18 atomes de carbone, dans lequel le biphényle est facultativement substitué par un ou deux groupements indépendamment sélectionnés parmi R⁵;
un arylalcényle de 8 à 16 atomes de carbone, dans lequel l'aryle est facultativement substitué par un ou deux groupements indépendamment sélectionnés parmi R⁵;
un cycloalkylalkyle ou un bicycloalkylalkyle de 4 à 12 atomes de carbone, dans lequel le groupement cycloalkyle ou bicycloalkyle est facultativement substitué par un ou deux groupements indépendamment sélectionnés parmi R⁵;
un hétérocycle mono- ou bicyclique saturé ou insaturé contenant un hétéroatome sélectionné parmi 0, S ou N-alkyle en C₁-C₆, facultativement substitué par un ou deux groupements indépendamment sélectionnés parmi R⁵;
R⁸R⁹N-(alcoxyaryle en C₁-C₆)-alkyle en C₁-C₆ où R⁸ et R⁹ sont indépendamment sélectionnés parmi un alkyle en C₁-C₆ ou R⁸ et R⁹ conjointement avec l'azote intercalé forment un cycle hétérocyclique saturé de 5-7 chaînons contenant facultativement un atome d'oxygène, dans lequel le groupement aryle est un phényle ou un naphtyle;
ou un sel pharmaceutiquement acceptable de celui-ci.

3. Composé suivant la revendication 2, dans lequel :
R¹ est un phényle, un naphtyle, un alkyle de 1 à 18 atomes de carbone ou un hétéroaryle comme un pyridyle, un thiényle, un imidazolyle ou un furanyle, facultativement substitué par un alkyle en C₁-C₆, un alcoxy en C₁-C₆, un aryloxy en C₆-C₁₀, un hétéroaryloxy, un alcényloxy en C₃-C₆, un alcynyloxy en C₃-C₆, un halogène; ou S(O)ₙ-alkyle en C₁-C₆, un alcoxyaryle en C₁-C₆ ou un alcoxyhétéroaryle en C₁-C₆;
A est -S-, -SO- ou -SO₂-;
R² et R³, considérés avec l'atome de carbone auquel ils sont liés, forment un cycle hétérocyclique de 5 à 7 chaînons contenant O, S ou N-R⁷ présentant facultativement une ou deux doubles liaisons;
R⁴ est un hydrogène,
un alkyle de 1 à 6 atomes de carbone, facultativement substitué par un ou deux groupements indépendamment sélectionnés parmi R⁵;
un alcényle de 3 à 18 atomes de carbone présentant 1 à 3 doubles liaisons, facultativement substitué par un ou deux groupements indépendamment sélectionnés parmi R⁵;
un alcynyle de 3 à 18 atomes de carbone présentant 1 à 3 triples liaisons, facultativement substitué par un ou deux groupements indépendamment sélectionnés parmi R⁵;
un phényle ou un naphtyle facultativement substitué par un ou deux groupements indépendamment sélectionnés parmi R⁵;
un cycloalkyle ou un bicycloalkyle en C₃-C₈ facultativement substitué par un ou deux groupements indépendamment sélectionnés parmi R⁵;
R⁵ est H, un aroyle en C₇-C₁₁, un alcanoyle en C₂-C₆, un alkyle en C₁-C₁₂, un alcényle en C₂-C₁₂, un alcynyle en C₂-C₁₂, F, Cl, Br, I, CN, CHO, un alcoxy en C₁-C₆, un aryloxy, un hétéroaryloxy, un alcényloxy en C₃-C₆, un alcynyloxy en C₃-C₆, un (alkylamino en C₁-C₆)alcoxy en C₁-C₆, un (alkylène en C₁-C₂)dioxy, une aryloxy (alkyle en C₁-C₆)amine, un perfluoroalkyle en C₁-C₁₂, S(O)ₙ-alkyle en C₁-C₆, S(O)ₙ-aryle, où n est 0, 1 ou 2; OCOO-alkyle en C₁-C₆, OCOO-aryle, OCONR⁶, COOH, COO-alkyle en C₁-C₆, COO-aryle, CONR⁶R⁶, CONHOH, NR⁶R⁶, SO₂NR⁶R⁶, NR⁶SO₂-aryle, -NR⁶CONR⁶R⁶, NHSO₂CF₃, SO₂NH-hétéroaryle, SO₂NHCO-aryle, CONHSO₂-alkyle en C₁-C₆, CONHSO₂-aryle, SO₂NHCO-aryle, CONHSO₂-alkyle en C₁-C₆, CONHSO₂-aryle, NH₂, OH, un aryle, un hétéroaryle, un cycloalkyle en C₃-C₈; ou
un hétérocycle mono- ou bicyclique saturé ou insaturé de 5 à 10 chaînons contenant un hétéroatome sélectionné parmi O, S ou NR⁷; dans lequel un alkyle en C₁-C₆ est linéaire ou ramifié, un hétéroaryle est un groupement hétéroaryle mono- ou bicycliqué de 5-10 chaînons présentant 1 à 3 hétéroatomes indépendamment sélectionnés parmi O, S ou NR⁷ et un aryle est un phényle ou un naphtyle, facultativement substitué par 1 ou 2 groupements sélectionnés parmi un halogène, un cyano, un amino, un nitro, un alkyle en C₁-C₆, un alcoxy en C₁-C₆, ou un hydroxy;
R⁶ est H, un alkyle en C₁-C₁₈ facultativement substitué par OH; un alcényle en C₃-C₆, un alcynyle en C₃-C₆, un perfluoroalkyle en C₁-C₆, S(O)ₙ-alkyle ou aryle où n est 0, 1 ou 2; ou CO-hétéroaryle;
dans lequel un hétéroaryle est un groupement hétéroaryle mono- ou bicyclique de 5-10 chaînons présentant 1 à 3 hétéroatomes indépendamment sélectionnés parmi O, S ou NR⁷ et un aryle est un phényle ou un naphtyle, facultativement substitué par 1 ou 2 groupements sélectionnés parmi un halogène, un cyano, un amino, un nitro, un alkyle en C₁-C₆, un alcoxy en C₁-C₆, ou un hydroxy;
et R⁷ est un aroyle en C₇-C₁₁, un alcanoyle en C₂-C₆, un perfluoroalkyle en C₁-C₁₂, S(O)ₙ-alkyle, S(O)ₙ-aryle où n est 0, 1 ou 2; COO-alkyle, COO-aryle, CONHR⁶, CONR⁶R⁶, CONHOH, SO₂NR⁶R⁶, SO₂CF₃, SO₂NH-hétéroaryle, SO₂NHCO-aryle, CONHSO₂-alkyle, CONHSO₂-aryle, un aryle, ou un hétéroaryle;
dans lequel un aryle est un phényle ou un naphtyle facultativement substitué par 1 ou 2 groupements indépendamment sélectionnés parmi un halogène, un cyano, un amiho, un nitro, un alkyle en C₁-C₆, un alcoxy en C₁-C₆, ou un hydroxy; et un hétéroaryle est un groupement hétéroaryle mono- ou bicyclique de 5-10 chaînons présentant 1 à 3 hétéroatomes indépendamment sélectionnés parmi O, S ou N-alkyle en C₁-C₆;
un alkyle de 1 à 18 atomes de carbone, facultativement substitué par un ou deux groupements indépendamment sélectionnés parmi R⁵;
un alcényle de 3 à 18 atomes de carbone présentant de 1 à 3 doubles liaisons, facultativement substitué par un ou deux groupements indépendamment sélectionnés parmi R⁵;
un alcynyle de 3 à 18 atomes de carbone présentant de 1 à 3 triples liaisons, facultativement substitué par un ou deux groupements indépendamment sélectionnés parmi R⁵;
un arylalkyle de 7 à 16 atomes de carbone facultativement substitué par un ou deux groupements indépendamment sélectionnés parmi R⁵;
un biphénylalkyle de 13 à 18 atomes de carbone facultativement substitué par un ou deux groupements indépendamment sélectionnés parmi R⁵;
un arylalcényle de 8 à 16 atomes de carbone facultativement substitué par un ou deux groupements indépendamment sélectionnés parmi R⁵;
un cycloalkylalkyle ou un bicycloalkylalkyle de 4 à 12 atomes de carbone facultativement substitué par un ou deux groupements indépendamment sélectionnés parmi R⁵;
un hétérocycle mono- ou bicyclique saturé ou insaturé contenant un hétéroatome sélectionné parmi 0, S ou NR-alkyle en C₁-C₆, facultativement substitué par un ou deux groupements indépendamment sélectionnés parmi R⁵;
R⁸R⁹N-(alcoxyaryle en C₁-C₆)-alkyle en C₁-C₆ où R⁸ et R⁹ sont indépendamment sélectionnés parmi un alkyle en C₁-C₆ ou R⁸ et R⁹ conjointement avec l'azote intercalé forment un cycle hétérocyclique saturé de 5-7 chaînons contenant facultativement un atome d'oxygène, dans lequel le groupement aryle est un phényle ou un naphtyle;
ou un sel pharmaceutiquement acceptable de celui-ci.

4. Composé suivant la revendication 1, qui est sélectionné parmi le groupe de composés comprenant :
l'hydroxyamide de l'acide 1-benzyl-4-(4-méthoxybenzènesulfonyl)pipéridine-4-carboxylique
l'hydroxyamide de l'acide 4-(4-méthoxybenzènesulfonyl)-1-(3-méthoxybenzyl)pipéridine-4-carboxylique
l'hydroxyamide de l'acide 1-(3,4-dichlorobenzyl)-4-(4-méthoxybenzènesulfonyl)pipéridine-4-carboxylique
l'hydroxyamide de l'acide 4-(4-méthoxybenzènesulfonyl)-1-(4-méthylbenzyl)pipéridine-4-carboxylique,
l'hydroxyamide de l'acide (4-méthoxybenzènesulfonyl)-1-naphtalène-2-ylméthylpipéridine-4-carboxylique,
l'hydroxyamide de l'acide 1-biphényl-4-ylméthyl-4-(4-méthoxybenzènesulfonyl)pipéridine-4-carboxylique,
l'hydroxyamide de l'acide 4-(4-méthoxybenzènesulfonyl)-1-(3-méthylbut-2-ényl)pipéridine-4-carboxylique,
l'hydroxyamide de l'acide 1-(4-bromobenzyl)-4-(4-méthoxybenzènesulfonyl)pipéridine-4-carboxylique,
l'hydroxyamide de l'acide 4-(4-méthoxybenzènesulfonyl)-1-[4-(2-pipéridin-1-yléthoxy)benzyl]pipéridine-4-carboxylique,
et des sels pharmaceutiquement acceptables de ceux-ci.

5. Composé suivant la revendication 1 ou un sel pharmaceutiquement acceptable de celui-ci qui est sélectionné parmi le groupe de composés comprenant :
l'hydroxyamide de l'acide 4-(4-méthoxybenzènesulfonyl)-1-(3-phénylpropyl)pipéridine-4-carboxylique,
l'hydroxyamide de l'acide 1-t-butyl-4-(4-méthoxybenzènesulfonyl)pipéridine-4-carboxylique,
l'hydroxyamide de l'acide 1-butyl-4-(4-méthoxybenzènesulfonyl)pipéridine-4-carboxylique,
l'hydroxyamide de l'acide 1-cyclooctyl-4-(4-méthoxybenzènesulfonyl)pipéridine-4-carboxylique
l'hydroxyamide de l'acide 1-éthyl-4-(4-méthoxybenzènesulfonyl)pipéridine-4-carboxylique,
l'hydroxyamide de l'acide 1-i-propyl-4-(4-méthoxybenzènesulfonyl)pipéridine-4-carboxylique,
l'hydroxyamide de l'acide 1-méthyl-4-(4-méthoxybenzènesulfonyl)pipéridine-4-carboxylique,
l'hydroxyamide de l'acide 1-benzyl-4-(4-butoxybenzènesulfonyl)pipéridine-4-carboxylique,
l'hydroxyamide de l'acide 1-(4-fluorobenzyl)-4-(4-méthoxybenzènesulfonyl)pipéridine-4-carboxylique,
l'hydroxyamide de l'acide 1-(4-fluorobenzyl)-4-(4-butoxybenzènesulfonyl)pipéridine-4-carboxylique,
l'hydroxyamide de l'acide 4-(4-méthoxybenzènesulfonyl)-1-(4-méthoxybenzyl)pipéridine-4-carboxylique,
l'hydroxyamide de l'acide 4-(4-méthoxybenzènesulfonyl)-1-[2-(4-méthoxyphényl)éthyl]pipéridine-4-carboxylique,
l'hydroxyamide de l'acide 4-(4-méthoxybenzènesulfonyl)-1-(2-phényléthyl)pipéridine-4-carboxylique,
l'hydroxyamide de l'acide 4-(4-n-butoxybenzènesulfonyl)-1-(4-méthoxybenzyl)pipéridine-4-carboxylique,
l'hydroxyamide de l'acide 4-(4-méthoxybenzènesulfonyl)-1-(3-phénoxypropyl)pipéridine-4-carboxylique,
l'hydroxyamide de l'acide 4-(4-n-butoxybenzènesulfonyl)-1-(3-phénoxypropyl)pipéridine-4-carboxylique,
l'hydroxyamide de l'acide 4-(4-méthoxybenzènesulfonyl)-1-(2-phénoxyéthyl)pipéridine-4-carboxylique,
l'hydroxyamide de l'acide 4-(4-n-butoxybenzènesulfonyl)-1-(2-phénoxyéthyl)pipéridine-4-carboxylique, et
l'hydroxyamide de l'acide 4-(4-méthoxybenzènesulfonyl)-1-[4-(2-pipéridin-1-yléthoxy)benzyl]pipéridine-4-carboxylique.

6. Composition pharmaceutique comprenant un vecteur pharmaceutique et une quantité efficace sur le plan thérapeutique d'une métalloprotéinase de matrice ou d'un composé inhibiteur de la TACE suivant l'une quelconque des revendications 1 à 5.

7. Composé suivant l'une quelconque des revendications 1 à 5, pour une utilisation comme médicament.

8. Utilisation d'un composé suivant l'une quelconque des revendications 1 à 5, pour la fabrication d'un médicament destiné à inhiber des changements pathologiques dus à des métalloprotéinases de matrice chez les mammifères ou destiné à inhiber des changements pathologiques dus à une enzyme de conversion du TNF-α (TACE) chez des mammifères.
